# EUROPEAN PATENT APPLICATION

(11) **EP 3 521 287 A1**
(43) Date of publication of application: **07.08.2019**
(21) Application number: 17856034.8
(22) Date of filing: 25.09.2017
(51) Int. Cl.: C07D 471/04, A01C 1/08, A01G 7/00, A01N 43/90, A01N 47/02, A01P 7/04, A61K 31/519, A61P 33/14

(54) **NOVEL MESOIONIC INSECTICIDAL COMPOUND**

(30) Priority: 28.09.2016 JP 2016189259
(71) Applicant: Nippon Kayaku Kabushiki Kaisha, Tokyo 100-0005 (JP)
(72) Inventor: KAMO Tomohiro, Kamisu-shi Ibaraki 314-0255 (JP); HASEGAWA Shinji, Kamisu-shi Ibaraki 314-0255 (JP); KAGOHARA Yuma, Kamisu-shi Ibaraki 314-0255 (JP); UENO Shotaro, Kamisu-shi Ibaraki 314-0255 (JP); MIYAKE Takaaki, Kamisu-shi Ibaraki 314-0255 (JP); KOBAYASHI Takeru, Kamisu-shi Ibaraki 314-0255 (JP); MATSUDA Ryusei, Kamisu-shi Ibaraki 314-0255 (JP); ASANO Shu, Kamisu-shi Ibaraki 314-0255 (JP)
(74) Representative: Gille Hrabal
(86) International application number: PCT/JP2017/034483
(87) International publication number: WO 2018/062082

(57) **Abstract**

The purpose of the present invention is to provide a mesoionic compound, which exhibits an improved controlling effect on various harmful organisms, or a salt thereof. A mesoionic compound represented by formula (1) (wherein: R¹ is selected from a hydrogen atom, etc.; R² represents an optionally substituted phenyl group; R³ is selected from a hydrogen atom, etc.; R⁴ is selected from a hydrogen atom, etc.; R⁵ is selected from an optionally substituted ethylene group, etc.; and X is selected from an oxygen atom, etc.) or a salt thereof.

## Description

### Technical Field

The present invention relates to a mesoionic compound, a manufacturing method thereof, and a harmful organism control agent containing the same as active ingredient.

### Background Art

In Patent Document 1 and Patent Document 2, mesoionic pyrimidinium compounds are disclosed as insecticides.

### Prior Art Documents

### Patent Document

Patent Document 1: JP 2013-501061 A
Patent Document 2: JP 2014-503550 A

### Summary of Invention

### Technical Problem

The mesoionic compounds described in Patent Document 1 and Patent Document 2 do not necessarily exhibit sufficient controlling effect against various harmful organisms. Furthermore, many of the harmful organisms easily acquire resistance to insecticides to considerably lessen the control effects of existing chemical agents, so that novel active ingredients of insecticides having a chemical structure, an action mechanism, or a site of action different from those of conventional insecticidal active compounds are desired. An object of the present invention is to provide a novel mesoionic compound or a salt thereof as active ingredient of insecticides having a more excellent controlling effect on various harmful organisms.

### Solution to Problem

Through extensive study of the technical problem, the present inventors found that the controlling activity of compounds represented by the formula (1), referred to also as "compound (1)", against harmful organisms can be considerably improved, so that the present invention has been accomplished.

The present invention relates to the following:

The present invention provides a composition comprising a compound (1) and at least one component selected from the group consisting of a surfactant, a solid diluent and a liquid diluent. In an embodiment, the present invention provides a composition for controlling harmful invertebrate organisms comprising the compound (1) and at least one component selected from the group consisting of a surfactant, a solid diluent and a liquid diluent, wherein the composition may further comprise at least one biologically active compound or chemical agent.

### Advantageous Effects of Invention

A mesoionic compound represented by the formula (1) according to the present invention having an imine bond exhibits excellent controlling effects against harmful organisms, being useful as a harmful organism-controlling agent.

### Description of Embodiments

A formula (1) provides a general definition of the compounds according to the present invention. Preferred substituents and/or the scope of the substituents in the compound (1) are specifically described below.

In the present specification, a halogen atom means a fluorine atom, a chlorine atom, a bromine atom or an iodine atom; "n-" means normal, "i-" means iso, "s-" means secondary, "c-" means cyclo, and "t-" means tertiary; "Me" means a methyl group, "Et" means an ethyl group, "Pr" means a propyl group, "Bu" means a butyl group, "Pen" means a pentyl group, "Ac" means an acetyl group, and "Ad" means an adamantly group. Furthermore, a numerical range shown by using "-" indicates a range including the respective numerical values prepositioned and post-positioned relative to "-" as the minimum and the maximum.

Each R¹ in the compound (1) is selected from R^{a} and C1 alkyl group, wherein the C1 alkyl group is optionally substituted with one or more R^{a}.

Each R^{a} including R^{a} in R²-R⁵ is independently selected from a hydrogen atom, a halogen atom, a cyano group, a nitro group, an SCN group, and a hydroxy group. In the present application, a "substituent" includes a hydrogen atom, which is expressed as "substituent" for convenience sake.

Specific examples of R¹ include a hydrogen atom, a fluorine atom, a chlorine atom, a bromine atom, an iodine atom, a methyl group, a monofluoromethyl group, a difluoromethyl group, a trifluoromethyl group, and a trichloromethyl group.

R² in the compound (1) is a phenyl group which is substituted with up to three R^{b}.

Each R^{b} including R^{b} in R³ is independently selected from the group consisting of: R^{a}, a pentafluorosulfanyl group, a straight or branched chain C1-C6 alkyl group, a straight or branched chain C2-C3 alkenyl group, a straight or branched chain C2-C3 alkynyl group, a straight or branched chain C1-C6 alkyloxy group, an amino group, a straight or branched chain C1-C4 alkylthio group, a C1-C4 alkylsulfoxy group, a C1-C4 alkylsulfonyl group, a phenyl group and a heterocyclic group.

R^{a} is as described above.

Regarding the substitution position of R^{b} on the phenyl group represented by R², in the case of one substitution, the substitution may occur at any one of the 2-position, the 3-position and the 4-position. In the case of two substitutions, the substitutions may occur at any one of combinations of the (2, 3) positions, the (2, 4) positions, the (2, 5) positions, the (2, 6) positions, the (3, 4) positions and the (3, 5) positions. In the case of three substitutions, the substitutions may occur at any one of combinations of the (2, 3, 4) positions, the (2, 3, 5) positions, the (2, 3, 6) positions, the (2, 4, 5) positions, the (2, 4, 6) positions and the (3, 4, 5) positions.

The C1-C6 alkyl group represented by each R^{b} may have a substituent. Examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include a methyl group, an ethyl group, a propyl group, an i-propyl group, a c-propyl group, an n-butyl group, an i-butyl group, an s-butyl group, a t-butyl group, a monochloromethyl group, a dichloromethyl group, a trichloromethyl group, a monofluoromethyl group, a difluoromethyl group, a trifluoromethyl group, a 2,2,2-trifluoroethyl group, a 1,1,2,2-tetrafluoroethyl group, a pentafluoroethyl group, a heptafluoro-n-propyl group, a 1,1,1,3,3,3-hexafluoro-2-propyl group, a heptafluoro-i-propyl group, a 1,1,1,3,3,3-hexafluoro-2-methyloxy-2-propyl group, a methyloxymethyl group, an ethyloxymethyl group, an n-propyloxymethyl group, a 2,2,2-trifluoroethyloxymethyl group, a 3,3,3-trifluoro-n-propyloxymethyl group, and a 2,2,3,3,3-pentafluoro-n-propyloxymethyl group.

The C2-C3 alkenyl group represented by each R^{b} may have a substituent, and examples of the substituent include one or more R^{a}.

Specific examples thereof include a vinyl group, a 1-propenyl group, a 2-propenyl group, and a 1-propen-2-yl group.

The C2-C3 alkynyl group represented by each R^{b} may have a substituent, and examples of the substituent include one or more R^{a}.

Specific examples thereof include an ethynyl group, a 1-propyn-1-yl group, and a 2-propyn-1-yl group.

The C1-C6 alkyloxy group represented by each R^{b} may have a substituent, and examples of the substituent include one or more of R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include a methyloxy group, an ethyloxy group, an n-propyloxy group, an i-propyloxy group, a c-propyloxy group, an n-butyloxy group, an i-butyloxy group, an s-butyloxy group, a t-butyloxy group, n-pentyloxy, a methyloxymethyloxy group, an ethyloxymethyloxy group, a methylthiomethyloxy group, a 2-methyloxyethyloxy group, a 2-ethyloxyethyloxy group, a 2-thiomethylethyloxy group, a difluoromethyloxy group, a trifluoromethyloxy group, a chlorodifluoromethyloxy group, a 2,2,2-trifluoroethyloxy group, a 1,1,2,2-tetrafluoroethyloxy group, and a 1,1,1,3,3,3-hexafluoro-2-propyloxy group.

The amino group represented by each R^{b} has two substituents selected from a hydrogen, a C1-C4 alkyl group and a C2 acyl group. The C1-C4 alkyl group may have a substituent including, for example, one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}. The C2 acyl group is optionally substituted with one or more R^{a}.

Specific examples of the C1-C4 alkyl group include a methyl group, an ethyl group, a propyl group, an i-propyl group, a c-propyl group, an n-butyl group, an i-butyl group, an s-butyl group, a t-butyl group, a trichloromethyl group, a monofluoromethyl group, a difluoromethyl group, a trifluoromethyl group, and a 2,2,2-trifluoroethyl group.

Specific examples of the C2 acyl group include an acetyl group and a trifluoroacetyl group.

The C1-C4 alkyl group in the C1-C4 alkylthio group, the C1-C4 alkylsulfoxy group and the C1-C4 alkylsulfonyl group represented by each R^{b} may have a substituent, and examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include a methylthio group, an ethylthio group, an n-propylthio group, an i-propylthio group, a c-propylthio group, an n-butylthio group, a methyloxymethylthio group, an ethyloxymethylthio group, a methylthiomethylthio group, a difluoromethylthio group, a trifluoromethylthio group, a chlorodifluoromethylthio group, a 2,2,2-trifluoroethylthio group, a methylsulfoxy group, an ethylsulfoxy group, an n-propylsulfoxy group, an i-propylsulfoxy group, a c-propylsulfoxy group, an n-butylsulfoxy group, a methyloxymethylsulfoxy group, an ethyloxymethylsulfoxy group, a difluoromethylsulfoxy group, a trifluoromethylsulfoxy group, a chlorodifluoromethylsulfoxy group, a 2,2,2-trifluoroethylsulfoxy group, a methylsulfonyl group, an ethylsulfonyl group, an n-propylsulfonyl group, an i-propylsulfonyl group, a c-propylsulfonyl group, an n-butylsulfonyl group, a methyloxymethylsulfonyl group, an ethyloxymethylsulfonyl group, a difluoromethylsulfonyl group, a trifluoromethylsulfonyl group, a chlorodifluoromethylsulfonyl group, and a 2,2,2-trifluoroethylsulfonyl group.

The phenyl group represented by R^{b} is substituted with one or more R^{a}.

Specific examples thereof include a phenyl group, a fluorophenyl group, a pentafluorophenyl group, a chlorophenyl group, a dichlorophenyl group, a nitrophenyl group, a methylphenyl group, a dimethylphenyl group, a trifluoromethylphenyl group, and a methoxyphenyl group.

The heterocyclic group represented by R^{b} has a ring composed of 2 to 6 carbon atoms and 1 to 2 heteroatoms, and may be alicyclic or aromatic cyclic. The heteroatoms are independently selected from an oxygen atom, a nitrogen atom, and a sulfur atom. The heterocyclic group is substituted by one or more R^{a}.

Specific examples thereof include an epoxy group, a 2-tetrahydrofuranyl group, a 3-tetrahydrofuranyl group, a 2-tetrahydrothiofuranyl group, a 3-tetrahydrothiofuranyl group, a c-butyloxy group, a c-hexyloxy group, a 2-tetrahydropyranyl group, a 3-tetrahydropyranyl group, a 4-tetrahydropyranyl group, a 2-tetrahydrothiopyranyl group, a 3-tetrahydrothiopyranyl group, a 4-tetrahydrothiopyranyl group, a 1,4-dioxane-2-yl group, a 1,3-dioxolan-2-yl group, a 1,3-dioxolan-2-yl group, a 2-pyrrolidinyl group, a 3-pyrrolidinyl group, a 2-N-methyl-pyrrolidinyl group, a 3-N-methyl-pyrrolidinyl group, a 2-piperidinyl group, a 3-piperidinyl group, a 2-N-methyl-piperidinyl group, a 3-N-methyl-piperidinyl group, a 4-N-methyl-piperidinyl group, a pyrrolyl group, an imidazolyl group, a pyrazolyl group, a pyridyl group, a chloropyridyl group, a pyrimidyl group, a pyrazinyl group, a furyl (furanyl) group, a thienyl group, a thiazolyl group, and a chlorothiazolyl group.

R³ represents R^{a}, a straight or branched chain C1-C6 alkyl group, a straight or branched chain C2-C6 alkenyl group, a straight or branched chain C3-C10 cycloalkyl group, a phenyl group, or a heterocyclic group.

The C1-C6 alkyl group represented by R³ may have a substituent; and examples of the substituent include one or more R^{a}; a C3-C6 cycloalkyl group which is optionally substituted with one or more R^{a}; a straight or branched chain C1-C6 alkyloxy group which is optionally substituted with one or more R^{a}; a C3-C6 cycloalkyloxy group which is optionally substituted with one or more R^{a}; a straight or branched chain C1-C6 alkylthio group which is optionally substituted with one or more R^{a}; a straight or branched chain C1-C6 alkylsulfoxy group which is optionally substituted with one or more R^{a}; a straight or branched chain C1-C6 alkylsulfonyl group which is optionally substituted with one or more R^{a}; an amino group having two substitutes independently selected from a hydrogen atom, a C1-C4 alkyl group which is substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a}, and C2 acyl group which is substituted with one or more R^{a}; an ester group which is substituted with a C1-C4 alkyl group, wherein the ester group is substituted with one or more R^{a}, and/or C1-C3 alkyloxy group substituted by one or more R^{a}, and/or a C1-C3 alkylthio group substituted with one or more R^{a}, and/or a C1-C3 alkylsulfoxy group substituted with one or more R^{a}, and/or a C1-C3 alkylsulfonyl group substituted with one or more R^{a}; a phenyl group which is substituted with one or more R^{b}; and a heterocyclic group which is substituted with one or more R^{b}, wherein the heterocyclic group has a ring composed of 2 to 6 carbon atoms and 1 to 2 heteroatoms, and may be alicyclic or aromatic cyclic, wherein the heteroatoms are independently selected from an oxygen atom, a nitrogen atom, and a sulfur atom.

Specific examples thereof include a methyl group, an ethyl group, an n-propyl group, an i-propyl group, an n-butyl group, an n-pentyl group, an n-hexyl group, an i-butyl group, an s-butyl group, a t-butyl group, a monochloromethyl group, a dichloromethyl group, a trichloromethyl group, a monofluoromethyl group, a difluoromethyl group, a trifluoromethyl group, a 2,2,2-trifluoroethyl group, a 3,3,3-trifluoropropyl group, a 1,1,2,2-tetrafluoroethyl group, a pentafluoroethyl group, a heptafluoro-n-propyl group, a 1,1,1,3,3,3-hexafluoro-2-propyl group, a heptafluoro-i-propyl group, a 1,1,1,3,3,3-hexafluoro-2-methyloxy-2-propyl group, a methyloxymethyl group, an ethyloxymethyl group, an n-propyloxymethyl group, a 2,2,2-trifluoroethyloxymethyl group, a 3,3,3-trifluoro-n-propyloxymethyl group, a 2,2,3,3,3-pentafluoro-n-propyloxymethyl group, an aminomethyl group, an N-methyl-aminomethyl group, an N,N-dimethylaminomethyl group, an aminoethyl group, an N-methylaminoethyl group, an N,N-dimethylaminoethyl group, a methyloxycarbonylmethyl group, a 1-methyloxycarbonylethyl group, an ethyloxycarbonylmethyl group, a 1-ethyloxycarbonylmethyl group, a benzyl group, a fluorobenzyl group, a pentafluorobenzyl group, a chlorobenzyl group, a dichlorobenzyl group, a nitrobenzyl group, a methylbenzyl group, a dimethylbenzyl group, a trifluoromethylbenzyl group, a methoxybenzyl group, a pyrrolylmethyl group, an imidazolylmethyl group, a pyrazolylmethyl group, a pyridylmethyl group, a chloropyridylmethyl group, a pyrimidylmethyl group, a pyrazinylmethyl group, a furyl (furanyl) methyl group, a thienylmethyl group, a thiazolylmethyl group, and a chlorothiazolylmethyl group.

The C2-C6 alkenyl group represented by R³ may have a substituent, and examples of the substituent include one or more R^{a}.

Specific examples thereof include a vinyl group, a 1-propenyl group, a 2-propenyl group, a 1-propen-2-yl group, a 3-methyl-2-butenyl group, a 2-butenyl group, a 3-chloro-2-propenyl group, a 3,3-dichloro-2-propenyl group, a 2-methyl-2-propenyl group, a 2-bromo-2-propenyl group, a 2-chloro-2-propenyl group, a 3-fluoro-2-propenyl group, a 3,3-difluoro-2-propenyl group, a 3,3-dibhloro-2-propenyl group, a 4,4,4-trifluoro-3-chloro-2-butenyl group, a 4,4,4-trifluoro-2-butenyl group, a 2,3-dichloro-2-propenyl group, and a 1,1,2-trifluoro-2-propenyl group.

The C3-C10 cycloalkyl group represented by R³ may be a single ring or a condensed ring, and may have a cross-linked structure. The C3-C10 cycloalkyl group represented by R³ may have a substituent, and examples thereof include one or more R^{a}, a straight or branched chain C1-C6 alkyl group which is optionally substituted with one or more R^{a}, a straight or branched chain C1-C6 alkyloxy group which is optionally substituted with one or more R^{a}, a straight or branched chain C1-C6 alkylthio group which is optionally substituted with one or more R^{a}, a straight or branched chain C1-C6 alkylsulfoxy group which is optionally substituted with one or more R^{a}, and a straight or branched chain C1-C6 alkylsulfonyl group which is optionally substituted with one or more R^{a}.

Specific examples thereof include a c-propyl group, a c-butyl group, a c-pentyl group, a c-hexyl group, abicyclo[2.2.1]heptyl group, abicyclo[4.2.1]heptyl group, a 1-adamantyl group, and a 2-adamantyl group.

The phenyl group represented by R³ is substituted with one or more R^{b}.

Specific examples thereof include a phenyl group, a fluorophenyl group, a pentafluorophenyl group, a chlorophenyl group, a dichlorophenyl group, a nitrophenyl group, a methylphenyl group, a dimethylphenyl group, a trifluoromethylphenyl group, and a methoxyphenyl group.

The heterocyclic group represented by R³ has a ring composed of 2 to 6 carbon atoms and 1 to 2 heteroatoms, which may be alicyclic or aromatic cyclic. The heteroatoms are independently selected from an oxygen atom, a nitrogen atom, and a sulfur atom. The heterocyclic group is substituted with one or more R^{b}.

Specific examples thereof include an epoxy group, a 2-tetrahydrofuranyl group, a 3-tetrahydrofuranyl group, a 2-tetrahydrothiofuranyl group, a 3-tetrahydrothiofuranyl group, a c-butyloxy group, a c-hexyloxy group, a 2-tetrahydropyranyl group, a 3-tetrahydropyranyl group, a 4-tetrahydropyranyl group, a 2-tetrahydrothiopyranyl group, a 3-tetrahydrothiopyranyl group, a 4-tetrahydrothiopyranyl group, a 1,4-dioxane-2-yl group, a 1,3-dioxolan-2-yl group, a 1,3-dioxolan-2-yl group, a 2-pyrrolidinyl group, a 3-pyrrolidinyl group, a 2-N-methyl-pyrrolidinyl group, a 3-N-methyl-pyrrolidinyl group, a 2-piperidinyl group, a 3-piperidinyl group, a 2-N-methyl-piperidinyl group, a 3-N-methyl-piperidinyl group, a 4-N-methyl-piperidinyl group, a pyrrolyl group, an imidazolyl group, a pyrazolyl group, a pyridyl group, a chloropyridyl group, a pyrimidyl group, a pyrazinyl group, a furyl (furanyl) group, a thienyl group, a thiazolyl group, and a chlorothiazolyl group.

Examples of R⁴ independently include R^{a}, a straight or branched chain C1-C6 alkyl group, a straight or branched chain C2-C6 alkenyl group, a straight or branched chain C2-C6 alkynyl group, a straight or branched chain C1-C6 alkyloxy group, an amino group, a straight or branched chain C1-C4 alkylthiol group, a straight or branched chain C1-C4 alkylsulfoxy group, and a straight or branched chain C1-C4 alkylsulfonyl group.

R^{a} represented by R⁴ is preferably a hydrogen atom.

The C1-C6 alkyl group represented by R⁴ is preferably a C1-C3 alkyl group. The alkyl group may have a substituent, and examples of the substituent include one or more R^{a}, a C2-C6 cycloalkyl group which is optionally substituted with one or more R^{a}, a straight or branched chain C1-C3 alkyloxy group which is optionally substituted with one or more R^{a}, a C2-C6 cycloalkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples of the C1-C6 alkyl group include a methyl group, an ethyl group, an n-propyl group, a c-propyl group, an n-butyl group, an i-butyl group, an s-butyl group, a t-butyl group, a monochloromethyl group, a dichloromethyl group, a trichloromethyl group, a monofluoromethyl group, a difluoromethyl group, a trifluoromethyl group, a 2,2,2-trifluoroethyl group, a 3,3,3-trifluoropropyl group, a 1,1,2,2-tetrafluoroethyl group, a pentafluoroethyl group, a heptafluoro-n-propyl group, a l,l,l,3,3,3-hexafluoro-2-propyl group, a heptafluoro-i-propyl group, a 1,1,1,3,3,3-hexafluoro-2-methyloxy-2-propyl group, a methyloxymethyl group, an ethyloxymethyl group, an n-propyloxymethyl group, a 2,2,2-trifluoroethyloxymethyl group, a 3,3,3-trifluoro-n-propyloxymethyl group, and a 2,2,3,3,3-pentafluoro-n-propyloxymethyl group.

The C2-C6 alkenyl group represented by each R⁴ is preferably a C2-C3 alkenyl group. The alkenyl group may have a substituent, and examples of the substituent include one or more R^{a}.

Specific examples thereof include a vinyl group, a 1-propenyl group, a 2-propenyl group, and a 1-propen-2-yl group.

The C2-C6 alkynyl group represented by each R⁴ is preferably a C2-C3 alkynyl group. The alkynyl group may have a substituent, and examples of the substituent include one or more R^{a}.

Specific examples thereof include an ethynyl group, a 1-propyn-1-yl group, and a 2-propyn-1-yl group.

The C1-C6 alkyloxy group represented by each R⁴ is preferably a C1-C4 alkyloxy group. The alkyloxy group may have a substituent, and examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include a methyloxy group, an ethyloxy group, an n-propyloxy group, an i-propyloxy group, a c-propyloxy group, an n-butyloxy group, an i-butyloxy group, an s-butyloxy group, a t-butyloxy group, n-pentyloxy, a methyloxymethyloxy group, an ethyloxymethyloxy group, a methylthiomethyloxy group, a 2-methyloxyethyloxy group, a 2-ethyloxyethyloxy group, a 2-thiomethylethyloxy group, a difluoromethyloxy group, a trifluoromethyloxy group, a chlorodifluoromethyloxy group, a 2,2,2-trifluoroethyloxy group, a 1,1,2,2-tetrafluoroethyloxy group, and a 1,1,1,3,3,3-hexafluoro-2-propyloxy group.

The amino group represented by each R⁴ has two substituents selected from a hydrogen, a C1-C4 alkyl group, and a C2-C6 acyl group.

The C1-C4 alkyl may be a straight chain or a branched chain. The C1-C4 alkyl may have a substituent, and examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

The C2-C6 acyl group may be a straight or branched chain, and may be substituted with one or more R^{a}.

Specific examples of the C1-C4 alkyl group include a methyl group, an ethyl group, a propyl group, an i-propyl group, a c-propyl group, an n-butyl group, an i-butyl group, an s-butyl group, a t-butyl group, a trichloromethyl group, a monofluoromethyl group, a difluoromethyl group, a trifluoromethyl group, and a 2,2,2-trifluoroethyl group.

Specific examples of the C2-C6 acyl group include an acetyl group, a trifluoroacetyl group, and a pivaloyl group.

The C1-C4 alkylthio group, the C1-C4 alkylsulfoxy group, and the C1-C4 alkylsulfonyl group represented by each R⁴ are preferably a C1-C3 alkylthio group, a C1-C3 alkylsulfoxy group, and a C1-C3 alkylsulfonyl group. These alkyl portion may have a substituent, and examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include a methylthio group, an ethylthio group, an n-propylthio group, an i-propylthio group, a c-propylthio group, an n-butylthio group, a methyloxymethylthio group, an ethyloxymethylthio group, a methylthiomethylthio group, a difluoromethylthio group, a trifluoromethylthio group, a chlorodifluoromethylthio group, a 2,2,2-trifluoroethylthio group, a methylsulfoxy group, an ethylsulfoxy group, an n-propylsulfoxy group, an i-propylsulfoxy group, a c-propylsulfoxy group, an n-butylsulfoxy group, a methyloxymethylsulfoxy group, an ethyloxymethylsulfoxy group, a difluoromethylsulfoxy group, a trifluoromethylsulfoxy group, a chlorodifluoromethylsulfoxy group, and a 2,2,2-trifluoroethylsulfoxy group, a methylsulfonyl group, an ethylsulfonyl group, an n-propylsulfonyl group, an i-propylsulfonyl group, a c-propylsulfonyl group, an n-butylsulfonyl group, a methyloxymethylsulfonyl group, an ethyloxymethylsulfonyl group, a difluoromethylsulfonyl group, a trifluoromethylsulfonyl group, a chlorodifluoromethylsulfonyl group, and a 2,2,2-trifluoroethylsulfonyl group.

R⁵ represents a straight or branched chain C1-C4 alkylene group, a straight or branched chain C2-C4 alkenylene group, or a straight or branched chain C2-C4 alkynylene group, which is substituted with one or more R^{a}.

Specific examples thereof include -(CH₂)₁₋₄-, -(CH₂)₀₋₂-CH(CH₃)-(CH₂)₀₋₂-, - (CH₂)₀₋₂-CH=CH-(CH₂)₀₋₂-, and -(CH₂)₀₋₂-CH≡C-(CH₂)₀₋₂-, though not limited thereto.

X is a direct bond, O, S(O)₀₋₂, NR^{c}, N(R^{c})-C(=O), N⁺(R^{c})₂, OC(=O), or OC(=O)O.

Each R^{c} is independently selected from the group consisting of R^{a}, a straight or branched chain C1-C4 alkyl groups, a straight or branched chain C2-C3 alkenyl group, a straight or branched chain C2-C3 alkynyl group, a C3-C6 cycloalkyl group, a straight or branched chain C1-C6 alkyloxy group, a straight or branched chain C1-C4 alkylthio group, a straight or branched chain C1-C4 alkylsulfoxy group, and a straight or branched chain C1-C4 alkylsulfonyl group, a phenyl group, and a heterocyclic group.

The C1-C4 alkyl group represented by each R^{c} may have a substituent, and examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include a methyl group, an ethyl group, a propyl group, an i-propyl group, a c-propyl group, an n-butyl group, an i-butyl group, an s-butyl group, a t-butyl group, a monochloromethyl group, a dichloromethyl group, a trichloromethyl group, a monofluoromethyl group, a difluoromethyl group, a trifluoromethyl group, a 2,2,2-trifluoroethyl group, a 1,1,2,2-tetrafluoroethyl group, a pentafluoroethyl group, a heptafluoro-n-propyl group, a 1,1,1,3,3,3-hexafluoro-2-propyl group, a heptafluoro-i-propyl group, a 1,1,1,3,3,3-hexafluoro-2-methyloxy-2-propyl group, a methyloxymethyl group, an ethyloxymethyl group, an n-propyloxymethyl group, a 2,2,2-trifluoroethyloxymethyl group, a 3,3,3-trifluoro-n-propyloxymethyl group, and a 2,2,3,3,3-pentafluoro-n-propyloxymethyl group.

The C2-C3 alkenyl group represented by each R^{c} may have a substituent, and examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include a vinyl group, a 1-propenyl group, a 2-propenyl group, and a 1-propen-2-yl group.

The C3-C6 cycloalkyl group represented by each R^{c} may have a substituent, and examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include a cyclopropyl group, a cyclobutyl group, a cyclopentyl group, and a cyclohexyl group.

The C2-C3 alkynyl group represented by each R^{c} may have a substituent, and examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include an ethynyl group, a 1-propyn-1-yl group, and a 2-propyn-1-yl group.

The C1-C4 alkyloxy group represented by each R^{c} may have a substituent, and examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include a methyloxy group, an ethyloxy group, an n-propyloxy group, an i-propyloxy group, a c-propyloxy group, an n-butyloxy group, an i-butyloxy group, an s-butyloxy group, a t-butyloxy group, an n-pentyloxy group, a methyloxymethyloxy group, an ethyloxymethyloxy group, a methylthiomethyloxy group, a 2-methyloxyethyloxy group, a 2-ethyloxyethyloxy group, a 2-thiomethylethyloxy group, a difluoromethyloxy group, a trifluoromethyloxy group, a chlorodifluoromethyloxy group, a 2,2,2-trifluoroethyloxy group, a 1,1,2,2-tetrafluoroethyloxy group, and a 1,1,1,3,3,3-hexafluoro-2-propyloxy group.

The C1-C4 alkyl group of the C1-C4 alkylthio group, the C1-C4 alkylsulfoxy group, and the C1-C4 alkylsulfonyl group represented by each R^{c} may have a substituent, and examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include a methylthio group, an ethylthio group, an n-propylthio group, an i-propylthio group, a c-propylthio group, an n-butylthio group, a methyloxymethylthio group, an ethyloxymethylthio group, a methylthiomethylthio group, a difluoromethylthio group, a trifluoromethylthio group, a chlorodifluoromethylthio group, a 2,2,2-trifluoroethylthio group, a methylsulfoxy group, an ethylsulfoxy group, an n-propylsulfoxy group, an i-propylsulfoxy group, a c-propylsulfoxy group, an n-butylsulfoxy group, a methyloxymethylsulfoxy group, an ethyloxymethylsulfoxy group, a difluoromethylsulfoxy group, a trifluoromethylsulfoxy group, a chlorodifluoromethylsulfoxy group, a 2,2,2-trifluoroethylsulfoxy group, a methylsulfonyl group, an ethylsulfonyl group, an n-propylsulfonyl group, an i-propylsulfonyl group, a c-propylsulfonyl group, an n-butylsulfonyl group, a methyloxymethylsulfonyl group, an ethyloxymethylsulfonyl group, a difluoromethylsulfonyl group, a trifluoromethylsulfonyl group, a chlorodifluoromethylsulfonyl group, and a 2,2,2-trifluoroethylsulfonyl group.

The phenyl group represented by each R^{c} may have a substituent, and examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include a phenyl group, a chlorophenyl group, and a methoxyphenyl group.

The heterocyclic group represented by each R^{c} has a ring composed of 2 to 6 carbon atoms and 1 to 2 heteroatoms, and may be alicyclic or aromatic cyclic. The heteroatoms are independently selected from the group consisting of a nitrogen atom, an oxygen atom, and a sulfur atom.

The heterocyclic group may have a substituent, and examples of the substituent include one or more R^{a}, a straight or branched chain C1-C3 alkyloxy group which is optionally substituted with one or more R^{a}, a straight or branched chain C1-C3 alkylthio group which is optionally substituted with one or more R^{a}, a straight or branched chain C1-C3 alkylsulfoxy group which is optionally substituted with one or more R^{a}, and a straight or branched chain C1-C3 alkylsulfonyl group which is optionally substituted with one or more R^{a}.

Specific examples thereof include an epoxy group, a 2-tetrahydrofuranyl group, a 3-tetrahydrofuranyl group, a 2-tetrahydrothiofuranyl group, a 3-tetrahydrothiofuranyl group, a c-butyloxy group, a c-hexyloxy group, a 2-tetrahydropyranyl group, a 3-tetrahydropyranyl group, a 4-tetrahydropyranyl group, a 2-tetrahydrothiopyranyl group, a 3-tetrahydrothiopyranyl group, a 4-tetrahydrothiopyranyl group, a 1,4-dioxane-2-yl group, a 1,3-dioxolan-2-yl group, a 1,3-dioxolan-2-yl group, a 2-pyrrolidinyl group, a 3-pyrrolidinyl group, a 2-N-methyl-pyrrolidinyl group, a 3-N-methyl-pyrrolidinyl group, a 2-piperidinyl group, a 3-piperidinyl group, a 2-N-methyl-piperidinyl group, a 3-N-methyl-piperidinyl group, a 4-N-methyl-piperidinyl group, a pyrrolyl group, an imidazolyl group, a pyrazolyl group, a pyridyl group, a chloropyridyl group, a pyrimidyl group, a pyrazinyl group, a furyl (furanyl) group, a thienyl group, a thiazolyl group, and a chlorothiazolyl group.

The present specification discloses all the combinations of each substituent of the compound (1).

In a preferred embodiment of the present invention, R¹ is a hydrogen atom, a halogen atom, a methyl group, or a trifluoromethyl group;
the substitution position of R¹ is at the 8-position; and
R³ represents a hydrogen atom, a cyano group, a nitro group, an SCN group, a straight or branched chain C1-C6 alkyl group, a straight or branched chain C2-C6 alkenyl group, a straight or branched chain C1-C6 alkyloxy group, a straight or branched C3-C6 cycloalkyl group, a straight or branched chain C2-C6 cycloalkyloxy group, a C2-C6 cycloalkyldioxy group, a C2-C6 cycloalkylthio group, a C2-C6 cycloalkylsulfonyl group, a C2-C6 cycloalkylsulfone group, a C2-C6 cycloalkylamino group, a straight or branched chain C1-C4 alkylthio group, a straight or branched chain C1-C4 alkylsulfoxy group, a straight or branched chain C1-C4 alkylsulfonyl group, a straight or branched chain C1-C6 alkylcyano group, a straight or branched chain C1-C6 alkylamino group having two substituents selected from a hydrogen, a straight or branched chain C1-C4 alkyl group and a C2 acyl group, a phenyl group which is substituted with up to three R^{c}, a pyridyl group, a pyrimidyl group, or an alicyclic heterocyclic group.

More preferably, R¹ is a hydrogen atom or a methyl group;
the substitution position of R¹ is at the 8-position;
R² is a phenyl group which is substituted with up to two R^{b};
R^{b} is independently selected from a hydrogen atom, a halogen atom, a pentafluorosulfanyl group, a methyl group, an ethyl group, an n-propyl group, an i-propyl group, a c-propyl group, an n-butyl group, an i-butyl group, an s-butyl group, a t-butyl group, an-n-pentyl group, a trifluoromethyl group, a methyloxy group, an ethyloxy group, an n-propyloxy group, an i-propyloxy group, a c-propyloxy group, an n-butyloxy group, an i-butyloxy group, an s-butyloxy group, a t-butyloxy group, an n-pentyloxy group, a trifluoromethyloxy group, and a 2,2,2-trifluoroethoxy group;
R³ is a methyl group, an ethyl group, an n-propyl group, an i-propyl group, an n-butyl group, an n-pentyl group, an n-hexyl group, an i-butyl group, an s-butyl group, a t-butyl group, a monochloromethyl group, a dichloromethyl group, a trichloromethyl group, a monofluoromethyl group, a difluoromethyl group, a trifluoromethyl group, a 2,2,2-trifluoroethyl group, a 3,3,3-trifluoropropyl group, a 1,1,2,2-tetrafluoroethyl group, a pentafluoroethyl group, a methyloxymethyl group, an ethyloxymethyl group, an n-propyloxymethyl group, a 2,2,2-trifluoroethyloxymethyl group, a 3,3,3-trifluoro-n-propyloxymethyl group, a 2,2,3,3,3-pentafluoro-n-propyloxymethyl group, a 1-methyloxycarbonylethyl group, a 1-propenyl group, a 2-propenyl group, a 1-propen-2-yl group, a 3-methyl-2-butenyl group, a 2-butenyl group, a 3-chloro-2-propenyl group, a 3,3-dichloro-2-propenyl group, a 2-methyl-2-propenyl group, a 2-bromo-2-propenyl group, a 2-chloro-2-propenyl group, a 3-fluoro-2-propenyl group, a 3,3-difluoro-2-propenyl group, a 3,3-dibhloro-2-propenyl group, a 4,4,4-trifluoro-3-chloro-2-butenyl group, a 4,4,4-trifluoro-2-butenyl group, a 2,3-dichloro-2-propenyl group, a 1,1,2-trifluoro-2-propenyl group, a methylthio group, an ethylthio group, an n-propylthio group, an i-propylthio group, a c-propylthio group, an n-butylthio group, a methyloxymethylthio group, a difluoromethylthio group, a trifluoromethylthio group, a chlorodifluoromethylthio group, a 2,2,2-trifluoroethylthio group, a methylsulfoxy group, an ethylsulfoxy group, an n-propylsulfoxy group, an i-propylsulfoxy group, a c-propylsulfoxy group, an n-butylsulfoxy group, a methyloxymethylsulfoxy group, an ethyloxymethylsulfoxy group, a difluoromethylsulfoxy group, a trifluoromethylsulfoxy group, a chlorodifluoromethylsulfoxy group, a 2,2,2-trifluoroethylsulfoxy group, a methylsulfonyl group, an ethylsulfonyl group, an n-propylsulfonyl group, an i-propylsulfonyl group, a c-propylsulfonyl group, an n-butylsulfonyl group, a methyloxymethylsulfonyl group, an ethyloxymethylsulfonyl group, a difluoromethylsulfonyl group, a trifluoromethylsulfonyl group, a chlorodifluoromethylsulfonyl group, a 2,2,2-trifluoroethylsulfonyl group, a cyanomethyl group, a cyanoethyl group, a cyanopropyl group, a methyloxy group, an ethyloxy group, an n-propyloxy group, an i-propyloxy group, an n-butyloxy group, an i-butyloxy group, an s-butyloxy group, a t-butyloxy group, an n-pentyloxy group, a methyloxymethyloxy group, an ethyloxymethyloxy group, a methylthiomethyloxy group, a 2-methyloxyethyloxy group, a 2-ethyloxyethyloxy group, a 2-thiomethylethyloxy group, a difluoromethyloxy group, a trifluoromethyloxy group, a chlorodifluoromethyloxy group, a 2,2,2-trifluoroethyloxy group, a 1,1,2,2-tetrafluoroethyloxy group, a 1,1,1,3,3,3-hexafluoro-2-propyloxy group, a 1-methyloxyethyl group, a 1-ethyloxyethyl group, a 1-i-propyloxy-ethyl group, a 1-c-hexyloxy-ethyl group, a 1-c-propyloxy-ethyl group, a 1-t-butyloxy-ethyl group, a 1-(2-chloroethoxy)-ethyl group, a c-propyloxy group, a c-butyl group, a c-pentyl group, a c-hexyl group, a c-propyl group, a 2-tetrahydrofuranyl group, a 3-tetrahydrofuranyl group, a 2-tetrahydrothiofuranyl group, a 3-tetrahydrothiofuranyl group, a c-butyloxy group, a c-hexyloxy group, a 2-tetrahydropyranyl group, a 3-tetrahydropyranyl group, a 4-tetrahydropyranyl group, a 2-tetrahydrothiopyranyl group, a 3-tetrahydrothiopyranyl group, a 4-tetrahydrothiopyranyl group, a 1,4-dioxan-2-yl group, a 1,3-dioxolan-2-yl group, a 1,3-dioxolan-2-yl group, 2-tetramethylene sulfoxide, 3-tetramethylene sulfoxide, 2-sulfolane, 3-sulfolane, 2-tetrahydrothiopyran-1-oxide, 3-tetrahydrothiopyran-1-oxide, 4-tetrahydrothiopyran-1-oxide, 2-tetrahydrothiopyran-1,1 -dioxide, 3-tetrahydrothiopyran-1,1 -dioxide, 4-tetrahydrothiopyran-1,1-dioxide, a 2-pyrrolidinyl group, a 3-pyrrolidinyl group, a 2-N-methyl-pyrrolidinyl group, a 3-N-methyl-pyrrolidinyl group, a 2-piperidinyl group, a 3-piperidinyl group, a 2-N-methyl-piperidinyl group, a 3-N-methyl-piperidinyl group, a 4-N-methyl-piperidinyl group, an amino group, an N-methyl-amino group, an N,N-dimethyl-amino group, an N-acetyl-amino group, an N-acetyl-N-methyl-amino group, an N-trifluoroacetyl-amino group, an N-trifluoroacetyl-N-methyl-amino group, an aminomethyl group, an N-methyl-aminomethyl group, an N,N-dimethylaminomethyl group, an aminoethyl group, an N-methylaminoethyl group, an N,N-dimethylaminoethyl group, a phenyl group, or a pyridyl group; wherein
the phenyl group and the pyridyl groups are substituted with up to two substituents independently selected from a hydrogen atom, a nitro group, a halogen atom, a trifluoromethyl group, a methyl group, a methyloxy group, and a trifluoromethyloxy group;
R⁴ is a hydrogen atom, a methyl group, an ethyl group, an amino group, a chloro atom, a trifluoromethyl group, an N-methyl-amino group, or an N,N-dimethyl-amino group;
R⁵ is a C1-C2 alkylene group, a C2 alkenylene group, or a C2 alkynylene group; and
X is O, OC(=O), or OC(=O)O.

More preferably, R¹ is a hydrogen atom;
R² is a phenyl group which is substituted with up to two substituents independently selected from a hydrogen atom, a halogen atom, a methyl group, an ethyl group, an n-propyl group, an i-propyl group, a c-propyl group, an n-butyl group, an i-butyl group, a t-butyl group, a trifluoromethyl group, a methyloxy group, an ethyloxy group, and a trifluoromethyloxy group;
R³ is a methyl group, an ethyl group, an n-propyl group, an i-propyl group, an n-butyl group, an i-butyl group, a monochloromethyl group, a monofluoromethyl group, a difluoromethyl group, a trifluoromethyl group, a 2,2,2-trifluoroethyl group, a 3,3,3-trifluoropropyl group, an n-propyloxymethyl group, a 2,2,2-trifluoroethyloxymethyl group, a 1-propenyl group, a 2-propenyl group, a 3-methyl-2-butenyl group, a 2-butenyl group, a 3-chloro-2-propenyl group, a 3,3-dichloro-2-propenyl group, a 2-methyl-2-propenyl group, a 2-bromo-2-propenyl group, a 2-chloro-2-propenyl group, a 3-fluoro-2-propenyl group, a 3,3-difluoro-2-propenyl group, a 3,3-dibhloro-2-propenyl group, a 4,4,4-trifluoro-3-chloro-2-butenyl group, a 4,4,4-trifluoro-2-butenyl group, a 2,3-dichloro-2-propenyl group, a 1,1,2-trifluoro-2-propenyl group, a methylthio group, an ethylthio group, an n-propylthio group, an i-propylthio group, a difluoromethylthio group, a trifluoromethylthio group, a 2,2,2-trifluoroethylthio group, a methylsulfoxy group, an ethylsulfoxy group, an n-propylsulfoxy group, an i-propylsulfoxy group, a methyloxymethylsulfoxy group, an ethyloxymethylsulfoxy group, a difluoromethylsulfoxy group, a trifluoromethylsulfoxy group, a 2,2,2-trifluoroethylsulfoxy group, a methylsulfonyl group, an ethylsulfonyl group, an n-propylsulfonyl group, an i-propylsulfonyl group, a difluoromethylsulfonyl group, a trifluoromethylsulfonyl group, a 2,2,2-trifluoroethylsulfonyl group, a cyanomethyl group, a cyanoethyl group, a cyanopropyl group, a methyloxy group, an ethyloxy group, an n-propyloxy group, an i-propyloxy group, an n-butyloxy group, an i-butyloxy group, an s-butyloxy group, a t-butyloxy group, an n-pentyloxy group, a methyloxymethyloxy group, an ethyloxymethyloxy group, a methylthiomethyloxy group, a 2-methyloxyethyloxy group, a 2-ethyloxyethyloxy group, a 2-thiomethylethyloxy group, a difluoromethyloxy group, a trifluoromethyloxy group, a 2,2,2-trifluoroethyloxy group, a 1-methyloxyethyl group, a 1-ethyloxyethyl group, a 1-i-propyloxy-ethyl group, a 1-c-hexyloxy-ethyl group, a 1-c-propyloxy-ethyl group, a 1-t-butyloxy-ethyl group, a 1-(2-chloroethoxy)-ethyl group, a c-propyloxy group, a c-butyl group, a c-pentyl group, a c-hexyl group, a c-propyl group, a 2-tetrahydrofuranyl group, a 3-tetrahydrofuranyl group, a 2-tetrahydrothiofuranyl group, a 3-tetrahydrothiofuranyl group, a c-butyloxy group, a c-hexyloxy group, a 2-tetrahydropyranyl group, a 3-tetrahydropyranyl group, a 4-tetrahydropyranyl group, a 2-tetrahydrothiopyranyl group, a 3-tetrahydrothiopyranyl group, a 4-tetrahydrothiopyranyl group, a 1,4-dioxan-2-yl group, a 1,3-dioxolan-2-yl group, a 1,3-dioxolan-2-yl group, 2-tetramethylene sulfoxide, 3-tetramethylene sulfoxide, 2-sulfolane, 3-sulfolane, 2-tetrahydrothiopyran-1-oxide, 3-tetrahydrothiopyran-1-oxide, 4-tetrahydrothiopyran-1 -oxide, 2-tetrahydrothiopyran-1,1-dioxide, 3-tetrahydrothiopyran-1,1-dioxide, 4-tetrahydrothiopyran-1,1-dioxide, a 2-pyrrolidinyl group, a 3-pyrrolidinyl group, a 2-N-methyl-pyrrolidinyl group, a 3-N-methyl-pyrrolidinyl group, a 2-piperidinyl group, a 3-piperidinyl group, a 2-N-methyl-piperidinyl group, a 3-N-methyl-piperidinyl group, a 4-N-methyl-piperidinyl group, an amino group, an N-methyl-amino group, an N,N-dimethyl-amino group, an N-acetyl-amino group, an N-acetyl-N-methyl-amino group, an N-trifluoroacetyl-amino group, N-trifluoroacetyl-N-methyl-amino group, an aminomethyl group, an N-methyl-aminomethyl group, an N,N-dimethylaminomethyl group, an aminoethyl group, an N-methylaminoethyl group, an N,N-dimethylaminoethyl group, a phenyl group, or a pyridyl group; wherein
the phenyl group and the pyridyl group are substituted with up to two substituents which are independently selected from a hydrogen atom, a nitro group, a halogen atom, a trifluoromethyl group, a methyl group, a methyloxy group, and a trifluoromethyloxy group, and preferably substituted with up to two substituents which are independently selected from a hydrogen atom, a nitro group, a fluorine atom, a chlorine atom, a trifluoromethyl group, a methyl group and a trifluoromethyloxy group;
R⁴ is a hydrogen atom, a methyl group, an amino group, an N-methyl-amino group, or an N,N-dimethyl-amino group;
R⁵ is an ethylene group; and
X is O, OC(=O), C(=O)O, or OC(=O)O.

The mesoionic compounds of the present invention may be a free base in a free form, or may be in a form of acid addition salt with a suitable acid. Examples of the specific acid addition salts include inorganic acids such as hydrochlorides, sulfates, nitrates, and phosphates, carboxylic acid salts such as formate, acetate, oxalate, citrate, succinate, maleate, fumarate, tartrate, lactate, benzoate, and phthalate, and sulfonic acid salts such as mesylate, tosylate, and benzenesulfonate.

Further, regarding the compound (1), specific examples of the following formulas (i) to (xii) are shown in Table 1 to Table 12, though the present invention is not limited thereto.

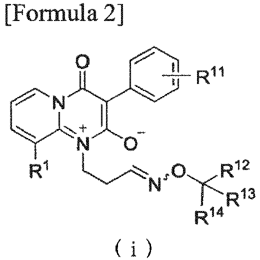

**[Table 1-1]**

| No. | R¹ | R¹¹ | R¹² | R¹³ | R¹⁴ |
|---|---|---|---|---|---|
| i-1 | H | 3-CF₃ | Me | H | H |
| i-2 | H | 3-CF₃ | Et | H | H |
| i-3 | H | 3-CF₃ | iPr | H | H |
| i-4 | H | 3-CF₃ | nPr | H | H |
| i-5 | H | 3-CF₃ | nBu | H | H |
| i-6 | H | 3-CF₃ | OMe | H | H |
| i-7 | H | 3-CF₃ | OMe | Me | H |
| i-8 | H | 3-CF₃ | OMe | Me | Me |
| i-9 | H | 3-CF₃ | OEt | H | H |
| i-10 | H | 3-CF₃ | OEt | Me | H |
| i-11 | H | 3-CF₃ | OiPr | Me | H |
| i-12 | H | 3-GF₃ | CN | H | H |
| i-13 | H | 3-CF₃ | CN | Me | H |
| i-14 | H | 3-CF₃ | CN | Me | Me |
| i-15 | H | 3-CF₃ | CH₂CN | H | H |
| i-16 | H | 3-CF₃ | CH₂CN | Me | H |
| i-17 | H | 3-CF₃ | CH₂CN | Me | Me |
| i-18 | H | 3-CF₃ | SMe | H | H |
| i-19 | H | 3-CF₃ | SMe | Me | H |
| i-20 | H | 3-CF₃ | SMe | Me | Me |
| i-21 | H | 3-CF₃ | SOMe | H | H |
| i-22 | H | 3-CF₃ | SO₂Me | H | H |
| i-23 | H | 3-CF₃ | CH₂OMe | H | H |
| i-24 | H | 3-CF₃ | CHa₂OMe | Me | H |
| i-25 | H | 3-CF₃ | CH₂SMe | H | H |
| i-26 | H | 3-CF₃ | CH₂SMe | Me | H |
| i-27 | H | 3-CF₃ | CH₂SOMe | H | H |
| i-28 | H | 3-CF₃ | CH₂SO₂Me | H | H |
| i-29 | H | 3-CF₃ | CF₃ | H | H |

**[Table 1-2]**

| | | | | | |
|---|---|---|---|---|---|
| i-30 | H | 3-CF₃ | CF₃ | Me | H |
| i-31 | Me | 3-CF₃ | CF₃ | H | H |
| i-32 | H | 3-CF₃ | CF₃ | Me | Me |
| i-33 | H | 3-CF₃ | CH₂CF₃ | H | H |
| i-34 | Me | 3-CF₃ | CH₂CF₃ | H | H |
| i-35 | H | 3-CF₃ | CF₂CF₃ | H | H |
| i-36 | H | 3-CF₃ | CH₂CH₂CF₃ | H | H |
| i-37 | H | 3-CF₃ | Me | Me | H |
| i-38 | H | 3-CF₃ | Me | Me | Me |
| i-39 | H | 3-CF₃ | CO₂Me | H | H |
| i-40 | H | 3-CF₃ | CO₂Me | Me | H |
| i-41 | H | 3-CF₃ | CH₂Cl | H | H |
| i-42 | H | 3-CF₃ | CH₂CH₂Cl | H | H |
| i-43 | H | 3,5-diCl | OEt | H | H |
| i-44 | H | 3,5-diCl | OEt | Me | H |
| i-45 | H | 3,5-diCl | OiPr | Me | H |
| i-46 | H | 3,5-diCl | Me | H | H |
| i-47 | H | 3,5-diCl | Et | H | H |
| i-48 | H | 3,5-diCl | nPr | H | H |
| i-49 | H | 3,5-diCl | OMe | H | H |
| i-50 | H | 3,5-diCl | OMe | Me | H |
| i-51 | H | 3,5-diCl | OMe | Me | Me |
| i-52 | H | 3,5-diCl | OEt | H | H |
| i-53 | H | 3,5-diCl | OEt | Me | H |
| i-54 | H | 3,5-diCl | OiPr | Me | H |
| i-55 | H | 3,5-diCl | CN | H | H |
| i-56 | H | 3,5-diCl | CN | Me | H |
| i-57 | Me | 3,5-diCl | CN | H | H |
| i-58 | H | 3,5-diCl | CH₂CN | H | H |
| i-59 | Me | 3,5-diCl | CH₂CN | H | H |

**[Table 1-3]**

| | | | | | |
|---|---|---|---|---|---|
| i-60 | H | 3,5-diCl | SMe | H | H |
| i-61 | H | 3,5-diCl | SMe | Me | H |
| i-62 | Me | 3,5-diCl | SMe | H | H |
| i-63 | H | 3,5-diCl | SOMe | H | H |
| i-64 | H | 3,5-diCl | SO₂Me | H | H |
| i-65 | Me | 3,5-diCl | SO₂Me | H | H |
| i-66 | H | 3,5-diCl | CH₂SMe | H | H |
| i-67 | H | 3,5-diCl | CH₂SMe | Me | H |
| i-68 | Me | 3,5-diCl | CH₂SMe | H | H |
| i-69 | H | 3,5-diCl | CH₂SOMe | H | H |
| i-70 | Me | 3,5-diCl | CH₂SOMe | H | H |
| i-71 | H | 3,5-diCl | CH₂SO₂Me | H | H |
| i-72 | Me | 3,5-diCl | CH₂SO₂Me | H | H |
| i-73 | H | 3,5-diCl | CF₃ | H | H |
| i-74 | H | 3,5-diCl | CF₃ | Me | H |
| i-75 | Me | 3,5-diCl | CF₃ | H | H |
| i-76 | H | 3,5-diCl | CH₂CF₃ | H | H |
| i-77 | Me | 3,5-diCl | CH₂CF₃ | H | H |
| i-78 | H | 3,5-diCl | CH₂CH₂CF₃ | H | H |
| i-79 | Me | 3,5-diCl | CH₂CH₂CF₃ | H | H |
| i-80 | H | 3,5-diCl | Me | Me | H |
| i-81 | Me | 3,5-diCl | Me | Me | Me |
| i-82 | H | 3,5-diCl | CH₂SMe | H | H |
| i-83 | H | 3-CF₃ | CH₂SMe | H | H |
| i-84 | H | 3,5-diCl | SMe | H | H |
| i-85 | H | 3-OMe | CH₂SMe | H | H |
| i-86 | H | 3-OMe | SMe | H | H |
| i-87 | H | 3-CF₃ | CH₂CF₃ | H | H |
| i-88 | H | 3-CF₃ | Me | Et | H |
| i-89 | H | 3-CF₃ | nPr | H | H |

**[Table 1-4]**

| | | | | | |
|---|---|---|---|---|---|
| i-90 | H | H | OEt | Me | H |
| i-91 | H | 3-Me | OEt | Me | H |
| i-92 | H | 3-CF₃ | CH₂*i*Pr | H | H |
| i-93 | H | 3-CF₃ | *n*Pr | Me | H |
| i-94 | H | H | *n*Bu | H | H |
| i-95 | H | H | *n*Pr | H | H |
| i-96 | H | 3,5-diCl | CH₂Cl | H | H |
| i-97 | H | 3,5-diCl | Me | Me | Me |
| i-98 | H | 3-CF₃ | OCH₂CH₂Cl | Me | H |
| i-99 | H | H | OCH₂CH₂Cl | Me | H |
| i-100 | H | 3-F | OMe | H | H |
| i-101 | H | H | OMe | Me | H |
| i-102 | H | 3-F | OMe | Me | H |
| i-103 | H | H | OMe | H | H |
| i-104 | H | H | CH₂Cl | H | H |
| i-105 | H | 4-OMe | OMe | H | H |
| i-106 | H | 4-F | OMe | H | H |
| i-107 | H | 4-Me | OMe | H | H |
| i-108 | H | 3-F | OEt | Me | H |
| i-109 | H | 4-F | OEt | Me | H |
| i-110 | H | 3-Cl | OEt | Me | H |
| i-111 | H | 3-Cl | CH₂Cl | H | H |

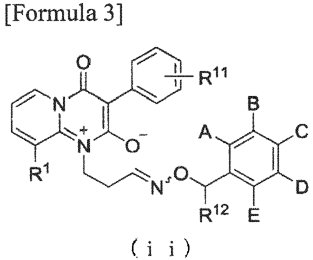

**[Table 2]**

| No. | R¹ | R¹¹ | R¹² | A | B | C | D | E |
|---|---|---|---|---|---|---|---|---|
| ii-1 | H | 3-CF₃ | H | H | H | H | H | H |
| ii-2 | H | 3-CF₃ | H | Cl | H | H | H | H |
| ii-3 | H | 3-CF₃ | H | Cl | H | H | H | Cl |
| ii-4 | H | 3-CF₃ | H | H | Cl | H | H | H |
| ii-5 | H | 3-CF₃ | H | I | H | H | H | H |
| ii-6 | H | 3-CF₃ | H | H | H | NO₂ | H | H |
| ii-7 | H | 3-CF₃ | H | F | F | F | F | F |
| ii-8 | H | 3-CF₃ | Me | H | H | H | H | H |
| ii-9 | H | 3-CF₃ | CF₃ | H | Cl | H | Cl | H |
| ii-10 | H | 3-CF₃ | CF₂H | H | Cl | H | Cl | H |
| ii-11 | H | 3-CF₃ | CH₂F | H | Cl | H | Cl | H |
| ii-12 | H | 3-CF₃ | Me | H | Cl | H | Cl | H |
| ii-13 | H | 3-CF₃ | CF₃ | H | CF₃ | H | Cl | H |
| ii-14 | H | 3-CF₃ | CF₂H | H | CF₃ | H | Cl | H |
| ii-15 | H | 3-CF₃ | CH₂F | H | CF₃ | H | Cl | H |
| ii-16 | H | 3-CF₃ | Me | H | CF₃ | H | Cl | H |
| ii-17 | H | 3-CF₃ | Me | H | CF₃ | H | CF₃ | H |
| ii-18 | H | 3-CF₃ | H | H | H | H | Cl | H |
| ii-19 | H | H | Me | H | H | H | H | H |
| ii-20 | H | 3-Cl | Me | H | H | H | H | H |

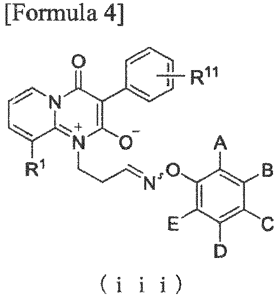

**[Table 3]**

| No. | R¹ | R¹¹ | A | B | C | D | E |
|---|---|---|---|---|---|---|---|
| iii-1 | H | 3-CF₃ | H | H | H | H | H |
| iii-2 | H | 3-CF₃ | F | H | H | H | H |
| iii-3 | H | 3-CF₃ | Cl | H | H | H | H |
| iii-4 | H | 3-CF₃ | Me | H | H | H | H |
| iii-5 | H | 3-CF₃ | H | Me | H | H | H |
| iii-6 | H | 3-CF₃ | H | CF₃ | H | H | H |
| iii-7 | H | 3-CF₃ | H | NO₂ | H | H | H |
| iii-8 | H | 3-CF₃ | H | Cl | H | H | H |
| iii-9 | H | 3-CF₃ | H | Cl | H | Cl | H |
| iii-10 | H | 3-CF₃ | H | H | Cl | H | H |
| iii-11 | H | 3-CF₃ | H | H | OCF₃ | H | H |
| iii-12 | H | 3-CF₃ | H | F | H | H | H |
| iii-13 | H | 3-CF₃ | H | H | F | H | H |
| iii-14 | H | 3-CF₃ | F | H | H | H | F |
| iii-15 | H | 3-OMe | H | CF₃ | H | H | H |

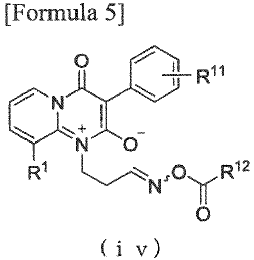

**[Table 4-1]**

| No. | R¹ | R¹¹ | R¹² |
|---|---|---|---|
| iv-1 | H | 3-CF3 | tBu |
| iv-2 | Me | 3-CF₃ | tBu |
| iv-3 | H | 3-Me | tBu |
| iv-4 | H | 3-OMe | tBu |
| iv-5 | H | 3-Cl | tBu |
| iv-6 | H | 3-OCF₃ | tBu |
| iv-7 | H | 3,5-diCl | tBu |
| iv-8 | H | H | tBu |
| iv-9 | H | 3-CF₃ | OMe |
| iv-10 | H | 3-Me | OMe |
| iv-11 | H | 3-OMe | OMe |
| iv-12 | H | 3-Cl | OMe |
| iv-13 | H | 3-OCF₃ | OMe |
| iv-14 | H | 3,5-diCl | OMe |
| iv-15 | H | H | OMe |
| iv-16 | H | 3-CF₃ | OEt |
| iv-17 | H | 3-Me | OEt |
| iv-18 | H | 3-OMe | OEt |
| iv-19 | H | 3-Cl | OEt |
| iv-20 | H | 3-OCF₃ | OEt |
| iv-21 | H | 3,5-diCl | OEt |
| iv-22 | H | H | OEt |
| iv-23 | H | 3-CF₃ | cPr |
| iv-24 | H | 3-Me | cPr |
| iv-25 | H | 3-OMe | cPr |
| iv-26 | H | 3-Cl | cPr |
| iv-27 | H | 3-OCF₈ | cPr |
| iv-28 | H | 3,5-diCl | cPr |
| iv-29 | H | H | cPr |

**[Table 4-2]**

| | | | |
|---|---|---|---|
| iv-30 | H | 3-CF₃ | 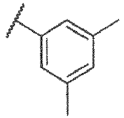 |
| iv-31 | H | 3-CF₃ | 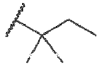 |
| iv-32 | H | 3-CF₃ | 1-adamantyl |

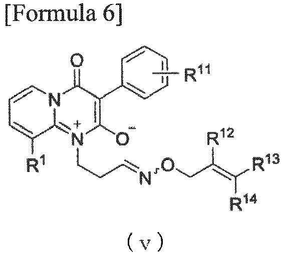

**[Table 5-1]**

| No. | R¹ | R¹¹ | R¹² | R¹³ | R¹⁴ |
|---|---|---|---|---|---|
| v-1 | H | 3-CF₃ | H | H | H |
| v-2 | H | 3-CF₃ | Cl | H | H |
| v-3 | H | 3-OCF₃ | Cl | H | H |
| v-4 | H | 3-CF₃ | Br | H | H |
| v-5 | H | 3-CF₃ | H | Cl | H |
| v-6 | H | 3-CF₃ | H | H | Cl |
| v-7 | H | 3-OMe | H | H | Cl |
| v-8 | H | 3-OCF₃ | H | H | Cl |
| v-9 | H | 3-Me | H | H | Cl |
| v-10 | H | 3,5-diMe | H | H | Cl |
| v-11 | H | H | H | H | Cl |
| v-12 | Me | 3-CF₃ | H | H | Cl |
| v-13 | H | 3-CF₃ | H | Me | Me |
| v-14 | H | 3,5-diCl | H | Me | Me |
| v-15 | H | 3,5-diMe | H | Me | Me |
| v-16 | H | 3-Cl | H | Me | Me |
| v-17 | H | 3,5-diF | H | Me | Me |
| v-18 | H | H | H | Me | Me |
| v-19 | H | 3-CF₃ | H | Cl | Cl |
| v-20 | H | 3-CF₃ | Cl | Cl | H |
| v-21 | H | 3-CF₃ | Cl | H | Cl |
| v-22 | H | 3-CF₃ | H | Cl | Cl |
| v-23 | H | 3,5-diCl | H | Cl | Cl |
| v-24 | H | 3,5-diCl | H | Cl | Me |
| v-25 | H | 3,5-diCl | H | H | Cl |
| v-26 | H | 3,5-diCl | H | Cl | H |
| v-27 | H | 3-CF₃ | H | F | F |
| v-28 | H | 3-Cl | H | F | F |
| v-29 | H | H | H | F | F |

**[Table 5-2]**

| | | | | | |
|---|---|---|---|---|---|
| v-30 | H | 3,5-diCl | H | F | F |
| v-31 | H | 3-OMe | H | F | F |
| v-32 | H | 3-Me | H | F | F |
| v-33 | H | 3-OCF₃ | H | F | F |
| v-34 | H | 3-F | H | F | F |
| v-35 | H | 3-Br | H | F | F |
| v-36 | H | 3-CF₃ | F | F | F |
| v-37 | H | 3-Cl | F | F | F |
| v-38 | H | H | F | F | F |
| v-39 | H | 3,5-diCl | F | F | F |
| v-40 | H | 3-OMe | F | F | F |
| v-41 | H | 3-Me | F | F | F |
| v-42 | H | 3-OCF₃ | F | F | F |
| v-43 | H | 3-F | F | F | F |
| v-44 | H | 3-Br | F | F | F |
| v-45 | H | 3,5-diMe | H | Cl | Cl |
| v-46 | H | H | H | Me | Me |
| v-47 | H | 3-Cl | H | Me | Me |
| v-48 | H | 3,5-diF | H | Cl | Cl |
| v-49 | H | 3,5-diCl | Me | H | H |
| v-50 | H | 3,5-diCl | H | Cl | H |
| v-51 | H | 3,5-diCl | H | Me | H |
| v-52 | H | H | H | H | Cl |
| v-53 | Me | 3,5-diCl | H | H | Cl |
| v-54 | H | 3-Cl | H | H | Cl |
| v-55 | H | 3-F | H | H | Cl |
| v-56 | H | 4-Cl | H | H | Cl |
| v-57 | H | 4-F | H | H | Cl |
| v-58 | H | 4-OMe | H | H | Cl |
| v-59 | H | 2,4-diCl | H | Me | Me |
| v-60 | H | 2,4-diF | H | Me | Me |

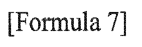

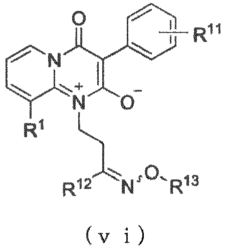

**[Table 6-1]**

| No. | R¹ | R¹¹ | R¹² | R¹³ |
|---|---|---|---|---|
| vi-1 | H | 3-CF₃ | H | H |
| vi-2 | H | 3-CF₃ | H | Me |
| vi-3 | Me | 3-CF₃ | H | H |
| vi-4 | Me | 3-CF₃ | H | Me |
| vi-5 | H | 3,5-diCl | H | H |
| vi-6 | H | 3-OMe | H | Me |
| vi-7 | Me | 3,5-diCl | H | Me |
| vi-8 | Me | 3,5-diCl | H | H |
| vi-9 | Me | 3-Me | H | H |
| vi-10 | Me | 3-Me | H | Me |
| vi-11 | H | 3-CF₃ | Me | Me |
| vi-12 | H | 3-CF₃ | Me | Me* |
| vi-13 | H | 3-CF₃ | NH₂ | H |
| vi-14 | H | 3-CF₃ | NH₂ | COtBu |
| vi-15 | H | 3-CF₃ | Cl | H |
| vi-16 | H | 3-CF₃ | Cl | Me |
| vi-17 | H | H | H | H |
| vi-18 | H | 3-Me | H | H |
| vi-19 | H | 3-CF₃ | Me | 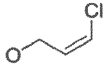 |
| vi-20 | H | 3-CF₃ | Me | 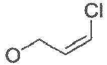 |
| vi-21 | H | 3,5-diCl | Me | 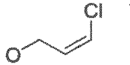 |
| vi-22 | H | 3,5-diCl | Me | 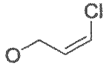 |
| vi-23 | Me | 3-CF₃ | H | CH₂OMe |
| vi-24 | H | 3,5-diCl | Me | CH₂OMe |
| vi-25 | H | 3,5-diCl | Me | CHaOMe |

**[Table 6-2]**

| | | | | |
|---|---|---|---|---|
| vi-26 | H | 3,5-diCl | Me | 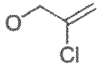 |
| vi-27 | H | 3,5-diCl | Me | 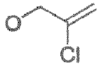 |
| vi-28 | H | 3-Me | H | Me |
| vi-29 | H | 3-Cl | H | H |
| vi-30 | H | 3,5-diF | H | H |
| vi-31 | H | 3-OMe | H | H |
| vi-32 | H | 3-F | H | H |
| vi-33 | H | 3,5-diMe | H | H |
| vi-34 | H | 3-OCF₃ | H | H |
| vi-35 | H | 4-Me | H | H |
| vi-36 | H | 4-F | H | H |
| vi-37 | H | 4-OMe | H | H |
| vi-38 | H | H | H | 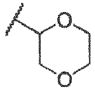 |
| vi-39 | H | 3-CF₃ | H | 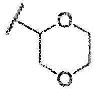 |
| vi-40 | H | H | H | 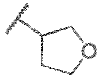 |
| vi-41 | H | 3-CF₃ | H | 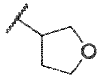 |
| vi-42 | H | H | H | 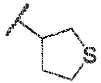 |

**[Table 6-3]**

| | | | | |
|---|---|---|---|---|
| vi-43 | H | H | H | 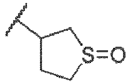 |
| vi-44 | H | H | H | 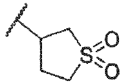 |
| vi-45 | H | 3-Br | H | H |
| vi-46 | H | 2,4-diF | H | H |
| vi-47 | H | 3-I | H | H |
| vi-48 | H | 3-CN | H | H |
| vi-49 | H | 2,4-diCl | H | H |
| vi-50 | H | 2,5-diF | H | H |

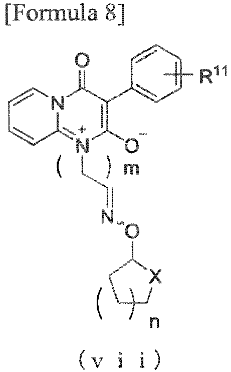

**[Table 7-1]**

| No. | R¹¹ | n | X | m |
|---|---|---|---|---|
| vii-1 | 3-CF₃ | 1 | CH₂ | 2 |
| vii-2 | 3-CF₃ | 1 | O | 2 |
| vii-3 | 3-CF₃ | 2 | O | 2 |
| vii-4 | H | 1 | O | 2 |
| vii-5 | 3-OCF₃ | 1 | O | 2 |
| vii-6 | 3-Me | 1 | O | 2 |
| vii-7 | 3-OMe | 1 | O | 2 |
| vii-8 | 3-Cl | 1 | O | 2 |
| vii-9 | 2-Cl | 1 | O | 2 |
| vii-10 | 4-Cl | 1 | O | 2 |
| vii-11 | 3-CF₃ | 1 | O | 1 |
| vii-12 | 3-CF₃ | 1 | O | 3 |
| vii-13 | 3,5-diCl | 1 | O | 2 |
| vii-14 | 2,4-diCl | 1 | O | 2 |
| vii-15 | 3-Me | 2 | O | 2 |
| vii-16 | 4-Cl | 2 | O | 2 |
| vii-17 | H | 2 | O | 2 |
| vii-18 | H | 1 | S | 2 |
| vii-19 | H | 2 | S | 2 |
| vii-20 | H | 1 | SO | 2 |
| vii-21 | H | 2 | SO | 2 |
| vii-22 | H | 1 | SO₂ | 2 |
| vii-23 | H | 2 | SO₂ | 2 |
| vii-24 | H | 1 | NH | 2 |
| vii-25 | H | 2 | NH | 2 |
| vii-26 | H | 1 | NMe | 2 |
| vii-27 | H | 2 | NMe | 2 |
| vii-28 | 4-Me | 1 | O | 2 |
| vii-29 | 4-F | 1 | O | 2 |

**[Table 7-2]**

| | | | | |
|---|---|---|---|---|
| vii-30 | 4-OMe | 1 | O | 2 |
| vii-31 | 3-CF₃ | 2 | CH₂ | 2 |
| vii-32 | H | 2 | CH₂ | 2 |
| vii-33 | 3-Me | 2 | CH₂ | 2 |
| vii-34 | 3-F | 1 | O | 2 |
| vii-35 | 2,4-diF | 1 | O | 2 |
| vii-36 | 3,5-diF | 1 | O | 2 |

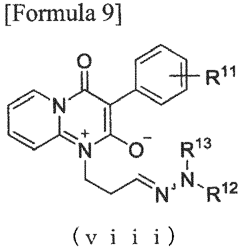

**[Table 8]**

| No. | R¹¹ | R₁₂ | H₁₃ |
|---|---|---|---|
| viii-1 | 3-CF₃ | H | H |
| viii-2 | 3-CF₃ | Me | H |
| viii-3 | 3-CF₃ | Me | Me |
| viii-4 | 3-CF₃ | COPh | H |
| viii-5 | 3-CF₃ | COCH₂CN | H |
| viii-6 | 3-CF₃ | 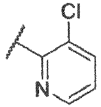 | H |
| viii-7 | 3-CF₃ | 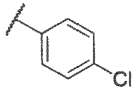 | H |
| viii-8 | 3-CF₃ | 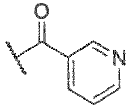 | H |

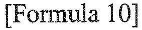

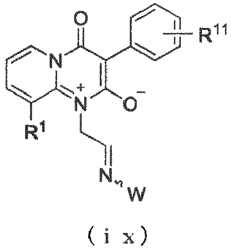

**[Table 9]**

| No. | R¹ | R¹¹ | W |
|---|---|---|---|
| ix-1 | H | 3-CF₃ | OH |
| ix-2 | H | 3-CF₃ | OMe |
| ix-3 | H | 2-F | OH |
| ix-4 | Me | 3-CF₃ | OH |
| ix-5 | Me | 3-CF₃ | OMe |
| ix-6 | H | 3-CF₃ | NMe₂ |
| ix-7 | H | 3-CF₃ | SOtBu |
| ix-8 | H | 4-CF₃ | OH |
| ix-9 | H | 2-CF₃ | OH |
| ix-10 | H | 3-CF₃ | 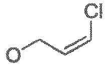 |
| ix-11 | H | 3-CF₃ | OCH₂Cl |
| ix-12 | H | 3-CF₃ | 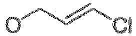 |
| ix-13 | H | 3,5-diCl | 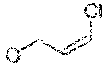 |
| ix-14 | H | 3,5-diCl | 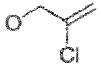 |
| ix-15 | Me | 3,5-diCl | 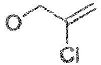 |
| ix-16 | Me | 3,5-diCl | 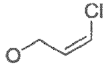 |
| ix-17 | H | 3,5-diCl | 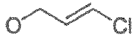 |
| ix-18 | Me | 3,5-diCl | 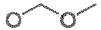 |
| ix-19 | Me | 3,5-diCl | 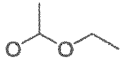 |

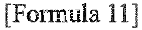

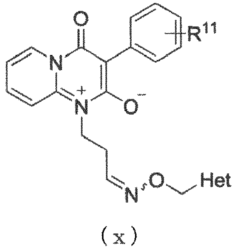

**[Table 10]**

| No. | R¹¹ | Het |
|---|---|---|
| x-1 | 3-CF₃ | 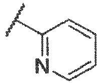 |
| x-2 | 3-CF₃ | 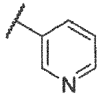 |
| x-3 | 3-CF₃ | 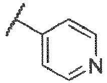 |
| x-4 | 3-CF₃ | 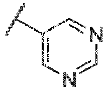 |
| x-5 | 3-CF₃ | 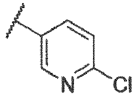 |
| x-6 | 3-CF₃ | 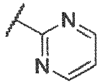 |
| x-7 | 3-CF₃ | 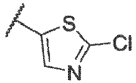 |

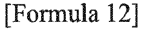

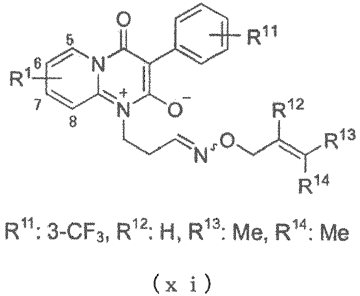

**[Table 11]**

| No. | R¹ |
|---|---|
| xi-1 | H |
| xi-2 | 8-F |
| xi-3 | 8-Cl |
| xi-4 | 8-Br |
| xi-5 | 8-I |
| xi-6 | 8-CN |
| xi-7 | 8-NO₂ |
| xi-8 | 8-SCN |
| xi-9 | 8-OH |
| xi-10 | 8-Me |
| xi-11 | 8-Et |
| xi-12 | 8-CH₂F |
| xi-13 | 8-CHF₂ |
| xi-14 | 8-CF₃ |
| xi-15 | 8-CCl₃ |
| xi-16 | 8-CH₂CN |
| xi-17 | 8-CH₂OH |
| xi-18 | 5-Me |
| xi-19 | 6-Me |
| xi-20 | 7-Me |

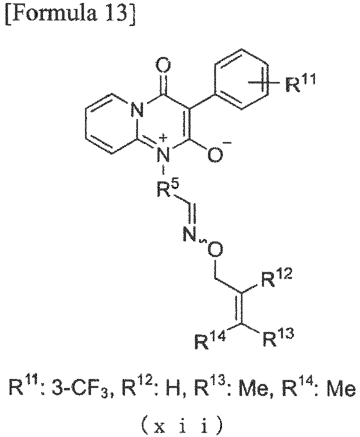

**[Table 12]**

| No. | R⁵ |
|---|---|
| xii-1 | -CH₂- |
| xii-2 | -C₂H₄- |
| xii-3 | -(CH₂)₃- |
| xii-4 | -CH₂-C(Me)- |
| xii-5 | -CH(Mc)₂- |
| xii-6 | -(CH₂)₄- |
| xi-7 | -CH₂-C(Et)- |
| xii-8 | -CH(nPr)- |
| xii-9 | -C(Me)(Et)- |
| xii-10 | -CH=CH- |
| xii-11 | -CH₂CH=CH- |
| xii-12 | -C≡C- |
| xii-13 | -(CH₂)₂-C≡C- |
| xii-14 | -CHF- |
| xii-15 | -CHCl- |
| xii-16 | -CH₂CHCl- |
| xii-17 | -CH=CHCl- |
| xii-18 | -CH₂CH(OH)- |
| xii-19 | -CH₂CH(CN)- |
| xii-20 | -CH₂CH(NO₂)- |

The compound (1) is a mesoionic intramolecular salt. The "intramolecular salt" also known as "zwitterions" in the art is an electrically neutral molecule, having positive and negative formal charges on different atoms in each valence bond structure, according to the valence bond theory. Moreover, the molecular structure of the compound (1) can be represented by six valence bond structures shown below, each of which has positive and negative formal charges on different atoms. Due to this resonance, the compound (1) is described to be "mesoionic". Although the molecular structure of formula (1) is shown herein as a single valence bond structure for simplicity, this particular valence bond structure should be understood as a representative of all the six valence bond structures shown below, relating to the bonds in the molecule of the compound (1). Any reference to the formula (1) herein, therefore, relates to all the six applicable valence bond structures and other structures, for example, in the molecular orbital theory, if not specified otherwise.

### <Six valence bond structures>

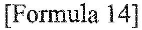

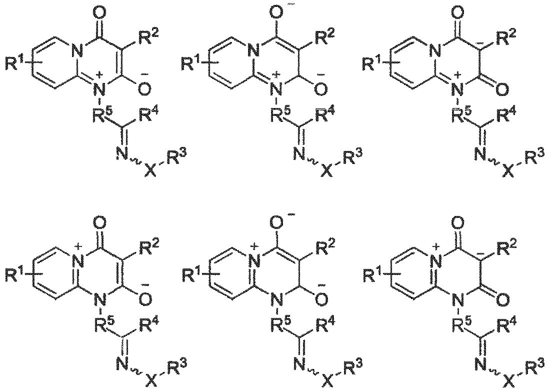

The compounds of the present invention can exist as one or more conformational isomers due to restricted bond rotation caused by steric hindrance. For example, the compound (1) having phenyl substituted with a bulky alkyl group such as isopropyl at the ortho position as R² can exist as two rotational isomers due to the restricted rotation relative to an R²-pyrimidinium ring bond. The present invention includes a mixture of conformational isomers.

The compound of the present invention has an imine bond, and an E-form and a Z-form are present depending on steric hindrance and thermal stability. Although there may exist the compounds in equilibrium, or the E-form or the Z-form alone in a solution depending on the nature of the compounds, the present invention is unrelated to the orientation thereof.

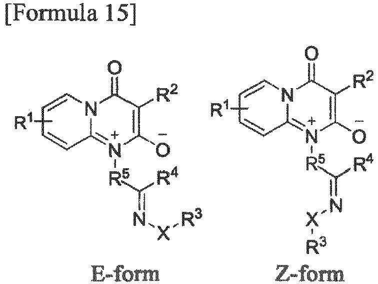

For example, a compound (1) can be synthesized by reacting a compound of formula (2) with an amine of formula (1a) as shown in Scheme 1. Examples of the amine include a hydrochloride, a hydrobromide, a trifluoroacetate, an acetate, an oxalate, and a free form. Optionally, an organic base such as pyridine may be used in some cases. The reaction is typically performed in the range from room temperature to 200°C. These reactions can be performed in the same temperature range with or without a halogen solvent. The typical reaction time is 5 minutes to 5 hours.

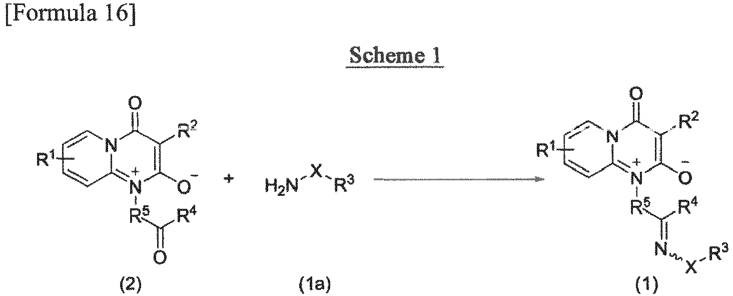

For example, a compound of formula (2) can be synthesized by reacting a compound of formula (3) with an acid as shown in Scheme 2. PG means a protective group of a hydroxyl group. Examples thereof include hydrochloric acid, trifluoroacetic acid, and acetic acid. The reaction is typically performed in the range from room temperature to 200°C. These reactions can be performed in the same temperature range with or without a halogen solvent. The typical reaction time is 1 hour to 20 hours.

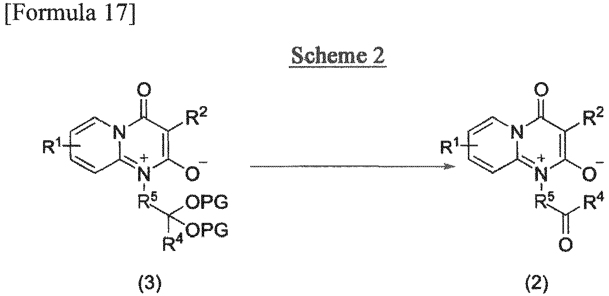

For example, a compound of formula (3) can be prepared by reacting a compound of formula (4) with a formula (3a) having an activated leaving group LvO as shown in Scheme 3. In view of the easiness in synthesis and the reactivity, examples of the preferred Lv include phenyl, 4-nitrophenyl or halogen-substituted phenyl such as 2,4,6-trichlorophenyl, pentachlorophenyl or pentafluorophenyl. The typical reaction temperature is in the range of 50 to 200°C. These reactions may be performed in a microwave reactor or a reaction vessel, with or without a solvent such as toluene, in the same temperature range. The typical reaction time is in the range of 5 minutes to 2 hours.

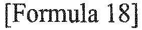

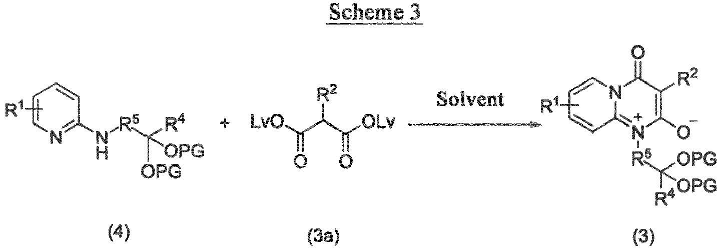

The compounds of formula (3) may be also prepared by condensing a compound of formula (4) and a compound of formula (3b) as shown in a scheme 4. These reactions are typically performed in an inert solvent such as dichloromethane, optionally in the presence of two or more equivalents of acid acceptor. Examples of the typical acid acceptor include triethylamine, N,N-diisopropyl-ethylamine, pyridine and substituted pyridine, and metal hydroxides, carbonates and bicarbonates, though not limited thereto.

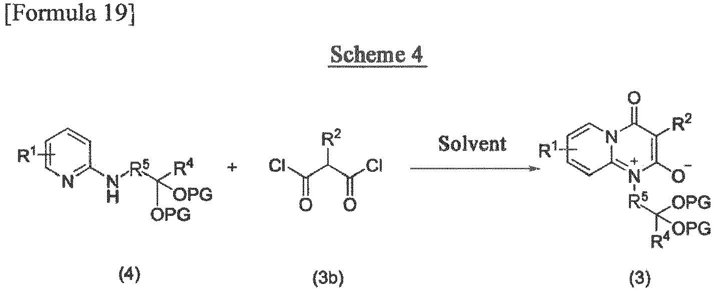

For example, a useful method for preparing a compound of the formula (4) is shown in Scheme 5. In the method of Scheme 5, the preparation can be achieved by reacting 2-aminopyridine of formula (4b) with an halogenated alkyl of formula (5) protected with corresponding acetal, dioxolane or the like, in the presence of a base such as sodium hydride.

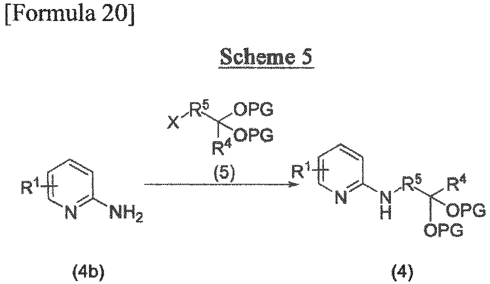

For example, another useful method for preparing a compound of the formula (4) is shown in Scheme 6. In the method of Scheme 6, pyridine oxide of a formula (4c) can be prepared by reacting an amine protected by a corresponding acetal, dioxane or the like with a formula (6) and a condensing agent such as hexafluorophosphate bromotris(pyrrolidino)phosphonium in the presence of a base such as diisopropylethylamine, though not limited thereto. Through preparation by using pyridine oxide of which two ortho positions are unsubstituted and asymmetric, a positional isomer of formula (4') and a positional isomer of formula (4") are produced.

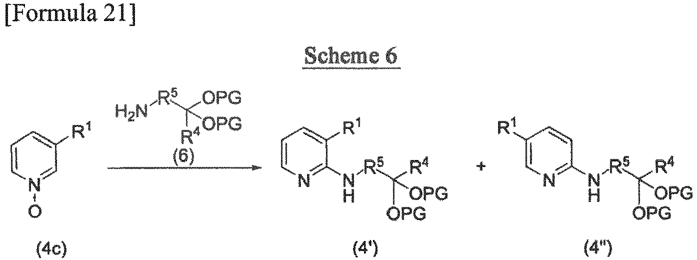

For example, a useful method for preparing a compound of formula (5) is shown in Scheme 7. In the method of Scheme 7, the preparation can be performed by using a corresponding halogenated alkyl or acid halide, an organic base such as pyridine, and an inorganic base such as sodium hydroxide.

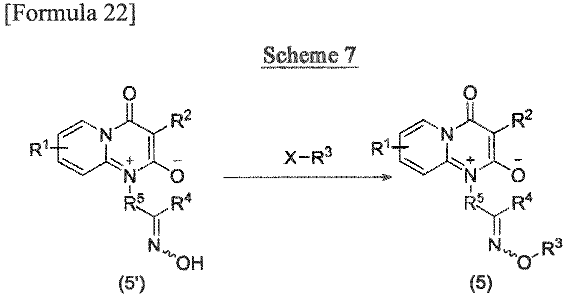

For example, a useful method for synthesizing la (compound of X=O, 1b-1d) in Scheme 1 is shown in Schemes 8 to 10. In the method of Scheme 8, a formula (6') can be prepared by reacting N-hydroxyphthalimide of formula (6") and a corresponding halogenated alkyl or acid halide with an organic base such as pyridine and trimethylamine and an inorganic base such as sodium hydroxide. A formula (1b) can be then produced by using a base such as hydrazine and sodium hydroxide. The formula (1b) can be prepared as a chloride by using an acidic substance such as hydrochloric acid in some cases.

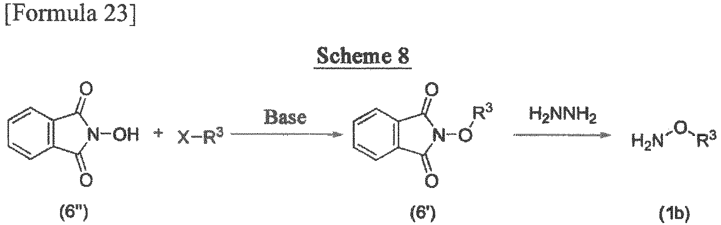

For example, a useful method for preparing a compound of formula (1c) is shown in Schemes 9. In the method of Scheme 9, the formula (1c) can be prepared by reacting phenol and hydroxylamine-O-sulfonic acid with an inorganic base such as sodium hydroxide. The formula (6b) can be prepared as a chloride by using an acidic substance such as hydrochloric acid in some cases.

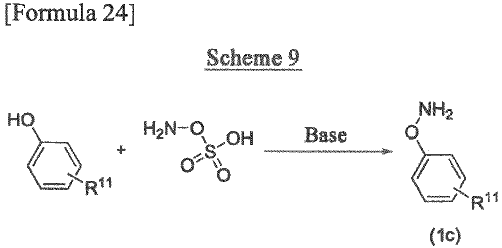

For example, a useful method for preparing a compound of (1d) is shown in Scheme 10. In the method of Scheme 10, a formula (6-1) can be prepared by reacting N-hydroxyphthalimide of formula (6") with a vinyl ether corresponding to the formula (1d) in the presence of an organic acid such as pyridinium paratoluene sulfonate. The typical reaction temperature is in the range from room temperature to 100°C. The formula (1d) can be then produced by using a base such as hydrazine and sodium hydroxide.

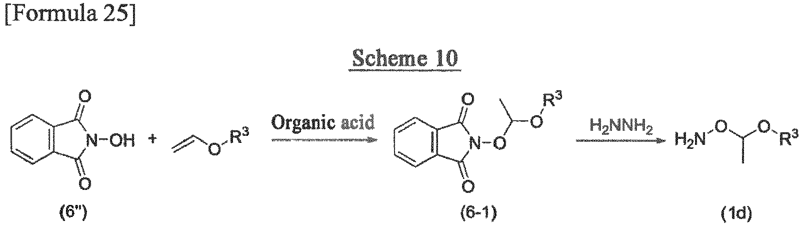

Also, the compound (1) can be prepared by condensing a compound of formula (7) and a compound of formula (3b) as shown in Scheme 11. These reactions are typically performed in an inert solvent such as dichloromethane, and in the presence of two or more equivalents of an acid receptor in some cases. Examples of the typical acid acceptor include triethylamine, N,N-diisopropyl-ethylamine, pyridine and substituted pyridine, as well as metal hydroxides, carbonates and bicarbonates, though not limited thereto.

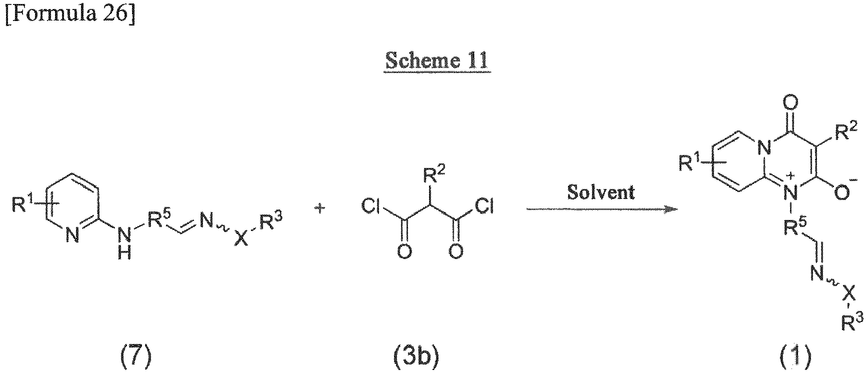

The compound (1) has an excellent controlling activity against agricultural and horticultural harmful insects as shown in the examples below. According to the present invention, therefore, an agricultural and horticultural insecticide comprising a compound (1) as an active ingredient is provided. Further, the agricultural and horticultural insecticide according to the present invention may contain agriculturally and horticulturally acceptable acid addition salts of these compounds as active ingredients.

Specific examples of the acid addition salt include inorganic acids such as hydrochloride, sulfate, nitrate, and phosphate, carboxylic acid salt such as formate, acetate, oxalate, citrate, succinate, maleate, fumarate, tartrate, lactate, benzoate and phthalate, and sulfonic acid salts such as mesylate, tosylate, and benzenesulfonate.

Insect species to be controlled in the present invention, i.e., insect species on which the compound (1) has control effect, is not limited in particular, and a wide range of agricultural and horticultural harmful insects can be controlled thereby. Examples of preferred insect species to be controlled include lepidopteran pests (e.g. Noctuids such as common cutworm, beet armyworm, rice armyworm, cabbage armyworm, black cutworm, Trichoplusia spp, Heliothis spp, and Helicoverpa spp; Pyralids such as rice stem borer, rice leafroller, European corn borer, cabbage webworm, bluegrass webworm, cotton leaf-roller, and Indian meal moth; Pierids such as small cabbage white butterfly; Tortricids such as Adoxophyes spp, oriental fruit moth, and codling moth; snoutworms such as peach fruit moth; leafminers such as Lyonetia spp; tussock moths such as Lymantria spp and Euproctis spp; Yponomeutids such as diamondback moth; gelechiid moths such as pink bollworm; garden tiger moths such as fall webworm; and tineid moths such as clothes moth and webbing clothes moth); hemipteran pests (e.g. aphids such as green peach aphid and cotton aphid; planthoppers such as Laodel phax sriatellus, brown planthopper, and white backed planthoppers; Cicadellids such as Bothrogonia japonica; stink bugs such as Trigonotylus caelestialium, brown winged green stink bug, Nezara viridula, and Riptortus pedestris; whiteflies such as greenhouse whiteflies and silver leaf whiteflies; Coccoids such as comstock mealybug; lace bugs; and Psyllids); coleopteran pests (e.g. weevils such as maize weevil, rice water weevil, and azuki bean weevil; Neatus picipes such as Tenebrio molitor Linnaeus; scarabs such as cupreous chafer and soybeen beetle; leaf beetles such as striped flea beetle, melon beetle, Colorado potato beetle, western corn root worm, and southern corn root worm; Epilachnas such as Inedorooimushi, Paederus fuscipes,
snoutworms, and Epilachna vigintioctopunctata; and Western bean cutworms); Acarian pests (e.g. spider mites such as two-spotted spider mite, Kanzawa spider mite, mandarin orange spider mite, apple spider mite, and Oligonicus spp; Eriophyids such as tomato rust mite, mandarin orange rust mite and tea rust mite; Tarsonemus such as tea plant Tarsonemus; and mold mites); hymenopteran pests (e.g. sawflies such as Athalia); orthopteran pests (e.g. grasshoppers); dipteran pests (e.g. muscids; culexes; anopheles; midges; blowflies; flesh flies; Fannia canicularis; anthomyiid flies; leafminer flies such as bean leafminer flies, tomato leafminer flies, and eggplant leafminer flies; fruit flies; Phorid flies; Drosophila; moth flies; black flies; Tabanid flies; and Stomoxys calcitrans); thrips pests (e.g. southern yellow thrips, western flower thrips, onion thrips, Thrips hawaiiesis, yellow tea thrips, Frankliniella intonsa, and Ponticulothrips diospyrosi); and plant-parasitic nematodes (e.g. root-knot nematode; Pratylenchus spp; cyst nematodes; Aphelenchoides such as white-tip nematode of rice; and pine wood nematode).

Use of the compound (1) of the present invention or the agriculturally and horticulturally acceptable acid addition salts thereof as the active ingredient of agricultural and horticultural insecticides is provided.

In use of the compound (1) as an agricultural and horticultural insecticide, the compound (1) may be used as it is, or may be mixed with a suitable solid carrier, liquid carrier, gaseous carrier, surfactant, dispersing agent, and other formulation aids or the like so as to prepare an agrochemical formulation. Examples of the preferred agrochemical formulation include an emulsion, an EW formulation, a solution, a suspension, a wettable powder, wettable granules, powders, DL powder, particulates, granules, tablets, an oil solution, an aerosol, a flowable formulation, a dry flowable formulation, and microcapsules. These dosage forms may be optionally selected for use as agrochemical formulation. In the present invention, a carrier refers to a solid carrier, a liquid carrier, or a gaseous carrier or the like.

Examples of the solid carrier include talc, bennite, clay, kaolin, diatomaceous earth, vermiculite, white carbon, calcium carbonate, acid clay, silica sand, silica stone, zeolite, perlite, attapulgite, pumice, ammonium sulfate, sodium sulfate, and urea.

Examples of the liquid carrier include alcohols such as methanol, ethanol, n-hexanol, ethylene glycol and propylene glycol; ketones such as acetone, methyl ethyl ketone and cyclohexanone; aliphatic hydrocarbons such as n-hexane, kerosene and lamp oil, aromatic hydrocarbons such as toluene, xylene and methylnaphthalene; ethers such as diethyl ether, dioxane and tetrahydrofuran; esters such as ethyl acetate; nitriles such as acetonitrile and isobutyronitrile, acid amides such as dimethylformamide and dimethylacetamide; vegetable oils such as soybean oil and cottonseed oil; dimethyl sulfoxide; and water.

Also, examples of the gaseous carrier include LPG, air, nitrogen, carbon dioxide, and dimethyl ether.

Examples of the surfactant and the dispersant include alkyl sulfates, alkyl(aryl)sulfonates, polyoxyalkylene alkyl(aryl) ethers, polyhydric alcohol esters, lignin sulfonates, alkyl sulfosuccinates, formalin condensates of alkylnaphthalene sulfonates, polycarboxylates, POE polystyrylphenyl ether sulfates and phosphates, and POE/POP block polymers.

Furthermore, examples of the formulation aid include carboxymethyl cellulose, hydroxypropyl cellulose, polyvinyl alcohol, xanthan gum, alpha-starch, gum arabic, polyvinyl pyrrolidone, an ethylene-acrylic acid copolymer, an ethylene-vinyl acetate copolymer, polyethylene glycol, liquid paraffin, calcium stearate, and defoamers and preservatives.

The various carriers, surfactants, dispersants, and formulation aids described above may be used alone or in combination on an as needed basis.

The content of the compound (1) as active ingredient in the agrochemical formulation is preferably 1 to 75 wt% in emulsions, 0.3 to 25 wt% in powder, 1 to 90 wt% in wettable powder, and 0.1 to 10 wt% in granules, though not particularly limited thereto.

The agricultural and horticultural insecticides of the present invention may be directly used or diluted for use. Further, the agricultural and horticultural insecticides of the present invention may be mixed with or used in combination with other insecticides, fungicides, miticides, herbicides, plant growth regulators, fertilizers and the like. Examples of the chemicals that can be mixed or used in combination include ones described in Pesticide Manual, 17th edition, published by The British Crop Protection Council, SHIBUYA INDEX, 16th Edition, 2011, published by SHIBUYA INDEX RESEARCH GROUP and SHIBUYA INDEX 17th edition, 2014, published by SHIBUYA INDEX RESEARCH GROUP, and Mode of Action Classification, version 8.2, published by IRAC and Mode of Action of Fungicide, 2017-year version, published by FRAC.

More specifically, examples of the insecticides may include:
carbamate compounds such as alanycarb, aldicarb, bendiocarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylylcarb, metolcarb, fenothiocarb, and fenoxycarb;
organic phosphoric acid ester compounds such as acephate, azamethiphos, azinphos-ethyl, azinphos-methyl, ethylthiometon, chlorethoxyfos, cadusafos, chlorethoxyfos, chlorfenvinphos, chlormephos, chlorpyrifos, chlorpyrifos-methyl, coumaphos, cyanophos, demeton-S-methyl, diazinon, dichlorvos, dicrotophos, dimethoate, dimethylvinphos, EPN, ethion, etoprophos, famphur, fenamifos, fenitrothion, fenthion, fosthiazate, heptenophos, imicyafos, isofenphos, isopropyl O-(methoxyaminothiophosphoryl)salicylate, isoxathion, malathion, mecarbam, methamidophos, methidathion, mevinphos, monocrotophos, naled, omethoate, oxydemeton ethyl, parathion, parathion-methyl, PAP, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, chlorpyrifos-methyl, disulfoton, sulprofos, flupyrazophos, phenthoate, fonofos, and tribufos;
organochlorine compounds such as endosulfan, alpha-endosulfan, gamma-HCH, dicofol, chlordane, dieldrin, and methoxyclor;
phenylpyrazole compounds such as acetoprole, fipronil, ethiprole, pyrafluprole, pyriprole, and flufiprole;
meta-diamide compounds such as broflanilide;
isoxazoline compounds such as afoxolaner, fluralaner, sarolaner, and fluxametamide;
pyrethroid compounds such as acrinathrin, allethrin, d-cis-trans allethrin, d-trans allethrin, bifenthrin, kappa-bifenthrin, bioallethrin S-cyclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, ethofenprox, fenpropathrin, fenvalerate, flucythrinate, flumethrin, tau-fluvalinate, halfenprox, imiprothrin, kadethrin, permethrin, phenothrin, prallethrin, pyrethrin, resmethrin, silafluofen, tefluthrin, kappa-tefluthrin, phthalthrin, tetramethrin, tralomethrin, transfluthrin, metoxadiazone, metofluthrin, profluthrin, pyrethrum, terallethrin, momfluorothrin, heptafluthrin, meperfluthrin, tetramethylfluthrin, dimefluthrin, protrifenbut, chloroparellethrin, epsilon-metofluthrin, and epsilon-momfluorothrin;
neonicotinoid compounds such as acetamiprid, clothianidin, dinotefuran, imidacloprid, nitenpyram, thiacloprid, and thiamethoxam;
sulfoxamine compounds such as sulfoxaflor;
butenolide compounds such as flupyradifurone;
mesoionic compounds such as triflumezopyrim and dicloromezotiaz;
2-aminopyridyl compounds such as flupyrimin
spinosyn compounds such as spinosad and spinetoram;
macrolide compounds such as abamectin, ivermectin, emamectin benzoate, milbemectin, and lepimectin;
juvenile hormone-like compounds such as hydroprene, kinoprene, Diofenolan, and methoprene;
4-phenoxyphenoxy compounds such as pyriproxyfene;
pyridine azomethine compounds such as pymetrozine;
pyridinecarboxamide compounds such as flonicamid;
oxazole compounds such as ethoxazole;
Bacillusthuringiensis and Bacillus sphaericus agents such as B.t. subsp. israelensis, B.t. subsp.aizawai, B.t. subsp. kurstaki, B.t. subsp. tenebrionis and insecticidal proteins they produce;
an insecticidal protein produced from Bt crops described above, i.e., genetically modified crops with genes producing a toxin of Bacillus thuringiensis incorporated
thiourea compounds such as diafenthiuron;
organic metal compounds such as azocyclotin, cyhexatin, fenbutatin oxide;
sulfurous acid ester/diphenyl ether compounds such as propargite;
diphenyl sulfone compounds such as tetradifon;
pyrrole compounds such as chlorfenapyr and tralopyril;
dinitro compounds such as DNOC;
nereistoxin analogs such as bensultap, cartap, thiocyclam, thiosultap, and thiosultap sodium;
benzoyl urea compounds such as bistrifluron, chlorfluazuron, diflubenzuron, flucycloxuron, flufenoxuron, hexaflumuron, lufenuron, novaluron, noviflumuron, teflubenzuron, triflumuron, and bistrifluron;
thiadiazine compounds such as buprofezin;
triazole compounds such as cyromazine;
diacyl hydrazine compounds such as chromafenozide, halofenozide, methoxyfenozide, and tebufenozide;
amidine compounds such as amitraz;
amidinohydrazone compounds such as hydramethylnon;
naphthoquinone compounds such as acequinocyl;
strobilurin compounds such as fluacrypyrim, pyriminostrobin, and Flufenoxystrobin;
quinazoline compounds such as fenazaquin;
phenoxypyrazole compounds such as fenpyroxymate;
phenoxyethylamine compounds such as pyrimidifen;
pyridazinone compounds such as pyridaben;
pyrazolecarboxamide compounds such as tebufenpyrad, tolfenpyrad, and pyflubumide;
hydrazinecarboxamide compounds such as metaflumizone;
tetronic acid and tetramic acid compounds such as spirodiclofen, spirotetaramat, spiromesifen, and spiropidion;
beta-ketonitrile compounds such as cyflumetofen and cyenopyrafen;
phthalic acid amide compounds such as flubendiamide and cyhalodiamide;
anthranilic acid amide compounds such as chlorantraniliprole, cyantraniliprole, cyclaniliprole, and tetraniliprole;
quinoxaline compounds such as quinomethionate;
thiazolidinone compounds such as hexythiazox;
hydrazine compounds such as bifenazate ;
pyridinamine compounds such as flufenerim;
aminoquinazoline compounds such as pyrifluquinazon;
6-phenoxy quinoline compounds such as flometoquin;
pyridinylethylbenzamide compounds such as fluopyram; and
sulfonamide compounds such as 8-chloro-N-((2-chloro-5-methoxyphenyl)sulfonyl)-6-(trifluoromethyl)imidazo[1,2-alpyridine-2-carboxamide] and amidoflumet.

Further, examples of other insecticides include nicotine, chloropicrin, sulfuryl fluoride, crylotie, clofentezine, diflovidazin, rotenone, indoxacarb, piperonyl butoxide, chlordimeform, pyridalyl, azadirachtin, benzoxymate, afidopyropen, fluhexafon, tioxazafen, fluensulfone, fluazaindolizine, benclothiaz, carzole, insecticidal soap, dimehypo, nithiazine, borate salt, metaaldehyde, ryanodine, sulfluramid, acynonapyl, benzpyrimoxan, tyclopyrazoflor, 3 -bromo-N-(2,4-dichloro-6-(methylcarbamoil)phenylyl)-1-(3,5-dichloropyridine-2-yl)-1H-pyrazole-5-carboxamide, N-(3-chloro-1-(pyridine-3-yl)-1H-pyrazole-4-yl)-N-ethyl-3-((3,3,3-trifluoropropyl)tio) propanamide, and N-(3-chloro-1-(pyridine-3-yl)-1H-pyrazole-4-yl)-N-ethyl-3-((3,3,3-trifluoropropyl)sulphinyl)propanamide. In addition, agricultural and horticultural insecticides of the present invention can may be mixed or used in combination with microbial pesticides such as insect pathogenic bacteria, insect-pathogenic virus and insect pathogenic fungi.

Examples of the fungicides for use include:
phenylamide compounds such as metalaxyl, metalaxyl-M, oxadixyl, ofurase, benalaxyl, benalaxyl-M, kiralaxyl, ofurace, furalaxyl, and cyprofuram;
hydroxypyrimidine compounds such as bupyrimate, dimethilimol, and ethilimol;
isoxazole compounds such as hymexazole and hydroxyisoxazole;
piperidinylthiazole isoxazoline compounds such as oxathiapiprolin;
isothiazolone compounds such as octhilinone;
carboxylic acid compounds such as oxolinic acid;
benzimidazole thiophanate compounds such as benomyl, thiophanate-methyl, carbendazole, fuberidazole, thiabendazole, and debacarb;
N-phenyl carbamate compounds such as diethofencarb;
toluamide compounds such as zoxamide;
ethylamino thiazolecarboxamide compounds such as ethaboxam;
phenylurea compounds such as pencycuron;
pyridinylmethylbenzamide compounds such as fluopicolide;
pyrimidinamine compounds such as diflumetorim and bupirimate;
pyrazolecarboxamide compounds such as tolfenpyrad;
benzanilide compounds such as benodanil, flutolanil, and mepronil;
phenyloxoethyl thiophene amide compounds such as isofetamid;
pyridinyl ethylbenzamide compounds such as fluopyram;
furancarboxamide compounds such as fenfuram;
oxathiincarboxamide compounds such as oxycarboxin and carboxin;
thiazolecarboxamide compounds such as thifluzamide;
pyrazole-4-carboxamide compounds such as fluxapyroxad, furametpyr, penflufen, penthiopyrad, benzovindiflupyr, bixafen, isopyrazam, sedaxane, and inpyrfluxam, fluindapyr, and isoflucypram;
pyridinecarboxamide compounds such as boscalid;
strobilurin compounds such as azoxystrobin, coumetoxystrobin, kresoxym-methyl, trifloxystrobin, picoxystrobin, pyraclostrobin, dimoxystrobin, metominostrobin, orysastrobin, fluoxastrobin, pyraoxystrobin, pyrametostrobin, flufenoxystrobin, fenaminstrobin, enoxastrobin, coumoxystrobin, mandestrobin, and triclopyricarb;
oxazolidinedione compounds such as famoxadon;
imidazolinone compounds such as fenamidone;
benzylcarbamate compounds such as triclopyricarab and pyribencarb;
cyano imidazole compounds such as cyazofamid;
sulfamoyltriazole compounds such as amisulbrom;
dinitrophenylcroton compounds such as binapacryl, meptyldinocap, dinocap;
2,6-dinitroaniline compounds such as fluazinam;
pyrimidinone hydrazone compounds such as ferimzone;
organic and inorganic metal compounds such as fentin-acetate, fentin chloride, fentin hydroxide, fenthin hydroxide, fenthin acetate and oxine copper;
thiophenecarboxamide compounds such as silthiofam;
triazolopyrimidine amine compounds such as ametoctradin;
anilinopyrimidine compounds such as mepanipyrim, nitrapyrin, pyrimethanil and cyprodinil;
enopiranuron acid antibiotics such as blasticidin-S;
hexopyranosyl antibiotics such as kasugamycin, kasugamycin hydrochloride hydrate;
glucopyranosyl antibiotics such as streptomycin;
tetracycline antibiotics such as oxytetracycline;
allyloxyquinoline compounds such as quinoxyfen;
quinazoline compounds such as proquinazid;
cyanopyrrole compounds such as fludioxonil and fenpiclonil;
dicarboximide compounds such as fluoroimid, procymidone, iprodione, and vinchlozolin;
phosphorothiorate compounds such as edifenphos, iprobenfos and pyrazophos;
dithiolane compounds such as isoprothiolane;
propylcarbamate compounds such as propamocarb and propamocarb hydrochloride;
genus Bacillus such as Bacillus subtilis and produced bactericidal proteins including QST713, FZB24, MBI600, D747 strains;
bactericidal protein compounds produced by the Bt crops described above;
terpene hydrocarbons and terpene alcohols such as extracts of tee tree;
piperazine compounds such as triforine;
pyridine compounds such as pyrifenox and pyrisoxazole;
pyrimidine compounds such as fenarimol and nuarimol;
azole compounds such as azaconazole, bromuconazole, diniconazole, diniconazole-M, epoxyconazole, fluquinconazole, oxpoconazole, pefurazoate, difenoconazole, fenbuconazole, imibenconazole, ipconazole, metconazole, tetraconazole, triadimefon, triadimenol, triticonazole, uniconazole, imazalil, bitertanol, triflumizole, etaconazole, propiconazole, penconazole, flusilazole, flutriafol, myclobutanil, paclobutrazol, prothioconazole, cyproconazole, tebuconazole, hexaconazole, prochloraz, simeconazole, ipfentrifluconazole, and mefentrifluconazole;
morpholine compounds such as aldimorph, dodemorph, dodemorph acetate, tridemorph, fenpropimorph, dimethomorph, flumorph, and pyrimorph;
piperidine compounds such as piperalin and fenpropidin;
spiroketalamine compounds such as spiroxamine;
hydroxyanilide compounds such as fenhexamid;
amino pyrazolinon compounds such as fenpyrazamine;
thiocarbamate/dithiocarbamate compounds such as ferbam, metam, metasulphocarb, metiram, thiram, mancozeb, maneb, zineb, ziram, polycarbamate, propineb, thiuram and pyributicarb;
glucopyranosyl antibiotics such as validamycin;
nucleoside antibiotics such as mildiomycin and polyoxin;
valine amide carbamate-based compounds such as benthiavalicarb, benthiavalicarb-isopropyl, valifenalate, and iprovalicarb;
mandelic acid amide compounds such as mandipropamid;
picolinamide compounds such as fenpicoxamid;
isobenzofuranon compounds such as fthalide;
pyrroloquinolinon compounds such as pyroquilone;
triazolobenzothiazole compounds such as tricyclazole;
cyclopropanecarboxamide compounds such as carpropamid;
carboxamide compounds such as diclocymet;
propionamide compounds such as fenoxanil;
benzothiadiazole compounds such as acibenzolar-S-methyl;
benzisothiazole compounds such as probenazole and dichlobentiazox;
thiadiazole carboxamide compounds such as tiadinil;
isothiazolecarboxamide compounds such as isotianil;
cyanoacetamide oxime compounds such as cymoxanil;
ethylphosphonate compounds such as fosetyl;
phthalamic acid compounds such as techlophthalam;
benzotriazine compounds such as triazoxide;
benzenesulfonic acid compounds such as flusulfamide;
pyridazinone compounds such as diclomezine;
phenylacetamide compounds such as cyflufenamide;
benzophenone compounds such as metrafenone;
benzoylpyridine compounds such as pyriofenone;
cyanomethylene thiazolidine compounds such as flutianil;
4-quinolyl acetic acid compounds such as tebufloquin;
organic phosphoric acid compounds such as fosetyl-aluminium and tolclofosmethyl;
1,2,4-thiadiazole compounds such as echlomezole;
trifluoroethyl carbamate compounds such as tolprocarb;
copper-based compounds such as Bordeaux mixture, copper acetate, basic copper sulfate, oxy copper chloride, copper hydroxide, and oxine-copper;
inorganic compounds such as copper and sulfur;
N-halogenothioalkyl-based compounds such as captan, captafol, and folpet;
organochlorine compounds such as anilazine, chlorothalonil, dichlorophen, pentachlorophenol and salts thereof, hexachlorobenzene, and quintozene;
guanidine compounds such as iminoctadine triacetate salt, iminoctadine albesilate, guanidine, dodine, dodine free base, guazatine, guazatine acetate salt, and albesilate;
anthraquinone compounds such as dithianon;
quinoxaline compounds such as quinomethionate;
maleimide compounds such as fluoroimide;
sulfenic acid compounds such as tolylfluanid and dichlofluanid;
dinitrophenol compounds such as dinobuton;
cyclic dithiocarbamate compounds such as dazomet; and
a pyrazolecarboxamide compound such as pydiflumetofen.

Examples of other fungicides include, dipymetitrone, pyraziflumid, picarbutrazox, tecnazen, nitrthal-isopropyl, dicyclomet, acibenzolar, prohexadione-calcium, bronopol, diphenylamine, flumetover, benthoxazin, biphenyl, chloroneb, CNA, iodcarb, prothiocarb, aminopyrifen, 3-chloro-5-phenyl-6-methyl-4-(2,6-difluorophenyl)pyridazine, and quinofumelin.

Also, the compound (1) or acid addition salts thereof which are agriculturally and horticulturally acceptable of the present invention in an effective amount can be applied to plants or soil for use in controlling agriculturally and horticulturally harmful insects. In other words, a method for controlling agriculturally and horticulturally harmful insects is provided. The controlling method of the present invention may also include a method for applying the compound (1) or agriculturally and horticulturally acceptable acid addition salts thereof by smoking treatment in a sealed space.

The compounds represented by the formula (1) or salts thereof of the present invention may be also used to control harmful parasitic invertebrate organisms which are parasitic on animals or birds and protect the animals and birds from the harmful parasitic invertebrate organisms. The composition in an effective amount for controlling the harmful parasitic invertebrate organisms which are parasitic on animals or birds is used. The composition may further contain at least one carrier.

The method for controlling harmful invertebrate organisms of the present invention comprises bringing the compound represented by the formula (1) or a salt thereof of the present invention in a biologically effective amount into contact with the harmful invertebrate organisms. Also, the method for producing crops with harmful invertebrate organisms controlled of the present invention comprises performing the method for controlling harmful invertebrate organisms.

The method for treating crops of the present invention comprises bringing the compound represented by the formula (1) or a salt thereof of the present invention in a biologically effective amount into contact with seeds or placentas of crops. Also, the method for producing treated crops of the present invention comprises performing the method for treating crops.

The seeds of the present invention comprise seeds of crops and about 0.0001 to 1 mass% of the compound represented by the formula (1) or a salt thereof of the present invention relative to the seeds of crops.

### Examples

### <Synthesis Examples>

Hereinafter, the present invention is specifically described with reference to examples, though the present invention is not limited thereto. In NMR data, "s" represents a singlet, "d" represents a doublet, "t" represents a triplet, "q" represents a quartet, "m" represents a multiplet, and "brs" represents a broad singlet.

Synthesis Example 1-1: Preparation of 1-(3-(((3-methyl-2-butenyl)oxy)imino)propyl)-4-oxo-3-(3-(trifluoromethyl)phenyl)-4H-pyrido[1,2-a]pyrimidinium inner salt (compound number: E-7)

### Step A: Preparation of 1,3-diethyl-2-(3-trifluoromethylphenyl)propanedioate

First, 1,10-phenanthrolene (2.52 g, 13.9 mmol) and copper(I) iodide (2.53 g, 13.3 mmol) was added to 1,4-dioxane (200 mL), and then cesium carbonate (36.1 g, 111 mmol), diethyl malonate (17.7 g, 111 mmol) and 3-iodobenzotrifluoride (25.1 g, 92.3 mmol) were added thereto. After stirring for 7.5 hours under reflux, the reaction mixture was cooled to room temperature. Aqueous 1 N hydrochloric acid was added to the reaction mixture, which was filtered with a filter aid to remove solids. The filtrate was extracted twice with ethyl acetate, dried over magnesium sulfate, and filtered. The solvent of the filtrate was distilled off under reduced pressure. The resulting crude product was purified by silica gel chromatography, so that the title compound (21.2 g, 75%, pale yellow liquid) was obtained.
¹H NMR (CDCl₃) δ = 7.72-7.57 (3H, m), 7.49 (1H, t, J = 7.8Hz), 4.66 (1H, s), 4.32-4.15 (4H, m), 1.32-1.22 (6H, m)

### Step B: Preparation of bis(2,4,6-trichlorophenyl)-2-(3-trifluoromethylphenyl)propanedioate

The product in the step A (18.7 g, 61.4 mmol) was added to 18% sodium hydroxide aqueous solution (219 g), and the reaction mixture was vigorously stirred for 1 hour at 50°C under nitrogen atmosphere. Subsequently, hydrochloric acid was added to the reaction solution until the pH reached 1. The reaction solution was extracted 3 times with ethyl acetate, dried over magnesium sulfate and filtered. The solvent of the filtrate was distilled off under reduced pressure by an evaporator. To the resulting white solid (13.2 g), dichloromethane (200 mL) was added, and oxalyl chloride (15.8 mL, 184 mmol) and N,N-dimethylformamide (4 mL) were added thereto. The reaction mixture was stirred for 3 hours at room temperature, and condensed under reduced pressure by an evaporator. To the resulting residue, dichloromethane (200 mL) was added, and then 2,4,6-trichlorophenol (24.3 g, 123 mmol) was added thereto. After stirring overnight at room temperature, the reaction mixture was concentrated under reduced pressure. Methanol was added to the resulting residue, so that solids precipitated out slowly from the solution. The precipitated solids were collected by filtration and dried at 50°C, so that the title compound (12.8 g, 34%, white solid) was obtained.
¹H NMR (CDCl₃) δ = 7.92 (1H, s), 7.85 (1H, d, J = 7.8Hz), 7.71 (1H, d, J = 7.8Hz), 7.60 (1H, t, J = 7.8Hz), 7.38 (4H, s), 5.38 (1H, s)

### Step C: Preparation of N-(3,3-diethoxypropyl)pyridin-2-amine

Into a flask containing weighed sodium hydride (4.60 g, 105 mmol), dimethyl sulfoxide (140 mL) was added. Further, 2-aminopyridine (8.99 g, 95.6 mmol) was added thereto, and the temperature was raised to 60°C for heating for 15 minutes. Then, 3-chloro-1,1-diethoxypropane (15.9 g, 95.7 mmol) dissolved in dimethylsulfoxide (40 mL) was added dropwise. The temperature was raised to 80°C for heating for 2 hours. After cooling to room temperature, water was added to the solution which was extracted twice with ethyl acetate, and washed once with brine (saturated brine). The solution was dried over magnesium sulfate, and filtered. The solvent of the filtrate was distilled off under reduced pressure. The resulting crude product was purified by column chromatography, so that the title compound (9.92 g, 46%, brown liquid) was obtained.
¹H NMR (CDCl₃) δ = 8.07 (1H, dd, J = 5.1, 1.2Hz), 7.36-7.42 (1H, m), 6.52-6.56 (1H, m), 6.37 (1H, d, J = 8.4Hz), 4.84 (1H, bs), 4.63 (1H, t, J = 5.4Hz), 3.68 (2H, dq, 7.2, 7.0Hz), 3.51 (2H, dq, J = 7.2, 7.0Hz), 3.38 (2H, q, J = 6.1Hz), 1.95 (2H, dt, J = 6.1, 5.7Hz), 1.22 (6H, t, J = 7.0Hz)

### Step D: Preparation of 1-(3,3-diethoxypropyl)-4-oxyo-3-(3-trifluoromethyl)phenyl)-4H-pyrido[1,2-a]pyrimidinium inner salt

Into toluene (40 mL), the product of the step C (4.04 g, 18.0 mmol) and the product of the step B (10.9 g, 18.0 mmol) were added. The reaction mixture was heated at 110°C for 2 hours. The reaction solution was cooled to room temperature and toluene was distilled off under reduced pressure. The crude product was dissolved in a small amount of ethyl acetate, and added dropwise into n-hexane (100 mL). The precipitated solid was collected by filtration, and the resulting crystals were hot air-dried at 60°C, so that the title compound (6.34 g, 80%, yellow solid) was obtained.
¹H NMR (CDCl₃) δ = 9.48-9.51 (1H, m), 8.05-8.12 (2H, m), 7.98-8.02 (1H, m), 7.72 (1H, d, J = 9.0Hz), 7.46-7.48 (2H, m), 7.32 (1H, dt, J = 6.9, 0.9Hz), 4.64 (1H, t, J = 4.8Hz), 4.45 (2H, t, J = 7.3Hz), 3.71 (2H, dq, 7.8, 6.9Hz), 3.53 (2H, dq, J = 7.2, 6.9Hz), 2.04-2.13 (2H, m), 1.21 (6H, t, J = 7.0Hz)

### Step E: Preparation of 4-oxo-1-(3-oxopropyl)-3-(3-(trifluoromethyl)phenyl)-4H-pyrido[1,2-a]pyrimidinium inner salt

The product of the step D (5.14 g, 11.7 mmol) was dissolved in methylene chloride (60 mL), and trifluoroacetic acid (4.5 mL, 58.8 mmol) was added dropwise thereto at room temperature and stirred at room temperature for 5 hours. The sodium hydrogen carbonate aqueous solution was added to the solution, which was then extracted twice with methylene chloride, and washed with brine. The solution was dried over magnesium sulfate, and filtered. The solvent of the filtrate was distilled off under reduced pressure. The resulting crude product was purified by column chromatography, so that the title compound (3.14 g, 80%, yellow solid) was obtained.
¹H NMR (CDCl₃) δ = 9.86 (1H, s), 9.53 (1H, dd, J = 6.9, 1.2Hz), 8.10-8.18 (2H, m). 7.97-8.01 (1H, m), 7.72 (1H, d, J = 9.0Hz), 7.47-7.49 (2H, m), 7.38 (1H, dt, J = 6.9, 0.9Hz), 4.64 (2H, t, J = 6.7Hz), 3.09 (2H, t, J = 6.6Hz)

### Step F: Preparation of 2-((3-methyl-2-butenyl)oxy)isoindolidinone

N-hydroxyphthalimide (10.6 g, 65.0 mmol) was dissolved in DMF (40 mL), and triethylamine (6.58 g, 65.0 mmol) was added to the solution, which was then stirred for 5 minutes. To the solution, 1-chloro-3-methyl-2-butene (6.53 g, 50.0 mmol) was added dropwise at room temperature and stirred at room temperature for 15 hours. Water (150 mL) was added thereto, and a precipitated pale yellow solid was collected by filtration. The solid thus produced was dissolved in ethyl acetate, washed with a sodium hydrogen carbonate aqueous solution and brine, dried over magnesium sulfate, and filtered. The solvent of the filtrate was distilled off under reduced pressure, so that the title compound (10.25 g, 68%, pale yellow solid) was obtained.
¹H NMR (CDCl₃) δ = 7.80-7.84 (2H, m), 7.72-7.76 (2H, m), 5.53 (1H, tt, J = 7.6, 1.3Hz), 4.72 (2H, d, J = 7.5Hz), 1.77 (3H, s), 1.73 (3H, d, J = 0.9Hz)

### Step G: Preparation of O-(3-methyl-2-butenyl)-hydroxylamine hydrochloride

The product of the step F (10.3 g, 44.3 mmol) was dissolved in chloroform (40 mL) and methanol (10 mL), and hydrazine monohydrate (5.54 g, 88.6 mmol) was added thereto at room temperature and stirred for 15 hours at room temperature. The precipitated white solid was removed by Celite filtration, and the filtrate was washed with aqueous ammonia and brine, dried over magnesium sulfate and filtered. The solvent of the filtrate was distilled off under reduced pressure. The resulting crude product was dissolved in diethyl ether (50 mL), and hydrochloric acid (dioxane solution, 3 mL) was added dropwise under ice-cooling. The mixture was stirred for 30 minutes at room temperature and a white solid was collected by filtration, so that the title compound (1.76 g, 29%, white solid) was obtained.
¹H NMR (300 MHz, DMSO d6) δ = 10.8 (3H, bs), 5.30 (1H, tt, J = 7.3, 1.3Hz), 4.49 (2H, d, J = 6.3Hz), 1.75 (3H, s), 1.70 (3H, s)

### Step H: Preparation of 1-(3-(((3-methyl-2-butenyl)oxy)imino)propyl)-4-oxo-3-(3-(trifluoromethyl)phenyl)-4H-pyrido[1,2-a]pyrimidinium inner salt

The product of the step E (200 mg, 0.552 mmol) and the product of the step G (150 mg, 1.09 mmol) were added to methylene chloride (10 mL), and pyridine (130 mg, 1.64 mmol) was added thereto at room temperature and stirred at room temperature for 2 hours. Water was added thereto, and the solution was extracted twice with methylene chloride and washed with 1 N hydrochloric acid, an aqueous solution of sodium hydrogen carbonate, and brine. The solution was dried over magnesium sulfate, and filtered. The solvent of the filtrate was distilled off under reduced pressure, so that the title compound (230 mg, 94%, yellow viscous material) was obtained.

### Synthesis Example 1-2: Alternative method for preparing bis(2,4,6-trichlorophenyl)-2-(3-trifluoromethylphenyl)propanedioate in the step B in Synthesis Example 1-1

To 1,4-dioxane (200 mL), potassium phosphate (23.5 g, 110 mmol), dimethyl malonate (14.7 g, 111 mmol), 3-iodobenzotrifluoride (25.1 g, 92.3 mmol), picolinic acid (2.50 g, 20.3 mmol) and copper(I) iodide (2.50 g, 13.1 mmol) were added. After stirring the reaction mixture under reflux for 5 hours, potassium phosphate (23.5 g, 110 mmol) was added thereto, and the mixture was further stirred under reflux for 2.5 hours. After cooling of the reaction liquid to room temperature, the reaction mixture was adjusted to pH 3 with hydrochloric acid, and filtered by a filter aid to remove solids. The filtrate was extracted twice with ethyl acetate, washed once each with a saturated sodium hydrogen carbonate aqueous solution and brine, dried over magnesium sulfate, and filtered. The solvent of the filtrate was distilled off under reduced pressure by an evaporator, so that a brown oily crude product (27.9 g) was obtained.

The resulting crude product (16.9 g) was added to 11% sodium hydroxide aqueous solution (112 g) and methanol (25 mL), and the reaction mixture was stirred from room temperature to 60°C for 2 hours. Hydrochloric acid was then added to the reaction solution until the pH reached 1. The solution was extracted twice with ethyl acetate, dried over magnesium sulfate, and filtered. The solvent of the filtrate was distilled off under reduced pressure by an evaporator. To the resulting slightly brown solid (15.7 g), dichloromethane (160 mL) was added, and oxalyl chloride (15.8 mL, 184 mmol) and N,N-dimethylformamide (0.8 mL) were then added thereto. After stirring of the reaction mixture at room temperature for 1 hour, 2,4,6-trichlorophenol (24.3 g, 123 mmol) was added thereto and the mixture was further stirred for 1.5 hours. The reaction mixture was concentrated under reduced pressure, and methanol was added to the residue obtained, so that solids precipitated out slowly from the solution. The precipitated solids were collected by filtration and dried at 60°C, so that the title compound (21.5 g, 63%, white solid) was obtained.

### Synthesis Example 2: Preparation of 1-(3-(hydroxyimino)propyl)-4-oxo-3-(3-(trifluoromethyl)phenyl)-4H-pyrido[1,2-a]pyrimidinium inner salt (compound number: F-1)

The product of Step E (2.00 g, 5.52 mmol) and hydroxylamine hydrochloride (0.580 g, 8.34 mmol) were added to methylene chloride (60 mL). To the mixture, pyridine (1.34 mL, 16.6 mmol) was added dropwise at room temperature, and the mixture was stirred at room temperature for 2 hours. The solvent was distilled off under reduced pressure, and the title compound (1.75 g, 84%, yellow solid) was obtained through purification by column chromatography.

### Synthesis Example 3: Preparation of 4-oxo-1-(3-((pivaloyloxy)imino)propyl)-3-(3-(trifluoromethyl)phenyl)-4H-pyrido[1,2-a]pyrimidinium inner salt (compound number: D-1)

The compound synthesized in Synthesis Example 2 (F-1) (242 mg, 0.717 mmol) and triethylamine (0.30 mL, 2.15 mmol) were dissolved in methylene chloride (15 mL) and cooled to 0°C, to which pivaloyl chloride (0.170 mL, 1.43 mmol) was added dropwise. The solution was stirred at 0°C for 1 hour, to which a sodium hydrogen carbonate aqueous solution was added for neutralization. The solution was extracted twice with methylene chloride and washed with brine. The solution was dried over magnesium sulfate, and filtered. The solvent of the filtrate was distilled off under reduced pressure. The resulting crude product was purified by column chromatography, so that the title compound (110 mg, 33%, yellow solid) was obtained.

Synthesis Example 4: Preparation of 1-(3-(((1-ethoxyethoxy)imino)propyl)-4-oxo-3-(3-trifluoromethylphenyl)-4H-pyrido[1,2-a]-pyrimidinium inner salt (compound number: A-7)

### Step A: Preparation of 2-(1-ethoxyethoxy)isoindolidinone

N-hydroxyphthalimide (16.3 g, 100 mmol) was dissolved in dioxane (100 mL), and ethyl vinyl ether (10.8 g, 150 mmol) was added to the solution, which was then stirred for 5 minutes. Pyridinium p-toluenesulfonate (5.00 g, 19.9 mmol) was added dropwise thereto at room temperature and the mixture was stirred at room temperature for 15 hours. Water (150 mL) was added thereto and a precipitated pale yellow solid was collected by filtration. The resulting solid was dissolved in ethyl acetate, washed with a sodium hydrogen carbonate aqueous solution and brine, dried over magnesium sulfate, and filtered. The solvent of the filtrate was distilled off under reduced pressure, so that the title compound (23.5 g, quant., white solid) was obtained.
¹H NMR (CDCl₃) δ = 7.81-7.85 (2H, m), 7.71-7.77 (2H, m), 5.30 (1H, q, J = 5.4Hz), 4.17 (1H, dq, J = 7.1, 6.9Hz), 3.82 (1H, dq, J = 7.1, 6.9Hz), 1.53 (3H, d, J = 5.4Hz), 1.24 (3H, t, J = 6.9Hz)

### Step B: Preparation of O-(1-ethoxyethyl)hydroxylamine

The product of the step A (23.5 g, 100 mmol) was dissolved in chloroform (40 mL) and methanol (10 mL), and hydrazine monohydrate (13.3 g, 212 mmol) was added thereto at room temperature. The mixture was stirred at room temperature for 15 hours. The precipitated white solid was removed by Celite filtration, washed with aqueous ammonia and brine, dried over magnesium sulfate and filtered. The solvent of the filtrate was distilled off under reduced pressure, so that the title compound (5.47 g, 52%) was obtained.
¹H NMR (CDCl₃) δ = 4.78 (1H, q, J = 5.4Hz), 3.77 (1H, dq, J = 7.1, 6.9Hz), 3.58 (1H, dq, J = 7.2, 7.0Hz), 1.31 (3H, d, J = 5.4Hz), 1.23 (3H, t, J = 7.2Hz)

### Step C: Preparation of 1-(3-(((1-ethoxyethoxy)imino)propyl)-4-oxo-3-(3-trifluoromethylphenyl)-4H-pyrido[1,2-a]pyrimidinium inner salt

The product of the step E (200 mg, 0.552 mmol) and the product of the step B (87.0 mg, 0.828 mmol) of Synthesis Example 1-1, and pyridinium p-toluenesulfonate (14.0 mg, 0.0552 mmol) were dissolved in dioxane (20 mL), and heated at 60°C for 1 hour. The solution was cooled to room temperature, and washed with water and brine. The solution was dried over magnesium sulfate and filtered. The solvent of the filtrate was distilled off under reduced pressure, so that the title compound (234 mg, 95%, yellow solid) was obtained.

Other compounds were synthesized in the same manner. The isomer ratio may be different depending on reaction conditions and isolation conditions. In some cases, a single isomer structure is present. The notation of the number of protons which is not an integer in NMR is due to the mixture of geometric isomers. The structures and melting points (mp) of compounds are shown in Tables 11 to 21. The compounds marked with an asterisk represent oily or amorphous compounds. In Table 22, the values of ¹H-NMR of the compounds are shown respectively.

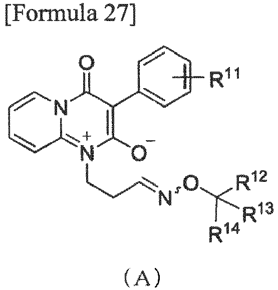

**[Table 13-1]**

| No. | R¹¹ | R¹² | R¹³ | R¹⁴ | mp (°C) |
|---|---|---|---|---|---|
| A-1 | 3-CF₃ | Me | H | H | * |
| A-2 | 3-CF₃ | Et | H | H | * |
| A-3 | 3-CF₃ | iPr | H | H | * |
| A-4 | 3-CF₃ | OMe | H | H | 111∼116 |
| A-5 | 3-CF₃ | OMe | Me | Me | 123∼132 |
| A-6 | 3-CF₃ | OEt | H | H | 113∼123 |
| A-7 | 3-CF₃ | OEt | Me | H | * |
| A-8 | 3-CF₃ | CN | H | H | * |
| A-9 | 3-CF₃ | SMe | H | H | * |
| A-10 | 3-CF₃ | CF₃ | H | H | 105∼107 |
| A-11 | 3-CF₃ | Me | Me | H | * |
| A-12 | 3-CF₃ | Me | Me | Me | 123∼141 |
| A-13 | 3-CF₃ | CO₂Me | H | H | 86∼95 |
| A-14 | 3-CF₃ | CO₂Me | Me | H | * |
| A-15 | 3-CF₃ | CH₂Cl | H | H | * |
| A-16 | 3-CF₃ | CH₂OMe | H | H | * |
| A-17 | 3,5-diCl | OEt | H | H | 123∼133 |
| A-18 | 3,5-diCl | OEt | Me | H | 86∼89 |
| A-19 | 3,5-diCl | OiPr | Me | H | 101∼104 |
| A-20 | 3-CF₃ | OiPr | Me | H | * |
| A-21 | 3,5-diCl | CH₂SMe | H | H | * |
| A-22 | 3-CF₃ | CH₂SMe | H | H | * |
| A-23 | 3,5-diCl | SMe | H | H | 139∼142 |
| A-24 | 3-OMe | CH₂SMe | H | H | * |
| A-25 | 3-OMe | SMe | H | H | * |
| A-26 | 3-CF₃ | CH₂CF₃ | H | H | * |
| A-27 | 3-GF₃ | Me | Et | H | * |
| A-28 | 3-CF₃ | nPr | H | H | * |
| A-29 | H | OEt | Me | H | * |

**[Table 13-2]**

| | | | | | |
|---|---|---|---|---|---|
| A-30 | 3-Me | OEt | Me | H | * |
| A-31 | 3-CF₃ | CH₂iPr | H | H | * |
| A-32 | 3-CF₃ | nPr | Me | H | * |
| A-33 | H | nBu | H | H | * |
| A-34 | H | nPr | H | H | * |
| A-35 | 3,5-diCl | CH₂Cl | H | H | 104∼107 |
| A-36 | 3,5-diCl | Me | Me | Me | 113∼116 |

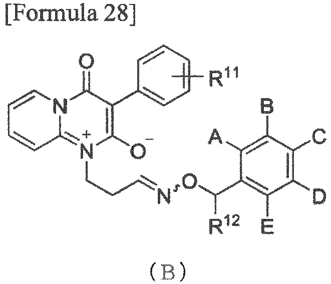

**[Table 14]**

| No. | R¹¹ | R¹² | A | B | C | D | E | mp (°C) |
|---|---|---|---|---|---|---|---|---|
| B-1 | 3-CF₃ | H | H | H | H | H | H | 79∼82 |
| B-2 | 3-CF₃ | H | Cl | H | H | H | H | 56∼63 |
| B-3 | 3-CF₃ | H | Cl | H | H | H | Cl | 51∼59 |
| B-4 | 3-CF₃ | H | I | H | H | H | H | 98∼102 |
| B-5 | 3-CF₃ | H | H | H | NO₂ | H | H | 64∼70 |
| B-6 | 3-CF₃ | H | F | F | F | F | F | 31∼34 |
| B-7 | 3-CF₃ | Me | H | H | H | H | H | 110∼116 |
| B-8 | 3-CF₃ | H | H | H | H | Cl | H | * |
| B-9 | H | Me | H | H | H | H | H | 149∼154 |

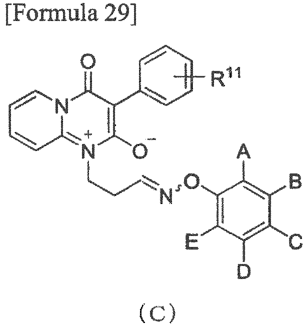

**[Table 15]**

| No. | R¹¹ | A | B | C | D | E | mp (°C) |
|---|---|---|---|---|---|---|---|
| C-1 | 3-CF₃ | H | H | H | H | H | 57∼63 |
| C-2 | 3-CF₃ | F | H | H | H | H | 80∼91 |
| C-3 | 3-CF₃ | Cl | H | H | H | H | 72∼85 |
| C-4 | 3-CF₃ | Me | H | H | H | H | 56∼62 |
| C-5 | 3-CF₃ | H | Me | H | H | H | 55∼61 |
| C-6 | 3-CF₃ | H | CF₃ | H | H | H | 49∼61 |
| C-7 | 3-CF₃ | H | NO₂ | H | H | H | 71∼79 |
| C-8 | 3-CF₃ | H | Cl | H | H | H | * |
| C-9 | 3-CF₃ | H | Cl | H | Cl | H | 67∼74 |
| C-10 | 3-CF₃ | H | H | Cl | H | H | 106 (decomp.) |
| C-11 | 3-CF₃ | H | H | OCF₃ | H | H | 58∼67 |
| C-12 | 3-CF₃ | H | H | F | H | H | 106∼112 |
| C-13 | 3-OMe | H | CF₃ | H | H | H | 51∼57 |

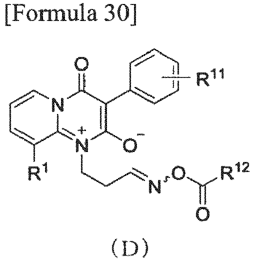

**[Table 16]**

| No. | R¹ | R¹¹ | R¹² | mp (°C) |
|---|---|---|---|---|
| D-1 | H | 3-CF₃ | tBu | 163∼166 |
| D-2 | Me | 3-CF₃ | tBu | 105∼113 |
| D-3 | H | 3-CF₃ | OMe | 121∼124 |
| D-4 | H | 3-CF₃ | cPr | 181∼184 |
| D-5 | H | 3-CF₃ | 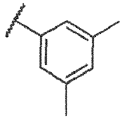 | * |
| D-6 | H | 3-CF₃ | 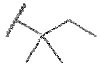 | 148∼154 |
| D-7 | H | 3-CF₃ | 1-adamantyl | 155∼163 |

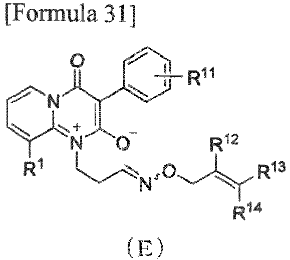

**[Table 17]**

| No. | R¹ | R¹¹ | R¹² | R¹³ | R¹⁴ | mp (°C) |
|---|---|---|---|---|---|---|
| E-1 | H | 3-CF₃ | H | H | H | * |
| E-2 | H | 3-CF₃ | Cl | H | H | * |
| E-3 | H | 3-OCF₃ | Cl | H | H | * |
| E-4 | H | 3-CF₃ | Br | H | H | * |
| E-5 | H | 3-CF₃ | H | Cl | H | * |
| E-6 | H | 3-CF₃ | H | H | Cl | * |
| E-7 | H | 3-CF₃ | H | Me | Me | * |
| E-8 | H | 3-CF₃ | Cl | Cl | H | * |
| E-9 | H | 3-CF₃ | Cl | H | Cl | * |
| E-10 | H | 3-CF₃ | H | Cl | Cl | * |
| E-11 | H | 3,5-diCl | H | Me | Me | 71∼77 |
| E-12 | H | 3,5-diCl | H | Cl | Cl | 79∼89 |
| E-13 | H | 3,5-diCl | H | Cl | Me | 69∼75 |
| E-14 | H | 3,5-diCl | H | H | Cl | 107∼113 |
| E-15 | H | 3-OCF₃ | H | H | Cl | * |
| E-16 | H | 3-Me | H | H | Cl | * |
| E-17 | Me | 3-CF₃ | H | H | Cl | * |
| E-18 | H | 3,5-diMe | H | Me | Me | 65∼79 |
| E-19 | H | 3,5-diF | H | Me | Me | * |
| E-20 | H | 3,5-diMe | H | Cl | Cl | 65∼70 |
| E-21 | H | H | H | Me | Me | * |
| E-22 | H | 3-Cl | H | Me | Me | 90∼97 |
| E-23 | H | 3,5-diF | H | Cl | Cl | * |
| E-24 | H | 3,5-diCl | Me | H | H | 76∼81 91∼93 |
| E-25 | H | 3,5-diCl | H | Cl | H | 106∼114 |
| E-26 (cis:trans mixture) | H | 3,5-diCl | H | Me | H | 105∼113 |
| E-27 | H | H | H | H | Cl | * |
| E-28 | Me | 3,5-diCl | H | H | Cl | 79∼82 |

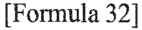

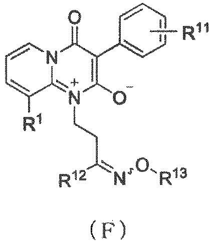

**[Table 18-1]**

| No. | R¹ | R¹¹ | R¹² | R¹³ | mp (°C) |
|---|---|---|---|---|---|
| F-1 | H | 3-CF₃ | H | H | 76∼84 |
| F-2 | H | 3-CF₃ | H | Me | 88∼91 |
| F-3 | Me | 3-CF₃ | H | H | 45∼55 |
| F-4 | Me | 3-CF₃ | H | Me | 100∼110 |
| F-5 | H | 3,5-diCl | H | H | 97∼107 |
| F-6 | H | 3-OMe | H | Me | 127∼135 |
| F-7 | Me | 3,5-diCl | H | Me | 165∼180 |
| F-8 | Me | 3,5-diCl | H | H | 80∼90 |
| F-9 | Me | 3-Me | H | H | 65∼70 |
| F-10 | Me | 3-Me | H | Me | 82∼89 |
| F-11(E-form) | H | 3-CF₃ | Me | Me | 95∼97 |
| F-12(Z-form) | H | 3-CF₃ | Me | Me | 145∼147 |
| F-13 | H | 3-CF₃ | NH₂ | H | 82∼92 |
| F-14 | H | 3-CF₃ | NH₂ | COtBu | 158∼162 |
| F-15 | H | H | H | H | 194∼198 |
| F-16 | H | 3-Me | H | H | 163∼168 |
| F-17(E-form) | H | 3-CF₃ | Me | 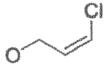 | * |
| F-18(Z-form) | H | 3-CF₃ | Me | 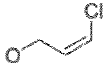 | * |
| F-19(E-form) | H | 3,5-diCl | Me | 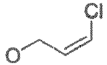 | 117∼120 |
| F-20(Z-form) | H | 3,5-diCl | Me | 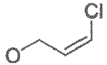 | 138∼141 |
| F-21 | Me | 3-CF₃ | H | CH₂OMe | 135∼140 |
| F-22(E-form) | H | 3,5-diCl | Me | CH₂OMe | 160∼162 |
| F-23(Z-form) | H | 3,5-diCl | Me | CH₂OMe | 123∼126 |
| F-24(E-form) | H | 3,5-diCl | Me | 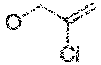 | 115∼118 |

**[Table 18-2]**

| F-25(Z-form) | H | 3,5-diCl | Me | 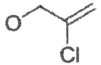 | 185∼190 |
|---|---|---|---|---|---|
| F-26 | H | 3-Me | H | Me | 45∼51 |
| F-27 | H | 3-Cl | H | H | 93∼103 |
| F-28 | H | 3,5-diF | H | H | 84∼92 |
| F-29 | H | 3-OMe | H | H | 99∼108 |

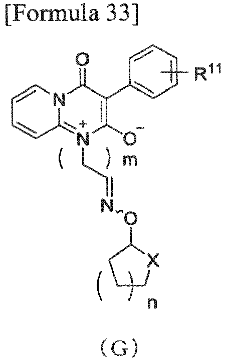

**[Table 19]**

| No. | R¹¹ | n | X | m | mp (°C) |
|---|---|---|---|---|---|
| G-1 | 3-CF₃ | 1 | CH₂ | 2 | 121∼140 |
| G-2 | 3-CF₃ | 1 | O | 2 | 64∼69 |
| G-3 | 3-CF₃ | 2 | O | 2 | 141∼155 |
| G-4 | H | 1 | O | 2 | 115∼121 |
| G-5 | 3-OCF₃ | 1 | O | 2 | 107∼110 |
| G-6 | 3-Me | 1 | O | 2 | 76∼83 |
| G-7 | 3-OMe | 1 | O | 2 | 95∼98 |
| G-8 | 3-Cl | 1 | O | 2 | 66∼74 |
| G-9 | 2-Cl | 1 | O | 2 | 88∼95 |
| G-10 | 4-Cl | 1 | O | 2 | 167∼174 |
| G-11 | 3-CF₃ | 1 | O | 1 | 59∼67 |
| G-12 | 3-CF₃ | 1 | O | 3 | 137∼139 |
| G-13 | 3,5-diCl | 1 | O | 2 | 67∼72 |
| G-14 | 2,4-diCl | 1 | O | 2 | 93∼102 |
| G-15 | 3-Me | 2 | O | 2 | 78∼83 |
| G-16 | 4-Cl | 2 | O | 2 | 80∼88 |
| G-17 | H | 2 | O | 2 | 72∼90 |

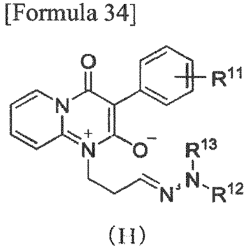

**[Table 20]**

| No. | R¹¹ | R¹² | R¹³ | mp (°C) |
|---|---|---|---|---|
| H-1 | 3-CF₃ | H | H | 84∼90 |
| H-2 | 3-CF₃ | Me | H | 34∼40 |
| H-3 | 3-CF₃ | Me | Me | 132∼134 |
| H-4 | 3-CF₃ | COPh | H | 210 |
| H-5 | 3-CF₃ | COCH₂CN | H | 150∼156 |
| H-6 | 3-CF₃ | 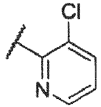 | H | 204 (decomp.) |
| H-7 | 3-CF₃ | 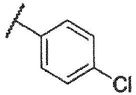 | H | 205 (decomp.) |
| H-8 | 3-CF₃ | 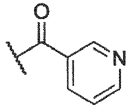 | H | 215 (decomp.) |

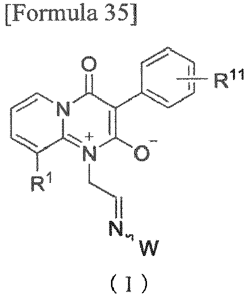

**[Table 21]**

| No. | R¹ | R¹¹ | W | mp (°C) |
|---|---|---|---|---|
| I-1 | H | 3-CF₃ | OH | 70∼80 |
| I-2 | H | 3-CF₃ | OMe | 49∼52 |
| I-3 | H | 2-F | OH | 216∼219 |
| I-4 | Me | 3-CF₃ | OH | 184 (decomp.) |
| I-5 | Me | 3-CF₃ | OMe | 125∼135 |
| I-6 | H | 3-CF₃ | NMe₂ | 149∼152 |
| I-7 | H | 3-CF₃ | SOtBu | 93∼95 |
| I-8 | H | 4-CF₃ | OH | 221∼228 |
| I-9 | H | 2-CF₃ | OH | 133∼140 |
| I-10 | H | 3-CF₃ | 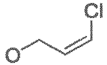 | * |
| I-11 | H | 3-CF₃ | OCH₂Cl | 108∼107 |
| I-12 | H | 3-CF₃ | 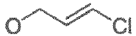 | 111∼116 |
| I-13 | H | 3,5-diCl | 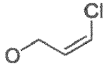 | 74∼86 |
| I-14 | H | 3,5-diCl | 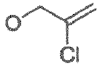 | 75∼89 |
| I-15 | Me | 3,5-diCl | 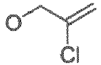 | 74∼77 |
| I-16 | Me | 3,5-diCl | 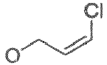 | 119∼123 |
| I-17 | H | 3,5-diCl | 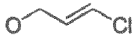 | 156∼160 |
| I-18 | Me | 3,5-diCl | 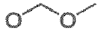 | 137∼143 |
| I-19 | Me | 3,5-diCl | 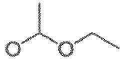 | 100∼105 |

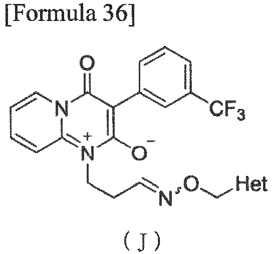

**[Table 22]**

| No. | Het | mp (°C) |
|---|---|---|
| J-1 | 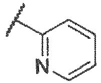 | * |
| J-2 | 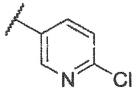 | * |
| J-3 | 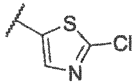 | * |

Others.

**[Table 23-1]**

| No. | Strucutre | mp (°C) |
|---|---|---|
| K-1 | 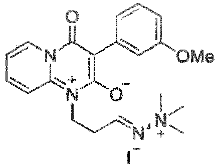 | 188∼192 |
| K-2 | 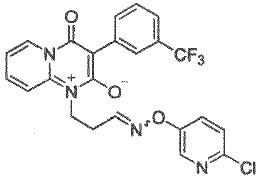 | 68∼74 |
| K-3 | 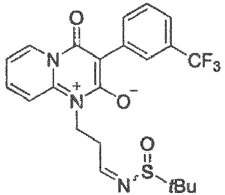 | 99∼102 |
| K-4 | 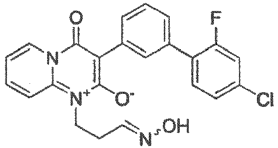 | 95∼105 |
| K-5 | 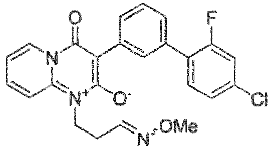 | 59∼69 |
| K-6 | 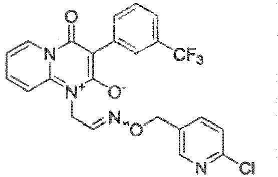 | * |

**[Table 23-2]**

| | | |
|---|---|---|
| K-7 | 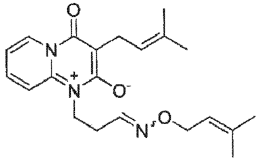 | 100∼104 |
| K-8 | 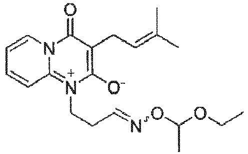 | 103∼108 |
| K-9 | 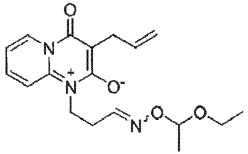 | * |
| K-10 | 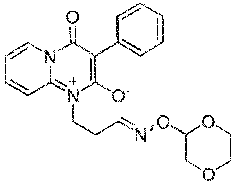 | 82∼90 |
| K-11 | 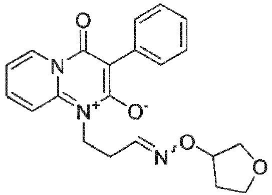 | * |

**[Table 24-1]**

| A-1 |
|---|
| ¹H NMR (300 MHz, CDCl₃): δ = 9.53 (1H, d, J = 6.9Hz), 8.09∼8.16 (2H, m), 7.99∼8.00 (1H, m), 7.77 (0.5H, d, J = 9.0Hz), 7.41∼7.56 (3H, m), 7.37 (1H, t, J = 7.5Hz), 6.91 (0.5H, t, J = 6.0Hz), 4.48∼4.58 (2H, m), 4.19 (1H, q, J = 7.0Hz), 4.05 (1H, q, J = 7.0Hz), 2.77 (1H, dt, J = 9.0, 6.0Hz), 2.68 (1H, dt, J = 9.0, 6.0Hz), 1.30 (1.5H, t, J = 7.0Hz), 1,21 (1.5H, t, J = 7.0Hz) |

| A-2 |
|---|
| ¹H NMR (300 MHz, CDCl₃): δ = 9.53 (1H, dt, J = 6.9, 1.8Hz), 8.06∼8.18 (2H, m), 7.94∼8.05 (1H, m), 7.76 (0.6H, d, J = 9.0Hz), 7.42∼7.58 (3H, m), 7.36 (1H, td, J = 7.2, 0.9Hz), 6.90 (0.4H, t, J = 6.0Hz), 4.45∼4.63 (2H, m), 4.11 (1.2H, t. J = 6.6Hz), 3.95 (0.8H, t, J = 6.6Hz), 2.78 (1.2H, dd, J = 15.9, 6.0Hz), 2.68 (0.8H, ddd, J = 13.5, 7.5, 5.7Hz), 1.71 (1.2H, sext, J = 7.5Hz), 1.62 (0.8H, sext, J = 6.9Hz), 0.98 (1.8H, t, 7.2Hz), 0.91 (1.2H, t, J = 7.5Hz) |

| A-3 |
|---|
| ¹H NMR (300 MHz, CDCl₃): δ = 9.53 (1H, d, J = 6.9Hz), 8.07∼8.14 (2H, m), 7.99∼8.00 (1H, m), 7.75 (0.5H, d, J = 9.0Hz), 7.47∼7.55 (3H, m), 7.36 (1H, t, J = 6.9Hz), 6.89 (0.5H, J = 5.8Hz), 4.48∼4.58 (2H, m), 3.93 (1H, d, J = 5.7Hz), 3.76 (1H, d, J = 7.2Hz), 2.79 (1H, dt, J = 9.3, 5.7Hz), 2.68 (1H, dt, J = 6.9, 6.9Hz), 1.86∼2.05 (1H, m), 0.95 (3H, d, J = 6.3Hz), 0.89 (3H, d, J = 6.6Hz) |

| A-4 |
|---|
| ¹H NMR (300 MHz, CDCl₃): δ = 9.54 (1H, dd, J = 6.9, 0.9Hz), 8.09∼8.20 (2H, m), 7.96∼8.04 (1H, m), 7.70 (0.3H, d, J = 5.4Hz), 7.63 (0.7H, t, J = 5.7Hz), 7.58 (0.7H, d, J = 9.0Hz), 7.46∼7.51 (2H, m), 7.38 (1H, t, J = 6.9Hz), 7.01 (0.3H, t, J = 6.0Hz), 5.15 (0.6H, s), 5.01 (1.4H, s), 4.50∼4.63 (2H, m), 3.45 (0.9H, s), 3.39 (2.1H, s), 2.86 (0.6H, dd, J = 15.0, 6.0Hz), 2.74 (1.4H, ddd, J = 12.9, 7.2, 5.4Hz) |

| A-5 |
|---|
| ¹H NMR (300 MHz, CDCl₃): δ = 9.50∼9.54 (1H, m), 8.02∼8.16 (2H, m), 7.96∼8.02 (1H, m), 7.75 (0.3H, d, J = 9.0Hz), 7.56∼7.60 (1.4H, m), 7.46∼7.49 (2H, m), 7.34∼7.39 (1H, m), 7.02 (0.3H, t, J = 7.2Hz), 4.58 (1.4H, t, J = 7.2Hz), 4.52 (0.6H, t, J = 7.8Hz), 3.23 (1H, s), 3.18 (2H, s), 2.70∼2.85 (2H, m), 1.47 (2H, s). 1.39 (4H, s) |

**[Table 24-2]**

| A-6 |
|---|
| ¹H NMR (300 MHz, CDCl₃): δ = 9.54 (1H, dd, J = 6.9, 1.5Hz), 8.08∼8.20 (2H, m), 7.95∼8.04 (1H, m), 7.67∼7.76 (0.6H, d, J = 9.0Hz), 7.54∼7.64 (1H, m), 7.42∼7.54 (2H, m), 7.37 (1H, t, J = 6.9Hz), 7.00 (0.4H, t, J = 5.7Hz), 5.22 (0.8H, s), 5.07 (1.2H, m). 4.48∼4.67 (2H, m), 3.68 (0.8H, q, J = 7.2Hz), 3.63 (1.2H, q, J = 6.9Hz), 2.84 (0.8H, dd, J = 15.3, 6.0Hz), 2.73 (1.2H, ddd, J = 13.5, 6.9, 5.7Hz), 1.22 (1.2H, t, J = 7.2Hz), 1.20 (1.8H, t, J = 6.9Hz) |

| A-7 |
|---|
| ¹H NMR (300 MHz, CDCl₃): δ = 9.54 (1H, dt, J = 6.9, 1.8Hz), 8.08∼8.19 (2H, m),7.97-8.04 (1H, m), 7.85 (0.6H, d, J = 9.3Hz), 7.51∼7.59 (1H, m), 7.45∼7.51 (2H, m), 7.38 (1H, td, J = 6.9, 0.9Hz), 6.95 (0.4H, t, J = 6.0Hz), 5.35 (0.6H, q, J = 5.4Hz), 5.17 (0.4H, q, J = 5.4Hz), 4.45∼4.63 (2H, m), 3.40∼3.90 (2H, m), 2.81 (1.2H, q, J = 7.8Hz), 2.72 (0.8H, q, J = 5.7Hz), 1.42 (1.8H, d, J = 5.4Hz), 1.36 (1.2H, d, J = 5.4Hz), 1.20 (1.8H, t, J = 6.9Hz), 1.17 (1.2H, t, J = 7.2Hz) |

| A-8 |
|---|
| ¹H NMR (300 MHz, CDCl₃): δ = 9.55 (1H, dd, J = 6.9, 1.5Hz), 8.17∼8.24 (1H, m), 8.18 (1H, s), 7.98∼8.01 (1H, m), 7.55∼7.64 (1.7H, m), 7.47∼7.52 (2H, m), 7.37∼7.42 (1H, m), 7.10 (0.3H. t, J = 5.8Hz), 4.77 (0.7H. s), 4.64 (1.3H, s), 4.52∼4.62 (2H, m). 2.74∼2.89 (2H, m) |

| A-9 |
|---|
| ¹H NMR (300 MHz, CDCl₃): δ = 9.52∼9.55 (1H, m), 8.13∼8.19 (1H, m), 8.12 (1H, s), 7.99∼8.03 (1H, m), 7.84 (0.5H, d, J = 8.7Hz), 7.55∼7.61 (1H, m), 7.47∼7.52 (2H, m), 7.38 (1H, tt, J = 6.9, 1.2Hz), 7.01 (0.5H, t, J = 6.0Hz), 5.20 (1H, s), 5.05 (1H, s), 4.51∼4.61 (2H, m), 2.71∼2.86 (2H, m), 2.25 (1.3H, s), 2.17 (1.7H, s) |

| A-10 |
|---|
| ¹H NMR (300 MHz, CDCl₃): δ = 9.53∼9.57 (1H, m), 8.11∼8.18 (2H, m), 8.00 (1H, t, J = 6.0Hz), 7.60∼7.67 (1H, m), 7.48∼7.52 (2.5H, m), 7.39 (1H, t, J = 6.0Hz), 7.04 (0.5H, t, J = 6.0Hz), 4.57 (2H, t, J = 7.2Hz), 4.53 (1H, t, J = 8.6Hz), 4.35 (1H, t, J = 8.5Hz), 2.84 (1H, dt, J = 9.6. 6.1Hz), 2.70∼2.76 (1H, m) |

**[Table 24-3]**

| A-11 |
|---|
| ¹H NMR (300 MHz, CDCl₃): δ = 9.52 (1H, dd, J = 5.1, 1.8Hz), 8.04∼8.18 (2H, m), 7.96∼8.04 (1H, m), 7.83 (0.6H, d, J = 8.7Hz), 7.42∼7.56 (3H, m), 7.35 (1H, td, J = 6.9, 0.6Hz), 6.91 (0.4H, t, J = 5.7Hz), 4.45∼4.62 (2H, m), 4.41 (0.6H, ddd, J = 6.0, 6.0, 6.0Hz), 4.26 (0.4H, ddd, J = 6.0, 6.0, 6.0Hz), 2.76 (1.2H, q, J = 6.0Hz), 2.67 (0.8H, q, J = 6.0Hz), 1.28 (3.6H, d, J = 6.0Hz), 1.18 (2.4H, d, J = 6.0Hz) |

| A-12 |
|---|
| ¹H NMR (300 MHz, CDCl₃): δ = 9.50∼9.54 (1H, m), 8.05∼8.16 (2H, m), 7.99∼8.02 (1H, m), 7.85 (0.6H, d, J = 9.3Hz), 7.53 (0.4H, d, J = 8.7Hz), 7.47∼7.49 (2H, m), 7.44 (0.4H, d, J = 5.4Hz), 7.36 (1H, dt, J = 6.9, 1.3Hz), 6.91 (0.6H, t, J = 6.0Hz), 4.57 (0.8H, t, J = 7.6Hz), 4.49 (1.2H, t, J = 8.0Hz), 2.65∼2.79 (2H, m), 1.31 (6H, s), 1.22 (3H, s) |

| A-13 |
|---|
| ¹H NMR (300 MHz, CDCl₃): δ = 9.54 (1H, dd, J = 6.9, 1.2Hz), 8.10∼8.28 (2H, m), 7.97∼8.08 (1.2H, m), 7.62 (0.8H, t, J = 5.4Hz), 7.56 (0.8H, d, J = 9.0Hz), 7.44∼7.53 (2H, m), 7.38 (1H, td, J = 7.2, 0.9Hz), 7.00 (0.2H, t, J = 6.3Hz), 4.76 (0.4H, s), 4.50∼4.68 (2H, m), 4.56 (1.6H, s), 3.81 (0.6H, s), 3.76 (2.4H, s), 2.86 (0.4H, dd, J = 15.6, 6.0Hz), 2.73 (1.6H, ddd, J = 13.0, 7.5, 5.7Hz) |

| A-14 |
|---|
| ¹H NMR (300 MHz, CDCl₃): δ = 9.48 (1H, dt, J = 6.9, 1.8Hz), 8.06∼8.20 (2H, m), 7.94∼8.06 (1.4H, m), 7.57 (0.6H, t, J = 5.7Hz), 7.51 (0.6H, d, J = 9.0Hz), 7.42∼7.51 (2H, m), 7.33 (0.4H, td, J = 7.2, 0.9Hz), 7.32 (0.6H, td, J = 7.2, 1.2Hz), 6.97 (0.4H, t, J = 6.0Hz), 4.83 (0.4H, q, J = 7.2Hz), 4.64 (0.6H, q, J = 7.2Hz), 4.44∼4.56 (2H, m), 3.77 (1.2H, s), 3.72 (1.8H, s), 2.84∼3.00 (0.4H, m), 2.62∼2.80 (1.6H, m), 1.52 (1.2H, d, J = 7.2Hz), 1.42 (1.8H, d, J = 7.2Hz) |

| A-15 |
|---|
| ¹H NMR (300 MHz, CDCl₃): δ = 9.53 (1H, td, J = 6.6, 1.2Hz), 8.08∼8.20 (2H, m), 7.96∼8.06 (1H, m), 7.85 (0.5H, d, J = 9.0Hz), 7.57 (0.5H, t, J = 5.4Hz), 7.44∼7.54 (2.5H, m), 7.38 (0.5H, td, J = 6.9, 1.2Hz), 7.37 (0.5H, td, J = 6.9, 1.2Hz), 7.00 (0.5H, t, J = 6.0Hz), 4.55 (2H, q, J = 6.6Hz), 4.42 (1H, dd, J = 5.1, 1.2Hz), 4.22 (1H, t, J = 6.0Hz), 3.80 (1H, dd, J = 5.1, 1.2Hz), 3.65 (1H, t, J = 6.6Hz), 2.82 (1H, dd, J = 15.9, 6.0Hz), 2.70 (1H, ddd, J = 13.8, 5.4, 1.5Hz) |

**[Table 24-4]**

| A-16 |
|---|
| ¹H NMR (300 MHz, CDCl₃): δ = 9.53 (1H, ddd, J = 8.1, 6.9, 1.2Hz), 8.09∼8.20 (2H, m), 7.96∼8.05 (1H, m), 7.94 (0.6H, d, J = 9.0Hz), 7.45∼7.62 (3H, m), 7.32∼7.42 (1H, m), 6.95 (0.4H, t, J = 6.0Hz), 4.46∼4.60 (2H, m), 4.33 (1.2H, dd, J = 4.5, 3.0Hz), 4.17 (0.8H, dd, J = 4.5, 3.3Hz), 3.68 (1.2H, dd, J = 4.5, 3.0Hz), 3.59 (0.8H, dd, J = 4.5, 3.3Hz), 3.39 (1.8H, s), 3.37 (1.2H, s), 2.78 (1.2H, q, J = 6.3Hz), 2.67 (0.8H, q. J = 5.4Hz) |

| A-17 |
|---|
| ¹H NMR (300 MHz, CDCl₃): δ = 9.50 (1H, dd, J = 6.9, 1.5Hz), 8.11∼8.12 (1H, m), 7.75∼7.77 (2H, m), 7.70 (0.5H, d, J = 8.7Hz), 7.55∼7.62 (1H, m), 7.38 (1H, t, J = 7.0Hz), 7.20∼7.22 (1H, m), 6.99 (0.5H, t, J = 5.5Hz), 5.20 (0.8H, s), 5.06 (1.2H, s), 4.49∼4.58 (2H, m), 3.59∼3.69 (2H, m), 2.68∼2.86 (2H, m), 1.21 (3H, q, J = 6.8Hz) |

| A-18 |
|---|
| ¹H NMR (300 MHz, CDCl₃): δ = 9.50 (1H, dt, J = 6.9, 1.8Hz), 8.11∼8.17 (1H, m), 7.84 (0.5H, d, J = 9.0Hz), 7.76∼7.77 (2H, m), 7.52∼7.57 (1H, m), 7.36∼7.40 (1H, m), 7.21 (1H, t, J = 1.8Hz), 6.95 (0.5H, t, J = 6.1Hz), 5.28 (0.5H, q, J = 5.4Hz), 5.17 (0.5H, q. J = 5.4Hz), 4.47∼4.57 (2H, m), 3.47∼3.86 (2H, m), 2.66∼2.82 (2H, m), 1.41 (1.5H, d, J = 5.4Hz), 1.36 (1.5H, d, J = 5.7Hz), 1.18 (3H, dt, J = 7.2, 6.9Hz) |

| A-19 |
|---|
| ¹H NMR (300 MHz, CDCl₃): δ = 9.46∼9.54 (1H, m), 8.14 (1H, td, J = 7.2, 1.5Hz), 7.88 (0.5H, d, J = 9.3Hz), 7.77 (2H, t, J = 2.1Hz), 7.48∼7.58 (1H, m), 7.38 (1H, t, J = 6.3Hz), 7.21 (1H, t, J = 1.8Hz), 6.95 (0.5H, t, J = 6.0Hz), 5.44 (0.5H, q, J = 5.4Hz), 5.24 (0.5H, q, J = 5.4Hz), 4.36∼4.62 (2H, m), 4.04 (0.5H, ddd, J = 6.3, 6.3, 6.3Hz), 3.95 (0.5H, ddd, J = 6.3, 6.3, 6.3Hz), 2.77 (1H, q, J = 7.2Hz), 2.70 (1H, q. J = 5.7Hz), 1.42 (1.5H, d, J = 5.4Hz), 1.35 (1.5H, d, J = 5.4Hz), 1.11∼1.18 (6H, m) |

| A-20 |
|---|
| ¹H NMR (300 MHz, CDCl₃): δ = 9.52 (1H, d, J = 6.0Hz), 8.13 (2H, s), 7.98∼8.02 (1H, m), 7.87 (0.6H, d, J = 9.0Hz), 7.45∼7.60 (3H, m), 7.36 (1H, t, J = 6.9Hz), 6.95 (0.4H, t, J = 6.0Hz), 5.44 (0.6H, q, J = 5.4Hz), 5.24 (0.4H, q, J = 5.4Hz), 4.41∼4.62 (2H, m), 3.90∼4.09 (1H, m), 2.65∼2.83 (2H, m), 1.41 (1.8H, d, J = 5.4Hz), 1.34 (1.2H, d. J = 5.4Hz). 1.05∼1.21 (7H, m) |

**[Table 24-5]**

| A-21 |
|---|
| ¹H NMR (300 MHz, CDCl₃). δ = 9.48∼9.53 (1H, m), 8.13∼8.19 (1H, m), 7.83 (0.5H, d, J = 9.0Hz), 7.77 (2H., t, J = 1.6Hz), 7.50∼7.54 (1H, m), 7.35∼7.41 (1H, m), 7.22 (1H, t, J = 2.0Hz), 6.95 (0.5H, t, J = 6.0Hz), 4.53 (2H, q, J = 7.4Hz), 4.32 (1H, t, J = 6.2Hz), 4.16 (1H, t, J = 6.6Hz), 2.64∼2.84 (4H, m), 2.14 (3H, d, J = 8.4Hz) |

| A-22 |
|---|
| ¹H NMR (300 MHz, CDCl₃): δ = 9.50 (1H, t, J = 5.6Hz), 8.11∼8.16 (2H, m), 7.99∼8.03 (1H, m), 7.81 (0.5H. d, J = 9.0Hz), 7.49∼7.53 (3H, m), 7.35 (1H, t, J = 6.9Hz), 6.94 (0.5H, t, J = 5.7Hz), 4.49∼4.57 (2H, m), 4.31 (1H, t, J = 6.2Hz), 4.16 (1H, t, J = 7.0Hz), 2.64∼2.83 (4H, m), 2.14 (3H, d, J = 9.0Hz) |

| A-23 |
|---|
| ¹H NMR (300 MHz, CDCl₃): δ = 9.51 (1H, d, J = 6.9Hz), 8.12∼8.19 (1H, m), 7.83 (0.5H, d, J = 8.4Hz), 7.77 (2H, d, J = 1.8Hz), 7.54∼7.57 (1H, m), 7.38 (1H, t, J = 7.2Hz), 7.21 (1H, brs), 7.01 (0.5H, t, J = 5.7Hz), 5.20 (0.9H, s), 5.05 (1.1H, s), 4.49∼4.59 (2H, m), 2.69∼2.85 (2H, m), 2.25 (1.4H, s), 2.17 (1.6H, s) |

| A-24 |
|---|
| ¹H NMR (300 MHz, CDCl₃): δ = 9.51∼9.56 (1H, m), 8.08∼8.15 (1H, m), 7.80 (0.5H, d, J = 8.7Hz), 7.48∼7.54 (1H, m), 7.30∼7.37 (4H, m), 6.95 (0.5H, t, J = 5.8Hz), 6.81∼6.83 (0.5H, m), 6.79∼6.80 (0.5H, m), 4.50∼4.57 (2H, m), 4.32 (1.1H, t, J = 6.2Hz), 4.17 (0.9H, t, J = 6.9Hz), 3.83 (3H, s), 2.65∼2.84 (4H, m), 2.16 (1.7H, s), 2.13 (1.3H, s) |

| A-25 |
|---|
| ¹H NMR (300 MHz, CDCl₃): δ = 9.54 (1H, d, J = 6.9Hz), 8.05∼8.17 (1H, m), 7.80 (0.5H, d, J = 9.0Hz), 7.50∼7.60 (1H, m), 7.28∼7.40 (4H, m), 7.02 (0.5H, t, J = 5.6Hz), 6.81 (1H, d, J = 7.8Hz), 5.21 (1H, s), 5.05 (1H, s), 4.45∼4.64 (2H, m), 3.83 (3H, m), 2.82 (1H, q, J = 6.0Hz), 2.74 (1H, q, J = 6.0Hz), 2.25 (1.5H, s), 2.18 (1.5H, s) |

| A-26 |
|---|
| ¹H NMR (300 MHz, CDCl₃): δ = 9.53 (1H, td, J = 4.2, 1.2Hz), 8.07∼8.19 (2H, m), 7.96∼8.04 (1H, m), 7.65 (0.5H, d, J = 9.0Hz). 7.45∼7.55 (3H, m), 7.37 (1H, td, J = 7.2, 0.9Hz), 6.97 (0.5H, t, J = 6.0Hz), 4.43∼4.60 (2H, m), 4.38 (1H, t, J = 6.0Hz), 4.21 (1H. t, J = 6.6Hz), 2.63∼2.85 (2H, m), 2.36∼2.60 (2H, m) |

**[Table 24-6]**

| A-27 |
|---|
| ¹H NMR (300 MHz, CDCl₃): δ = 9.53 (1H, dt, J = 7.2, 1.8Hz), 8.06∼8.18 (2H, m), 7.96∼8.04 (1H, m), 7.83 (0.5H, d, J = 7.2Hz), 7.55 (0.5H, d. J = 9.0Hz), 7.45∼7.51 (2.5H, m), 7.37 (1H, td, J = 6.9, 0.9Hz), 6.91 (0.5H, t, J = 6.0Hz), 4.45∼4.61 (2H, m), 4.22 (0.5H, q, J = 6.3Hz), 4.05 (0.5H, q, J = 6.3Hz), 2.63∼2.85 (2H, m), 1.40∼1.80 (2H, m), 1.26 (1.5H, d, J = 6.3Hz), 1.16 (1.5H, d, J = 6.3Hz), 0.95 (1.5H, t, J = 6.3Hz), 0.89 (1.5H, t, J = 6.3Hz) |

| A-28 |
|---|
| ¹H NMR (300 MHz, CDCl₃): δ = 9.56 (1H, d, J = 6.9Hz), 8.04∼8.15 (2H, m), 7.95∼8.04 (1H, m), 7.74 (0.5H, d, J = 9.0Hz), 7.42∼7.58 (3H, m), 7.35 (1H, t, J = 6.9Hz), 5.90 (0.5H, t, J = 5.7Hz), 4.45∼4.60 (2H, m), 4.14 (1H, t, J = 6.6Hz), 3.99 (1H, t, J = 6.6Hz), 2.77 (1H, q, J = 6.3Hz), 2.67 (1H, q, J = 6.3Hz), 1.52∼1.74 (2H, m), 1.29∼1.48 (2H, m), 0.96 (1.5H, t, J = 7.2Hz), 0.91 (1.5H, t, J = 7.2Hz) |

| A-29 |
|---|
| ¹H NMR (300 MHz, CDCl₃): δ = 9.53 (1H, dt, J = 6.9, 1.5Hz), 8.09 (1H, m), 7.81 (0.5H, d, J = 9.0Hz), 7.75 (2H, m), 7.55 (0.5H, t, J = 5.7Hz), 7.51 (0.5H, d, J = 8.7Hz), 7.36 (3H, m), 7.23 (1H, m), 6.95 (0.5H, t, J = 6.0Hz), 5.34 (0.5H, q, J = 5.4Hz), 5.18 (0.5H, q, J = 5.4Hz), 4.53 (2H, m), 3.68∼3.88 (1H, m), 3.56∼3.67 (1H, m), 2.75∼3.85 (1H, m), 2.63∼2.75 (1H, m), 1.42 (1.5H, d, J = 5.4Hz), 1.37 (1.5H, d, J = 5.4Hz), 1.20 (1.5H, t, J = 6.9Hz), 1.18 (1.5H, t, J = 7.2Hz) |

| A-30 |
|---|
| ¹H NMR (300 MHz, CDCl₃): δ = 9.53 (1H, dt, J = 6.9, 1.5Hz), 8.09 (1H, m), 7.81 (0.5H, d, J = 8.7Hz), 7.49∼7.57 (3H, m), 7.24∼7.38 (2H, m), 7.05 (1H, d, J = 7.5Hz), 6.95 (0.5H, t, J = 7.5Hz), 5.35 (0.5H, q, J = 5.1Hz), 5.18 (0.5H, q, J = 5.7Hz), 4.53 (2H, m), 3.70∼3.90 (1H, m), 3.48∼3.68 (1H, m), 2.75∼2.87 (1H, m), 2.66∼2.75 (1H, m), 2.38 (3H, s), 1.42 (1.5H, d, J = 5.4Hz), 1.37 (1.5H, d, J = 5.4Hz), 1.20 (1.5H, t, J = 6.9Hz), 1.18 (1.5H, t, J = 6.9Hz) |

**[Table 24-7]**

| A-31 |
|---|
| ¹H NMR (300 MHz, CDCl₃): δ = 9.54 (1H, dt, J = 7.5, 1.5Hz), 8.07∼8.17 (2H, m), 7.99∼8.01 (1H, m), 7.75 (0.5H, d, J = 9.0Hz), 7.55 (0.5H, d, J = 9.0Hz), 7.47∼7.52 (2.5H, m), 7.37 (1H, t, J = 6.9Hz), 6.90 (0.5H, t, J = 5.7Hz), 4.48∼4.58 (2H, m), 4.17 (1H, t, J = 6.9Hz), 4.03 (1H, t, J = 6.0Hz), 2.73∼2.83 (1H, m), 2.64∼2.72 (1H, m), 1.42∼1.76 (3H, m), 0.95 (3H, d, J = 6.3Hz), 0.90 (3H, t, J = 6.3Hz) |

| A-32 |
|---|
| ¹H NMR (300 MHz, CDCl₃): δ = 9.52 (1H, dt, J = 6.9, 1.5Hz), 8.06∼8.17 (2H, m), 7.99∼8.02 (1H, m), 7.81 (0.5H, d, J = 9.0Hz), 7.53 (0.5H, d, J = 9.0Hz), 7.44∼7.50 (2.5H, m), 7.33∼7.38 (1H, m), 6.90 (0.5H, t, J = 6.0Hz), 4.47∼4.58 (2H, m), 4.23∼4.33 (0.5H, m), 4.05∼4.17 (0.5H, m), 2.60∼2.85 (2H, m), 1.50∼1.74 (2H, m), 1.27∼1.50 (2H, m), 1.25 (1.5H, d, J = 6.3Hz), 1.16 (1.5H, d, J = 6.3Hz), 0.94 (1.5H, t, J = 7.2Hz), 0.90 (1.5H, t, J = 6.9Hz) |

| A-33 |
|---|
| ¹H NMR (300 MHz, CDCl₃): δ = 9.55 (1H, dt, J = 6.9, 1.5Hz), 8.03∼8.14 (1H, m), 7.71∼7.77 (2.5H, m), 7.47∼7.54 (1H, m), 7.32∼7.42 (3H, m), 7.21∼7.25 (1H, m), 6.90 (0.5H, t, J = 5.7Hz), 4.45∼4.59 (2H, m), 4.14 (1H, t, J = 6.6Hz), 3.99 (1H, t, J = 6.6Hz), 2.73∼2.83 (1H, m), 2.64∼2.72 (1H, m), 1.50∼1.75 (2H, m), 1.27∼1.42 (4H, m), 0.86∼0.97 (3H, m) |

| A-34 |
|---|
| ¹H NMR (300 MHz, CDCl₃): δ = 9.55 (1H, dt, J = 6.9, 1.8Hz), 8.03∼8.14 (1H, m), 7.70∼7.79 (2.5H, m), 7.46∼7.55 (1H, m), 7.30∼7.44 (3H, m), 7.19∼7.26 (1H, m), 6.90 (0.5H, t, J = 6.0Hz), 4.46∼4.60 (2H, m), 4.15 (1H, t, J = 6.9Hz), 4.00 (1H, t, J = 6.9Hz), 2.73∼2.83 (1H, m), 2.63∼2.73 (1H, m), 1.50∼1.73 (2H, m), 1.30∼1.49 (2H, m), 0.87∼1.00 (3H, m) |

| A-35 |
|---|
| ¹H NMR (300 MHz, CDCl₃): δ = 9.50 (1H, dt, J = 6.6, 1.2Hz), 8.12∼8.19 (1H, m), 7.84 (0.6H, d, J = 9.0Hz), 7.77 (2H, t, J = 2.0Hz), 7.56 (0.4H, t, J = 5.4Hz), 7.51 (0.4H, t, J = 9.0Hz), 7.35∼7.41 (1H, m), 7.21 (1H, t, J = 2.0Hz), 6.99 (0.6H, t, J = 6.0Hz), 4.49∼4.57 (2H, m), 4.39∼4.43 (1.2H, m), 4.22 (0.8H, t, J = 5.9Hz), 3.77∼3.81 (1.2H, m), 3.66 (0.8H, t, J = 5.9Hz), 2.77∼2.84 (1.2H, m), 2.65∼2.72 (0.8H, m) |

**[Table 24-8]**

| A-36 |
|---|
| ¹H NMR (300 MHz, CDCl₃): δ = 9.48∼9.52 (1H, m), 8.06∼8.17 (1H, m), 7.84 (0.6H, d, J = 9.0Hz), 7.77 (2H, t, J = 2.0Hz), 7.52 (0.4H, d, J = 9.0Hz), 7.45 (0.4H, t, J = 5.3Hz), 7.34∼7.39 (1H. m), 7.21 (1H, t, J = 1.8Hz), 6.91 (0.6H, t, J = 5.9Hz), 4.45∼4.58 (2H, m), 2.64∼2.78 (2H, m), 1.31 (5H, s), 1.22 (4H, s) |

| B-1 |
|---|
| ¹H NMR (300 MHz, CDCl₃): δ = 9.51 (0.3H, d, J = 6.8Hz), 9.41 (0.7H, d, J = 6.8Hz), 8.10 (1H, m), 7.97∼8.06 (2H, m), 7.57 (0.3H, t, J = 5.3Hz), 7.43∼7.48 (6H, m), 7.29∼7.36 (2H, m), 7.15∼7.20 (1H, m), 7.00 (0.7H, t, J = 6.0Hz), 5.18 (1.3H, s), 5.03 (0.7H, s), 4.54 (0.7H, t, J = 7.3Hz), 4.45 (1.3H, t, J = 8.7Hz), 2.71∼2.78 (2H, m) |

| B-2 |
|---|
| ¹H NMR (300 MHz, CDCl₃): δ = 9.49 (0.4H, dd, J = 6.9, 1.5Hz), 9.44 (0.6H, d, J = 6.6Hz), 7.95∼8.15 (2.4H, m), 7.54∼7.65 (1.6H, m), 7.40∼7.54 (3.8H, m), 7.29∼7.40 (2.4H, m), 7.20∼7.25 (1.2H, m), 7.00 (0.6H, t, J = 6.0Hz), 5.30 (1.2H, s), 5.14 (0.8H, s), 4.55 (0.8H, t, J = 7.5Hz), 4.48 (1.2H, t, J = 5.1Hz), 2.66∼2.84 (2H, m) |

| B-3 |
|---|
| ¹H NMR (300 MHz, CDCl₃): δ = 9.53 (0.3H, dd, J = 6.6, 1.2Hz), 9.45 (0.7H, d, J = 6.6Hz), 8.07∼8.16 (1.3H, m), 7.94∼8.02 (1H, m), 7.15∼7.60 (8H, m), 7.01 (0.7H, t, J = 6.0Hz), 5.51 (1.4H, s), 5.33 (0.6H, s), 4.40∼4.60 (2H, m), 2.60∼2.80 (2H, m) |

| B-4 |
|---|
| ¹H NMR (300 MHz, CDCl₃): δ = 9.49 (0.4H, dd. J = 6.6, 1.2Hz), 9.43 (0.6H, dd, J = 6.6, 1.2Hz). 7.95∼8.15 (2.4H, m), 7.93 (0.6H, dd, J = 7.8, 0.9Hz), 7.81 (0.4H, d, J = 7.8Hz), 7.63 (0.4H. t, J = 5.1Hz), 7.51∼7.58 (1H, m), 7.38∼7.50 (4H, m), 7.27∼7.36 (1H, m), 7.21 (0.6H, td, J = 6.9, 1.2Hz), 7.12 (0.6H, td, J = 7.2, 2.1Hz), 6.94∼7.06 (1H, m), 5.24 (1.2H, s), 5.03 (0.8H, s), 4.44∼4.60 (2H, m), 2.68∼2.84 (2H, m) |

| B-5 |
|---|
| ¹H NMR (300 MHz, CDCl₃): δ = 9.53 (0.6H, d, J = 6.9Hz), 9.49 (0.4H, d, J = 6.9Hz), 8.20 (0.8H, d, J = 8.7Hz), 8.17 (1.2H, d, J = 6.9Hz), 8.05∼8.14 (1.6H, m). 7.96∼8.04 (1H, m), 7.92 (0.4H, t, J = 7.2Hz), 7.63 (0.6H, t, J = 5.4Hz), 7.40∼7.55 (5H, m), 7.36 (0.6H, t, J = 6.6Hz), 7.31 (0.4H, t, J = 6.6Hz), 7.00 (0.4H, J = 5.7Hz), 5.19 (0.8H, s). 5.10 (1.2H, s), 4.44∼4.62 (2H, m), 2.86 (0.8H, q, J = 6.6Hz), 2.68 (1.2H, q, J = 6.6Hz) |

**[Table 24-9]**

| B-6 |
|---|
| ¹H NMR (300 MHz, CDCl₃): δ = 9.52∼9.55 (1H, m), 8.08∼8.18 (2H, m), 7.98 (1H, t, J = 4.2Hz), 7.62 (0.5H, d, J = 9.0Hz), 7.47∼7.53 (3H, m), 7.39 (1H, dt, 6.9, 0.9Hz), 6.97 (0.5H, t, 6.0Hz), 5.20 (1H, s), 5.08 (1H, s), 4.53 (1H, t, J = 7.5Hz), 4.46 (1H, t, J = 7.6Hz), 2.77 (1H, dt, J = 9.3, 6.2Hz), 2.66 (1H, dt, J = 9.3, 5.4Hz) |

| B-7 |
|---|
| ¹H NMR (300 MHz, CDCl₃): δ = 9.49 (0.4H, dd, J = 6.6, 1.5Hz), 9.45 (0.6H, d, J = 6.6Hz), 8.11 (1H, s), 7.93∼8.04 (1.4H, m), 7.53∼7.59 (2H, m), 7.45∼7.50 (2H, m), 7.36∼7.42 (3H, m), 7.27∼7.35 (2H, m), 7.20∼7.25 (1H, m), 6.96 (0.6H, t, J = 6.0Hz), 5.32 (0.6H, q, J = 6.6Hz), 5.15 (0.4H, q, J = 6.6Hz), 4.35∼4.60 (2H, m), 2.73∼2.85 (1.2H, m), 2.62∼2.70 (0.8H, m), 1.65 (1.8H, d, J = 6.6Hz), 1.49 (1.2H, d, J = 6.6Hz) |

| B-8 |
|---|
| ¹H NMR (300 MHz, CDCl₃): δ = 9.51 (0.5H, dd, J = 6.9, 1.2Hz), 9.43 (0.5H, dd, J = 6.9, 1.5Hz), 8.09∼8.11 (1H, m), 8.03∼8.07 (0.5H, m), 7.97∼8.01 (1H, m), 7.62∼7.68 (0.5H, m), 7.57 (0.5H, t, J = 5.4Hz), 7.44∼7.49 (3H, m), 7.32∼7.37 (2H, m), 7.22∼7.30 (2.5H, m), 7.17∼7.19 (0.5H, m), 6.99 (0.5H, t, J = 6.0Hz), 5.11 (1H, s), 4.99 (1H, s), 4.45∼4.56 (2H, m), 2.66∼2.82 (2H, m) |

| B-9 |
|---|
| ¹H NMR (300 MHz, CDCl₃): δ = 9.46 (1H, dt, J = 6.9, 1.2Hz), 7.71∼7.78 (2H, m), 7.53∼7.58 (2H, m), 7.18∼7.44 (9H, m), 6.95 (1H, t, J = 6.0Hz), 5.31 (1H, q, J = 6.0Hz). 4.38∼4.60 (2H, m), 2.73∼2.84 (2H, m), 1.64 (3H, d, J = 6.6Hz) |

| C-1 |
|---|
| ¹H NMR (300 MHz, CDCl₃): δ = 9.53∼9.55 (0.6H, m), 9.46∼9.49 (0.4H, m), 8.10∼8.17 (2H, m), 7.98∼8.00 (1H, m), 7.88 (0.5H, t, J = 5.4Hz), 7.74 (0.4H, d, J = 9.0Hz), 7.56 (0.6H, d, J = 8.7Hz), 7.47∼7.49 (2H, m), 7.24∼7.40 (3.5H, m), 7.00∼7.11 (3H, m), 4.60∼4.69 (2H, m), 3.03 (0.8H, dt, J = 8.7, 6.3Hz), 2.82∼2.89 (1.2H, m) |

| C-2 |
|---|
| ¹H NMR (300 MHz, CDCl₃): δ = 9.52 (1H, ddd, J = 9.0, 6.9, 1.2Hz), 8.21 (0.6H, ddd, J = 9.0, 6.9, 1.2Hz), 8.07∼8.17 (1.4H, m), 7.93∼8.05 (2H, m), 7.45∼7.57 (3H, m), 7.27∼7.45 (2H, m), 6.94∼7.20 (3H, m), 4.64 (2H, q, J = 7.2Hz), 3.00 (1.2H, ddd, J = 15.3, 6.3, 2.1Hz), 2.86 (0.8H, ddd, J = 12.9, 5.7, 1.5Hz) |

**[Table 24-10]**

| C-3 |
|---|
| ¹H NMR (300 MHz, CDCl₃): δ = 9.52 (0.4H, dd, J = 6.9, 1.2Hz), 9.47 (0.6H, dd, J = 6.9, 1.2Hz), 8.06∼8.18 (2H, m), 7.94∼8.04 (1.4H, m), 7.87 (0.6H, d, J = 8.7Hz), 7.41∼7.56 (3H, m), 7.28∼7.40 (3H, m), 7.14∼7.27 (1H, m), 7.02 (0.6H, ddd, J = 9.3, 7.8, 1.5Hz), 6.97 (0.4H, ddd, J = 9.0, 7.2, 1.5Hz), 4.66 (2H, t, J = 7.5Hz), 3.00∼3.12 (1.2H, m), 2.86 (0.8H, ddd, J = 14.7, 6.9, 5.4Hz) |

| C-4 |
|---|
| ¹H NMR (300 MHz, CDCl₃): δ = 9.54 (0.6H, dd, J = 6.9, 1.2Hz), 9.48 (0.4H, dd, J = 6.9, 1.2Hz), 8.05∼8.16 (2H, m), 7.96∼8.04 (1H, m), 7.93 (0.6H, t, J = 5.4Hz), 7.64 (0.4H, d, J = 8.7Hz), 7.56 (0.6H, d, J = 8.7Hz), 7.42∼7.52 (2H, m), 7.36 (0.6H, td, J = 6.9, 1.2Hz), 7.34 (0.4H, td, J = 6.9, 1.2Hz), 7.23∼7.27 (0.4H, m), 7.09∼7.25 (3H, m), 6.90∼7.02 (1H, m), 4.65 (2H, q, J = 7.5Hz), 3.06 (0.8H, ddd, J = 14.7, 7.5, 6.3Hz). 2.86 (1.2H, ddd, J = 14.7, 7.2, 5.4Hz), 2.21 (3H, s) |

| C-5 |
|---|
| ¹H NMR (300 MHz, CDCl₃): δ = 9.54 (0.5H, dd, J = 6.9, 1.2Hz), 9.47 (0.5H, dd, J = 7.2, 1.5Hz), 8.08∼8.18 (2H, m), 7.94∼8.04 (1H, m), 7.87 (0.5H, t, J = 5.4Hz), 7.74 (0.5H, d, J = 9.0Hz), 7.56 (0.5H, d, J = 9.0Hz), 7.43∼7.52 (2H, m), 7.37 (0.5H, td, J = 6.9, 0.9Hz), 7.35 (0.5H, td. J = 7.2, 1.2Hz), 7.25∼7.27 (0.5H, m), 7.19 (0.5H, t, J = 7.8Hz), 7.17 (0.5H, t. J = 7.5Hz), 6.81∼6.94 (3H, m), 4.58∼4.72 (2H, m), 2.96∼3.08 (1H, m), 2.85 (1H, ddd, J = 14.1, 7.5, 5.4Hz), 2.34 (1.5H, s), 2.33 (1.5H, s) |

| C-6 |
|---|
| ¹H NMR (300 MHz, CDCl₃): δ = 9.55 (0.6H, dd, J = 6.9, 1.5, 0.6Hz), 9.47 (0.4H, dd, J = 6.9, 1.5, 0.4Hz), 8.14∼8.20 (1H, m), 8.09 (1H, s), 7.91∼7.98 (2H, m), 7.66 (0.4H, d, J = 9.0Hz), 7.56 (0.6H, d, J 8.7Hz), 7.47∼7.50 (2H, m), 7.30∼7.45 (3H, m), 7.22∼7.29 (2H, m), 4.67 (0.8H, t, J = 7.5Hz), 4.65 (1.2H, t, J = 7.8Hz), 3.06 (0.8H, dt, J = 7.2. 6.0Hz), 2.85∼2.91 (1.2H, m) |

**[Table 24-11]**

| C-7 |
|---|
| ¹H NMR (300 MHz, CDCl₃): δ = 9.56 (0.6H, dd, J = 6.9, 1.2Hz), 9.49 (0.4H, dd, J = 6.6, 1.2Hz), 8.22 (0.4H, ddd, J = 10.5, 6.9, 1.8Hz), 8.21 (0.6H, ddd, J = 10.5, 6.9, 1.5Hz), 8.08 (0.4H, s), 8.05 (0.6H, s), 7.92∼8.01 (2.2H, m), 7.85∼7.92 (1H, m), 7.78∼7.81 (0.4H, m), 7.65 (0.4H, d, J = 8.7Hz), 7.58 (0.6H, d, J = 8.7Hz), 7.31∼7.52 (5.4H, m), 4.63∼4.74 (2H, m), 3.03∼3.13 (0.8H, m), 2.85∼2.95 (1.2H, m) |

| C-8 |
|---|
| ¹H NMR (300 MHz, CDCL₃): δ = 9.38∼9.48 (1H, m), 7.96∼8.11 (3H, m), 7.83 (0.5H, t, J = 5.2Hz), 7.68 (0.3H, d, J = 9.3Hz), 7.58 (0.5H, d, J = 9.3Hz), 7.45∼7.52 (2.7H, m), 7.14∼7.32 (2H, m), 7.09 (0.5H, s), 6.88∼7.03 (2.5H, m), 4.53∼4.65 (2H, m), 2.97 (1H, dt, J = 6.9, 6.6Hz), 2.79 (1H, dt, J = 6.9, 6,6Hz) |

| C-9 |
|---|
| ¹H NMR (300 MHz, CDCl₃): δ = 9.57 (0.6H, dd, J = 6.9, 1.2Hz), 9.50 (0.4H, dd, J = 6.9, 1.2Hz), 8.19 (1H, ddd, J = 8.7, 6.9, 1.8Hz), 8.06∼8.13 (1H, m), 7.93∼8.01 (1H, m), 7.89 (0.6H, t, J = 5.4Hz), 7.61 (0.4H, d, J = 8.7Hz), 7.55 (0.6H, d, J = 9.0Hz), 7.45∼7.52 (2H, m), 7.41 (1H, td, J = 6.9, 0.9Hz), 7.32 (0.4H, t, J = 5.7Hz), 7.03 (0.4H, t, J = 1.8Hz), 6.99∼7.01 (2H, m), 6.94 (0.6H, d, J = 1.8Hz), 4.58∼4.71 (2H, m), 2.97∼3.08 (0.8H, m), 2.87 (1.2H, ddd, J = 14.6, 7.2, 5.4Hz) |

| C-10 |
|---|
| ¹H NMR (300 MHz, CDCl₃): δ = 9.52 (0.6H, dd, J = 6.9, 1.2Hz), 9.48 (0.4H, dd, J = 6.9, 1.2Hz), 8.09∼8.15 (2H, m), 7.95∼8.01 (1H, m), 7.86 (0.6H, t, J = 5.4Hz), 7.64 (0.4H, d, J = 8.7Hz), 7.46∼7.50 (2H, m), 7.33∼7.39 (1H, m), 7.20∼7.28 (3H, m), 6.98∼7.04 (2H, m), 4.62 (2H, dt, J = 9.3, 7.2Hz), 3.01 (0.8H, dt, J = 7.2, 6.3Hz), 2.80∼2.86 (1.2H, m) |

| C-11 |
|---|
| ¹H NMR (300 MHz, CDCl₃): δ = 9.54 (0.6H, dd, J = 5.1, 1.2Hz), 9.48 (0.4H, dd, J = 6.9, 1.2Hz), 8.00∼8.20 (2H, m), 7.94∼8.04 (1H, m), 7.89 (0.6H, t, J = 5.4Hz), 7.64 (0.4H, d, J = 8.7Hz), 7.44∼7.56 (2.6H, m), 7.37 (0.6H, td, J = 6.9, 0.9Hz), 7.36 (0.4H. td, J = 7.2, 0.9Hz), 7.28 (0.4H, t, J = 6.0Hz), 7.03∼7.20 (4H, m), 4.64 (2H. q, J = 9.0Hz), 3.03 (0.8H, dd, J = 16.5, 7.2Hz), 2.84 (1.2H, ddd, J = 14.7, 7.2, 5.7Hz) |

**[Table 24-12]**

| C-12 |
|---|
| ¹H NMR (300 MHz, CDCl₃): δ = 9.55 (0.6H, dd, J = 6.9, 1.2Hz), 9.50 (0.4H, dd, J = 7.2, 1.2Hz), 8.08∼8.19 (2H, m), 7.94∼8.04 (1H, m), 7.87 (0.6H, t, J = 5.4Hz), 7.68 (0.4H, d, J = 9.0Hz), 7.54 (0.6H, d, J = 9.0Hz), 7.43∼7.52 (2H, m), 7.38 (0.6H, td, J = 7.2, 1.2Hz), 7.37 (0.4H, td, J = 6.9, 1.2Hz), 7.23 (0.4H, t, J = 6.0Hz), 6.90∼7.10 (4H, m), 4.65 (2H, q, J = 7.2Hz). 3.03 (0.8H, q, J = 7.2Hz), 2.84 (1.2H, ddd, J = 8.7, 7.5, 5.4Hz) |

| C-13 |
|---|
| ¹H NMR (300 MHz, CDCl₃): δ = 9.54 (0.6H, dd, J = 6.9, 1.5Hz), 9.47 (0.4H, dd, J = 7.2, 1.2Hz), 8.11 (1H, ddt, J = 7.2, 1.2Hz), 7.92 (0.6H, t, J = 5.7Hz), 7.61 (0.4H, d, J = 9.0Hz), 7.52 (0.6H, d, J = 9.0Hz), 7.20∼7.46 (8.4H, m), 6.76∼6.81 (1H, m), 4.64 (2H, q, J = 7.5Hz), 3.83 (1.2H, s), 3.81 (1.8H, s), 3.04 (0.8H, dd, J = 13.2, 7.2Hz), 2.86 (1.2H, ddd, J = 13.5, 6.9, 6.6Hz) |

| D-1 |
|---|
| ¹H NMR (300 MHz, CDCl³): δ = 9.51∼9.54 (1H, m), 8.13∼8.22 (1H, m), 8.08∼8.10 (1H, m), 7.95∼8.00 (1H, m), 7.90 (1H, t, J = 5.7Hz), 7.71 (1H, t, J = 8.7Hz), 7.45∼7.50 (2H, m), 7.36∼7.43 (1H, m), 4.65 (2H, t, J = 6.9Hz), 2.90 (2H, dt, 6.9, 5.7Hz), 1.25 (9H, s) |

| D-2 |
|---|
| H NMR (300 MHz, CDCl₃): δ = 9.46 (1H, dd. J = 6.6, 1.5Hz), 8.12 (1H, s), 7.94∼8.04 (2H, m), 7.77 (1H, t, J = 5.1Hz), 7.46∼7.50 (2H, m), 7.31 (1H, t, J = 6.9Hz), 4.74 (2H, t, J = 6.6Hz), 2.90 (2H, dt, J = 6.6, 5.1Hz), 2.80 (3H, s), 1.21 (9H) |

| D-3 |
|---|
| H NMR (300 MHz, CDCl₃): δ = 9.52∼9.55 (1H, m), 8.13∼8.19 (1H, m), 8.08 (1H, s), 7.96∼7.99 (1H. m), 7.87 (0.7H, t, J = 5.7Hz), 7.67 (1H, dd, J = 13.8. 9.0Hz), 7.47∼7.52 (2H, m), 7.37∼7.42 (1.3H, m), 4.54∼4.65 (2H, m), 3.92 (1H, s), 3.87 (2H, s), 2.86∼2.94 (2H, m) |

**[Table 24-13]**

| D-4 |
|---|
| ¹H NMR (300 MHz, CDCl₃): δ = 9.53 (1H, dd, J = 6.9, 1.2Hz), 8.15 (1H, ddd, J = 8.7, 6.9, 1.5Hz), 8.06∼8.12 (1H, m), 7.94∼8.02 (1H. m), 7.91 (1H, t, J = 8.7Hz), 7.67 (1H, d, J = 8.7Hz), 7.44∼7.56 (2H, m), 7.38 (1H, td, J = 6.9, 1.2Hz), 4.64 (2H, t, J = 6.9Hz), 2.90 (2H, dd, J = 14.1, 6.9Hz), 1.60∼1.68 (1H, m), 1.14∼1.12 (2H, m), 0.90∼1.00 (2H, m) |

| D-5 |
|---|
| H NMR (300 MHz, CDCl₃): δ = 9.52∼9.58 (1H, m), 8.16∼8.24 (1H, m), 8.09 (1H, s), 8.07 (0.6H, d, J = 8.7Hz), 7.95∼7.99 (1H, m), 7.74 (1H, t, J = 8.7Hz), 7.56 (1.4H, s), 7.37∼7.50 (4H, m), 7.23 (1H, s), 4.67 (2H, dt. J = 18.3, 6.3Hz), 2.99 (2H, dt, J = 6.9, 6.6Hz)2.37 (6H, s) |

| D-6 |
|---|
| ¹H NMR (300 MHz, CDCl₃): δ = 9.53 (1H, d, J = 6.9Hz), 8.06∼8.26 (2H, m), 7.94∼8.03 (1H, m), 7.90 (1H, t, J = 5.7Hz), 7.71 (1H, d, J = 9.0Hz), 7.44∼7.56 (2H, m), 7.39 (1H, t, J = 6.9Hz), 4.58∼4.72 (2H, m), 2.91 (2H, dd, J = 12.6, 6.3Hz), 1.48∼1.68 (8H, m), 0.85 (3H, t, J = 7.5Hz) |

| D-7 |
|---|
| ¹H NMR (300 MHz, CDCl₃): δ = 9.53 (1H, d, J = 6.9Hz), 8.11∼8.26 (1H, m), 8.09 (1H, s), 7.94∼8.04 (1H, m), 7.90 (1H, t, J = 5.7Hz), 7.70 (1H, d, J = 8.7Hz), 7.46∼7.54 (2H, m), 7.39 (1H, t, J = 6.9Hz), 4.64 (2H, t, J = 6.3Hz), 2.90 (2H, dd, J = 12.3, 6.3Hz), 2.03 (3H, bs), 1.94 (3H, s), 1.92 (3H, s), 1.60∼1.80 (6H, m) |

| E-1 |
|---|
| ¹H NMR (300 MHz, CDCl₃): δ = 9.50∼9.54 (1H, m), 8.05∼8.16 (2H, m), 7.99∼8.01 (1H, m), 7.74 (0.5H, d, J = 9.0Hz), 7.52∼7.56 (1H, m), 7.47∼7.49 (2H, m), 7.34∼7.39 (1H, m), 6.95 (0.5H, t. J = 5.8Hz), 5.86∼6.07 (1H, m), 5.18∼5.37 (2H, m), 4.63 (1H, d, J = 6.2Hz), 4.50 (1H, d, J = 5.8Hz), 4.49∼4.58 (2H, m), 2.79 (1H, dt, J = 9.4, 6.2Hz), 2.69 (1H, dt, J = 7.6, 7.1Hz) |

**[Table 24-14]**

| E-2 |
|---|
| ¹H NMR (300 MHz, CDCl₃): δ = 9.55 (0.4H, dd, J = 4.5, 1.8Hz), 9.53 (0.6H. dd, J = 5.1. 1.5Hz), 8.09∼8.20 (2H, m), 7.95∼8.05 (1H, m), 7.80 (0.6H, d, J = 9.0Hz). 7.60 (0.4H, t, J = 5.4Hz), 7.54 (0.4H, d, J = 9.0Hz), 7.45∼7.51 (2H, m), 7.38 (1H, t, J = 6.9Hz), 7.01 (0.6H, t, J = 6.0Hz), 5.49∼5.52 (0.6H, m), 5.48 (0.6H, d, J = 1.5Hz), 5.36 (0.4H, dd, J = 2.7, 1.2Hz), 5.32∼5.35 (0.4H, m), 4.72 (1.2H, s), 4.55 (2H, q, J = 7.2Hz), 4.53 (0.8H, s), 2.77∼2.89 (1.2H, m), 2.73 (0.8H, ddd, J = 14.4, 7.2, 5.7Hz) |

| E-3 |
|---|
| ¹H NMR (300 MHz, CDCl₃): δ = 9.51∼9.55 (1H, m), 8.09∼8.16 (1H, m), 7.74∼7.80 (2.5H. m), 7.60 (0.5H, t, J = 5.5Hz), 7.52 (0.5H, d, J = 9.0Hz), 7.36∼7.41 (2H, m), 7.06∼7.10 (1H, m), 7.01 (0.5H, t, J = 6.0Hz), 5.50∼5.51 (0.5H, m), 5.47 (0.5H, d, J = 1.5Hz), 5.33∼5.37 (1H, m), 4.71 (1H. d, J = 0.6Hz), 4.50∼4.58 (3H, m), 2.68∼2.86 (2H, m) |

| E-4 |
|---|
| ¹H NMR (300 MHz, CDCl₃): δ = 9.54 (1H, ddd, J = 6.3, 4.2, 1.2Hz), 8.15 (1H, ddd, J = 8.7, 6.9, 1.5Hz), 8.12 (1H, s), 7.94∼8.04 (1H, m). 7.82 (0.5H, d, J = 9.0Hz), 7.61 (0.5H, t, J = 5.4Hz), 7.53 (0.5H, d, J = 9.0Hz), 7.43∼7.51 (2H,m), 7.38 (1H, t, J = 5.7Hz), 7.01 (0.5H, t, J = 5.7Hz), 5.94 (0.5H, quin, J = 0.9Hz), 5.81 (0.5H, td, J = 1.5, 1.5Hz), 5.72 (0.5H, d, J = 1.8Hz), 5.58 (0.5H, quin, J = 0.9Hz), 4.77 (1H, s), 4.46∼4.64 (3H, m), 2.84 (1H, dd, J = 15.9, 6.3Hz), 2.73 (1H, ddd, J = 12.9, 7.3. 5.7Hz) |

| E-5 |
|---|
| ¹H NMR (300 MHz, CDCl₃): δ = 9.51∼9.55 (1H, m), 8.09∼8.18 (2H, m), 7.98∼8.03 (1H, m), 7.68 (0.5H, d, J = 9.3Hz), 7.47∼7.54 (3H, m), 7.38 (1H, t, J = 7.0Hz), 6.96 (0.5H, t, J = 6.0Hz), 6.34 (0.5H, dt, J = 13.5, 1.2Hz), 6.24 (0.5H, dt, J = 13.5, 1.2Hz), 6.00∼6.15 (1H, m). 4.45∼4.59 (4H, m), 2.65∼2.82 (2H, m) |

**[Table 24-15]**

| E-6 |
|---|
| ¹H NMR (300 MHz, CDCl₃): δ = 9.51∼9.56 (1H, m), 8.09∼8.18 (2H, m), 7.98∼8.01 (1H, m), 7.70 (0.5H, d, J = 8.7Hz), 7.47∼7.56 (3H, m), 7.35∼7.40 (1H, m), 6.97 (0.5H, t, J = 6.0Hz), 6.26 (0.5H, dt, J = 7.2, 1.3Hz), 6.13∼6.18 (0.5H, m), 5.90∼6.07 (1H, m), 4.82 (1H, dd, J = 6.3, 1.5Hz), 4.70 (1H, dd, J = 6.0, 1.5Hz). 4.48∼4.59 (2H, m), 2.66∼2.83 (2H, m) |

| E-7 |
|---|
| ¹H NMR (300 MHz, CDCl₃): δ = 9.46∼9.55 (1H td, J = 6.9, 1.5Hz), 8.08∼8.16 (1.5H, m), 7.97∼8.06 (1.5H, m), 7.82 (0.5H, d, J = 9.0Hz), 7.44∼7.57 (3H, m), 7.35 (1H, td, J = 6.9, 0.9Hz), 6.93 (0.5H, t, J = 6.0Hz), 5.43 (0.5H, td, J = 6.0, 1.2Hz), 5.37 (0.5H, td, J = 6.0, 1.2Hz), 4.64 (1H, d, 6.6Hz), 4.45∼4.60 (3H, m), 2.63∼2.82 (2H, m), 1.82 (1.5H, s), 1.76 (1.5H, s), 1.74 (1.5H, s), 1.69 (1.5H, s) |

| E-8 |
|---|
| ¹H NMR (300 MHz, CDCl₃): δ = 9.51∼9.56 (1H, m), 8.11∼8.19 (2H, m), 7.98∼8.01 (1H, m), 7.72 (0.5H, d, J = 9.0Hz), 7.59 (0.5H, t, J = 5.4Hz), 7.47∼7.52 (2.5H, m), 7.39 (1H, t, J = 6.9Hz), 7.01 (0.5H, t, J = 6.0Hz), 6.58 (0.5H, s), 6.42 (0.5H, t, J = 1.0Hz), 4.76 (1H, d, J = 0.6Hz), 4.59 (1H, d, J = 0.9Hz), 4.50∼4.56 (2H. m), 2.68∼2.85 (2H, m) |

| E-9 |
|---|
| ¹H NMR (300 MHz, CDCl₃): δ = 9.51∼9.55 (1H, m), 8.09∼8.16 (2H, m), 7.99∼8.03 (1H, m), 7.75 (0.5H, d, J = 9.0Hz), 7.59 (0.5H, t, J = 5.2Hz), 7.47∼7.54 (2.5H, m), 7.37 (1H, dt, J = 7.0, 0.9Hz), 7.02 (0.5H, t, J = 6.0Hz), 6.44 (0.5H, s), 6.33 (0.5H, s), 4.97 (1H, s). 4.77 (1H, s), 4.50∼4.59 (2H, m), 2.68∼2.86 (2H, m) |

| E-10 |
|---|
| ¹H NMR (300 MHz, CDCl₃): δ = 9.54 (1H, dt, J = 6.9, 1.5Hz), 8.10∼8.20 (2H, m), 7.95∼8.04 (1H, m), 7.65 (0.6H, d, J = 9.0Hz), 7.52 (1H, t, J = 5.1Hz), 7.44∼7.51 (2H, m), 7.38 (1H, td, J = 7.2, 0.9Hz), 6.97 (0.4H, t, J = 6.0Hz), 6.14 (0.4H, t, J = 6.9Hz), 6.06 (0.6H, t, J = 6.3Hz). 4.70 (0.8H, d, J = 6.9Hz), 4.59 (1.2H, q. J = 6.3Hz), 4.45∼4.59 (2H, m), 2.79 (0.8H, q, J = 6.0Hz), 2.69 (1.2H, q, J = 5.4Hz) |

**[Table 24-16]**

| E-11 |
|---|
| ¹H NMR (300 MHz, CDCl₃): δ = 9.50 (1H, td, J = 6.0, 3.0Hz), 8.14 (0.5H, td, J = 9.0, 3.0Hz), 8.04 (0.5H, td, J = 9.0, 3.0Hz), 7.74∼7.85 (2.5H, m), 7.45∼7.58 (1H, m), 7.36 (1H, td, J = 6.0, 3.0Hz), 7.21 (1H, t, J = 3.0Hz), 6.93 (0.5H, t. J = 6.0Hz), 5.29∼5.51 (1H, m), 4.64 (1H, d, J = 6.0Hz), 4.51 (3H, td. J = 12.0, 6.0Hz), 2.62∼2.80 (2H, m), 1.82 (1.5H, s), 1.76 (1.5H, s), 1.74 (1. 5H, s), 1.69 (1.5H, s) |

| E-12 |
|---|
| ¹H NMR (300 MHz, CDCl₃): δ = 9.51 (1H, d, J = 7.2Hz), 8.15 (1H, ddd, J = 6.9, 6.9, 1.8Hz), 7.76 (2H, d, J = 2.1Hz), 7.66 (0.5H, d, J = 9.3Hz), 7.52 (1H, t, J = 5.1Hz), 7.39 (1H, t, J = 6.9Hz), 7.21 (1H, t, J = 2.1Hz), 6.97 (0.5H, t, J = 6.0Hz), 6.13 (0.5H, t, J = 6.6Hz), 6.06 (0.5H, t, J = 6.6Hz), 4.70 (1H, d, J = 6.3Hz), 4.60 (1H. d, J = 6.3Hz), 4.58 (2H, m), 2.77 (1H, q, 6.0Hz), 2.66 (1H, q, 5.4Hz) |

| E-13 |
|---|
| ¹H NMR (300 MHz, CDCl₃): δ = 9.50 (1H, ddd, J = 6.3, 5.1, 1.2Hz), 8.15 (0.5H, td, J = 7.2, 1.5Hz), 8.12 (0.5H, td, J = 7.2, 1.8Hz), 7.77 (2H, dd, J = 1.8, 1.2Hz), 7.72 (0.5H, d, J = 9.0Hz), 7.47∼7.57 (1H, m), 7.38 (1H, t, J = 6.9Hz), 7.21 (1H, t, J = 1.8Hz), 6.95 (0.5H, t, J = 6.0Hz), 5.79∼5.90 (0.25H, m), 5.75 (0.375H, td, J = 6.6, 1.2Hz), 5.65 (0.375H, td, J = 6.0, 1.2Hz), 4.73 (0.75H, dd. J = 6.6. 0.9Hz), 4.63 (0.75H, dd, J = 6.0, 1.2Hz), 4.42∼4.64 (2.5H, m), 2.76 (1H, q, J = 6.3Hz), 2.67 (1H, q, J = 7.2Hz), 2.15∼2.20 (1.5H, d, J = 1.2Hz), 2.09∼2.15 (1.5H, m) |

| E-14 |
|---|
| ¹H NMR (300 MHz, CDCl₃): δ = 9.47 (1H, dd, J = 6.6, 1.5Hz), 8.12 (1H, ddd, J = 8.7, 7.2, 1.8Hz), 7.75 (2H, d, J = 2.1Hz), 7.67 (0.5H, d, J = 8.7Hz), 7.47∼7.55 (1H, m), 7.36 (1H, td, J = 6.9, 1.2Hz), 7.20 (1H, t, J = 2.1Hz), 6.95 (0.5H, t, 6.0Hz), 6.26 (0.5H, td, J = 6.9, 1.5Hz), 6.16 (0.5H, td, J = 7.2, 1.5Hz), 6.03 (0.5H, q, 6.3Hz), 5.93 (0.5H, q, 6.0Hz), 4.81 (1H, dd, J = 6.3, 1.2Hz), 4.70 (1H, dd, J = 6.0, 1.5Hz), 4.44∼4.60 (2H, m), 2.77 (1H, q, J = 6.3Hz), 2.66 (1H, q, J = 6.0Hz) |

**[Table 24-17]**

| E-15 |
|---|
| ¹H NMR (300 MHz, CDCl₃): δ = 9.51∼9.55 (1H, m), 8.08∼8.17 (1H, m), 7.74∼7.79 (2H, m), 7.69 (0.5H, d, J = 9.0Hz), 7.51∼7.55 (1H, m), 7.34∼7.41 (2H. m), 7.06∼7.10 (1H, m), 6.95 (0.5H, t, J = 6.0Hz), 6.26 (0.5H, dt, J = 7.2, 1.5Hz), 6.16 (0.5H, dt, J = 7.5, 1.6Hz), 6.03 (0.5H, dt, J = 7.2, 6.3Hz), 5.93 (0.5H, dt, J = 7.2, 6.0Hz), 4.82 (1H, dd, J = 6.6, 1.5Hz), 4.70 (1H, dd, J = 6.0, 1.8Hz), 4.47∼4.56 (2H, m), 2.66∼2.82 (2H, m) |

| E-16 |
|---|
| ¹H NMR (300 MHz, CDCl₃): δ = 9.51∼9.55 (1H, m), 8.05∼8.13 (1H, m), 7.66 (0.5H, d, J = 9.3Hz), 7.50∼7.55 (3H, m), 7.28∼7.37 (2H, m), 7.06 (1H, d, J = 7.5Hz), 6.96 (0.5H, t, J = 6.1Hz), 6.26 (0.5H, dt, J = 7.2, 1.3Hz), 6.16 (0.5H, dt, J = 7.2, 1.6Hz), 6.04 (0.5H, dt, J = 7.2, 6.3Hz), 5.94 (0.5H, dt, J = 7.2, 6.1Hz), 4.81 (1H, dd, J = 6.3, 1.5Hz), 4.70 (1H, dd, J = 6.0, 1.8Hz), 4.47∼4.58 (2H, m), 2.66∼2.83 (2H, m), 2.38 (3H, s) |

| E-17 |
|---|
| ¹H NMR (300 MHz, CDCl₃): δ = 9.50 (0.3H, dd, J = 6.9, 1.2Hz), 9.48 (0.7H, dd, J = 6.9, 1.2Hz), 8.13 (1H, s), 7.97∼8.04 (1H, m), 7.94 (1H, d, J = 7.2Hz), 7.42∼7.54 (2H, m), 7.38 (0.7H, t, J = 5.1Hz), 7.32 (1H, t, J = 7.2Hz), 6.75 (0.3H, t, J = 6.0Hz), 6.17 (0.3H, dt, J = 7.5, 1.5Hz), 6.14 (0.7H, dt, J = 7.2, 1.8Hz), 5.80∼5.96 (1H, m), 4.62∼4.74 (2.8H, m), 4.52 (1.2H, dd, J = 6.0, 1.8Hz), 2.74∼2.81 (3.6H, m), 2.70 (1.4H, ddd, J = 13.2, 6.6, 4.8Hz) |

| E-18 |
|---|
| ¹H NMR (300 MHz, CDCl₃): δ = 9.53 (1H, t, 6.6Hz), 8.09 (0.5H, td, J = 7.2, 1.8Hz), 7.99 (0.5H, td, J = 7.2, 1.5Hz), 7.79 (0.5H, d, J = 9.0Hz), 7.47∼7.55 (1H, m), 7.32 (3H, s), 6.93 (0.5H, t, J = 6.0Hz), 6.89 (1H, s), 5.45 (0.5H, t, J = 7.2Hz), 5.38 (0.5H, t, J = 7.2Hz), 4.65 (1H, d, J = 6.9Hz), 4.44∼4.60 (3H, m), 2.75 (1H, q, J = 6.0Hz), 2.67 (1H, q, J = 5.7Hz), 2.34 (6H. s), 1.83 (1.5H, s), 1.77 (1.5H, s), 1.75 (1.5H, s), 1.70 (1.5H, s) |

**[Table 24-18]**

| E-19 |
|---|
| ¹H NMR (300 MHz, CDCl₃): δ = 9.51 (1H, td, J = 6.9, 1.2Hz), 8.14 (0.4H, td, J = 7.2, 1.8Hz), 8.04 (0.6H, td, J = 6.9, 1.8Hz), 7.81 (0.4H, td, J = 9.0Hz), 7.31∼7.57 (4H, m), 6.94 (0.6H, t, 6.0Hz), 6.67 (1H, td, J = 9.0, 2.4Hz), 5.43 (0.6H, tt, J = 6.0, 1.2Hz), 5.37 (0.4H, tt, J = 6.0, 1.2Hz), 4.64 (1H, d, J = 7.2Hz), 4.40∼4.60 (3H, m), 2.70∼2.75 (1.2H, m), 2.62∼2.72 (0.8H, m). 1.82 (1.8H, s), 1.76 (1.2H, s), 1.74 (1.8H, s), 1.69 (1.2H, s) |

| E-20 |
|---|
| ¹H NMR (300 MHz, CDCl₃): δ = 9.54 (1H, dt, J = 6.9, 1.7Hz), 8.06∼8.14 (1H, m), 7.63 (0.5H, d, J = 9.0Hz), 7.48∼7.53 (1H, m), 7.32∼7.37 (3H, m), 6.96 (0.5H. t, J = 6.0Hz), 6.89 (1H, s), 6.14 (0.5H, t, J = 6.7Hz), 6.07 (0.5H, t, J = 6.5Hz), 4.71 (1H, d, J = 6.6Hz), 4.60 (1H, d, J = 6.3Hz), 4.47∼4.57 (2H, m), 2.65∼2.82 (2H, m), 2.34 (6H, s) |

| E-21 |
|---|
| ¹H NMR (300 MHz, CDCl₃): δ = 9.46∼9.53 (1H, m), 7.94∼8.09 (1H, m), 7.73∼7.78 (2.5H, m), 7.48∼7.51 (1H, m), 7.19∼7.40 (4H, m), 6.92 (0.5H, t, J = 5.6Hz), 5.34∼5.46 (1H, m), 4.64 (1H, d, J = 7.5Hz), 4.45∼4.55 (3H, m), 2.63∼2.78 (2H, m), 1.78 (3H, d, J = 17.7Hz), 1.71 (3H, d, J = 13.5Hz) |

| E-22 |
|---|
| ¹H NMR (300 MHz. CDCl₃): δ = 9.50 (1H, dt, J = 6.6, 1.5Hz), 7.98∼8.13 (1H, m), 7.77∼7.81 (1.5H, m), 7.69 (1H, dt, J = 7.8, 1.4Hz), 7.48∼7.53 (1H, m), 7.27∼7.37 (2H, m), 7.17∼7.21 (1H, m), 6.93 (0.5H, t, J = 5.8Hz), 5.34∼5.46 (1H, m), 4.64 (1H d, J = 7.2Hz), 4.45∼4.56 (3H, m), 2.64∼2.78 (2H, m), 1.79 (3H, d, J = 18.6Hz), 1.71 (3H, d, J = 14.4Hz) |

| E-23 |
|---|
| ¹H NMR (300 MHz, CDCl₃): δ = 9.53 (1H, dt, J = 6.9, 1.8Hz), 8.11∼8.19 (1H, m), 7.65 (0.5H. d. J = 9.0Hz). 7.36∼7.54 (4H, m), 6.97 (0.5H, t, J = 5.9Hz), 6.67 (1H, tt, J = 9.1, 2.4.Hz), 6.10 (1H, dt, J = 21.0, 5.9Hz), 4.70 (1H, d, J = 6.6Hz), 4.59 (1H, d, J = 6.3Hz), 4.47∼4.58 (2H, m), 2.65∼2.82 (2H, m) |

**[Table 24-19]**

| E-24 |
|---|
| ¹H NMR (300 MHz, CDCl₃): δ = 9.48∼9.52 (1H, m), 8.06∼8.17 (1H, m), 7.73∼7.77 (2.5H, m), 7.52∼7.57 (1H, m), 7.34∼7.39 (1 H, m), 7.21 (1H, t. J = 2.0Hz), 6.95 (0.5H, t, J = 5.9Hz), 5.00 (1H, d, J = 0.9Hz), 4.90 (1 H, d, J = 5.7Hz), 4.57 (1H, s), 4.47∼4.57 (2H, m), 4.41 (1H, s), 2.65∼2.83 (2H, m), 1.75 (3H, d, J = 18.9Hz) |

| E-25 |
|---|
| ¹H NMR (300 MHz, CDCl₃): δ = 9.47∼9.52 (1H, m), 8.09∼8.18 (1H, m), 7.76 (2H, d, J = 1.8Hz), 7.67 (0.5H, d, J = 9.3Hz), 7.49∼7.54 (1H, m), 7.38 (1H, t, J = 6.9Hz), 7.21 (1H, t, J = 2.1Hz), 6.95 (0.5H, t, J = 5.9Hz), 6.34 (0.5H, dt, J = 13.5, 1.2Hz), 6.24 (0.5H, dt, J = 13.5, 1.2Hz), 6.00∼6.14 (1H, m), 4.45∼4.59 (4H, m), 2.64∼2.80 (2H, m) |

| E-26 |
|---|
| ¹H NMR (300 MHz, CDCl₃): δ = 9.64∼9.51 (1H, m), 8.10-8.16 (0.5H, m), 8.02∼8.08 (0.5H. m), 7.78 (0.5H, brs), 7.76 (2H, d, J = 1.8Hz), 7.49∼7.54 (1H, m), 7.33∼7.39 (1H, m), 7.20 (1H, t, J = 1.8Hz), 6.92 (0.5H, t, J = 5.8Hz), 5.55∼5.88 (2H, m), 4.42∼4.56 (4H, m), 2.63∼2.79 (2H, m), 1.77 (1.5H, dd, J = 6.3, 1.2Hz), 1.71 (1.5H, dd, J = 6.3. 1.2Hz) |

| E-27 |
|---|
| ¹H NMR (300 MHz, CDCl₃): δ = 9.51∼9.56 (1H, m), 8.05-8.13 (1H, m), 7.76 (1H, d, J = 1.2Hz), 7.74 (1H, d, J = 0.9Hz), 7.67 (0.5H, d, J = 8.7Hz), 7.49∼7.54 (1H, m), 7.31∼7.41 (3H, m), 7.20∼7.24 (1H, m), 6.96 (0.5H, t, J = 5.8Hz), 6.25 (0.5H, dt, J = 7.2, 1.4Hz), 6.15 (0.5H, dt, J = 7.2, 1.7Hz), 6.03 (0.5H, dt, J = 7.5, 6.0Hz), 5.94 (0.5H, dt, J = 7.2, 6.0Hz), 4.81 (1H, dd, J = 6.3, 1.5Hz), 4.70 (1H, dd, J = 6.0, 1.8Hz), 4.48∼4.58 (2H, m), 2.75∼2.83 (1H, m), 2.65∼2.73 (1H, m) |

| E-28 |
|---|
| ¹H NMR (300 MHz, CDCl₃): δ = 9,44∼9.48 (1H, m), 7.95 (1H, d, J = 7.2Hz), 7.76∼7.78 (2H, m), 7.37 (0.7H, t, J = 5.1Hz), 7.32 (1H, t, J = 7.1Hz), 7.19∼7.22 (1H, m), 6.74 (0.3H, t, J = 5.9Hz), 6.16∼6.19 (0.3H, m), 6.14 (0.7H, dt, J = 7.2, 1.7Hz), 5.91 (0.3H, dt, J = 7.5, 6.0Hz), 5.85 (0.7H, dt, J = 7.2, 6.0Hz), 4.63∼4.68 (2.7H, m), 4.50 (1.3H, dd. J = 6.0, 1.8Hz), 2.78 (1H, s). 2.76 (2H, s), 2.66∼2.74 (2H, m) |

**[Table 24-20]**

| F-1 |
|---|
| ¹H NMR (300 MHz, CDCl₃): δ = 9.55 (0.5H, t, J = 2.0Hz), 9.52 (0.5H, t, J = 2.0Hz), 8.12∼8.17 (2H, m), 7.98∼8.00 (1H, m), 7.77 (0.5H, d, J = 8.1Hz), 7.47∼7.53 (3H, m), 7.37 (1H, t, J = 7.2Hz), 7.03∼7.10 (0.5H, m), 4.52∼4.59 (2H, m), 2.83 (1H, dt, J = 8.1, 6.3Hz), 2.70 (1H, 8.1, 6.9Hz) |

| F-2 |
|---|
| ¹H NMR (300 MHz, CDCl₃): δ = 9.53∼9.55 (1H, m), 8.12∼8.17 (2H, m), 7.99∼8.01 (1H, m), 7.68 (0.5H, d, J = 9.0Hz), 7.47∼7.56 (3H, m), 7.37 (1H, dt, J = 6.9, 0.9Hz,) 6.90 (0.5H, t, J = 5.8Hz), 4.48∼4.58 (2H, m), 3.93 (1.5H, s), 3.81 (1.5H, s), 2.77 (1H, dt, J = 9.3, 5.7Hz), 2.68 (1H, dt, J = 9.3, 5.7Hz) |

| F-3 |
|---|
| ¹H NMR (300 MHz, DMSO d6): δ = 10.8 (0.5H, s), 10.5 (0.5H, s), 9.27∼9.30 (1H, m), 8.23 (1H, d, J = 7.2Hz), 8.16 (1H, s), 8.08 (1H, d, J = 7.5Hz), 7.46∼7.56 (3H, m), 7.29 (0.5H, t, J = 5.5Hz), 6.68 (0.5H, t, J = 5.5Hz), 4.53 (2H, dt, J = 7.2, 6.9Hz), 2.71 (3H, d, J = 3.3Hz), 2.52∼2.67 (2H, m) |

| F-4 |
|---|
| ¹H NMR (300 MHz, CDCl₃): δ = 9.48∼9.52 (1H, m), 8.12 (1H, s), 7.99-8.01 (1H, m), 7.94 (1H, d, J = 7.2Hz), 7.46∼7.52 (2H, m), 7.29∼7.35 (1.7H, m), 6.68 (0.3H, t, J = 6.0Hz), 4.65∼4.71 (2H, m), 3.74 (1H, s), 3.63 (2H, s), 2.78 (3H, d, 3.9Hz), 2.63∼2.74 (2H, m) |

| F-5 |
|---|
| ¹H NMR (300 MHz, DMSO d6): δ = 10.9 (0.5H, s), 10.5 (0.5H, s), 9.29 (1H, d, J = 6.9Hz), 8.35 (1H, td, J = 7.2, 1.5Hz), 7.99 (0.5H, d, J = 9.0Hz), 7.96 (0.5H, d, J = 9.0Hz), 7.91 (2H, dd, J = 2.7, 1.8Hz), 7.54 (1H, t, J = 7.2Hz), 7.41 (0.5H, t, J = 5.7Hz), 7.35 (1H, t, J = 1.2Hz). 6.86 (0.5H, t, J = 5.4Hz), 4.46 (2H, t, J = 7.2Hz), 2.65 (1H. q, J = 6.9Hz), 2.51 (1H, m) |

| F-6 |
|---|
| ¹H NMR (300 MHz, CDCl₃): δ = 9.55 (0.5H, t, J = 1.7Hz), 9.53 (0.5H, t, J = 1.7Hz), 8.07∼8.13 (1H, m), 7.65 (0.5H, d, J = 8.7Hz), 7.47∼7.53 (1H, m), 7.31∼7.36 (4H, m), 6.90 (0.5H, t, J = 5.8Hz), 6.79-6.83 (1H, m), 4.50∼4.57 (2H, m), 3.92 (1.5H, s), 3.82 (1H, s), 3.81 (1.5H, s), 2.77 (1H, dt, J = 9.3, 6.0Hz), 2.67 (1H, dt, J = 9.3, 6.0Hz) |

**[Table 24-21]**

| F-7 |
|---|
| ¹H NMR (300 MHz, CDCl₃): δ = 9.46 (1H, t, J = 6.3Hz), 7.94 (1H, d, J = 7.2Hz), 7.76∼7.78 (2H, m), 7.29∼7.34 (1.7H, m), 7.20 (1H, s), 6.67 (0.3H, t, J = 5.8Hz), 4.62∼4.69 (2H, m), 3.73 (1H, s), 3.62 (2H, s), 2.76 (3H, d, J = 3.6Hz), 2.63∼2,72 (2H, m) |

| F-8 |
|---|
| ¹H NMR (300 MHz, DMSO d6): δ = 10.8 (0.5H, s), 10.5 (0.5H, s), 9.26 (1H, d, J = 6.9Hz), 8.23 (1H, d, J = 7.8Hz), 7.92 (2H, d, J = 1.8Hz), 7.51 (1H, t, J = 7.2Hz), 7.34 (1H, s), 7.28 (0.5H, t, J = 5.7Hz), 6.84 (0.5H, t, J = 1.8Hz). 4.51 (2H, q, J = 7.2Hz), 2.70 (3H, d, J = 3.0Hz), 2.51∼2.66 (2H, m) |

| F-9 |
|---|
| ¹H NMR (300 MHz, DMSO d6): δ = 10.8 (0.4H, s), 10.5 (0.6H, s), 9.25∼9.29 (1H, m), 8.23 (0.2H, d, J = 8.1Hz), 8.19 (0.8H, d, J = 7.2Hz), 7.42∼7.55 (3H, m), 7.29 (0.5H, t, J = 5.5Hz), 7.15∼7.21 (1H, m), 6.96 (1H, d, J = 7.5Hz), 6.67 (0.5H, t, J = 5.5Hz), 4.48∼4.56 (2H, m), 3.03 (0.5H, t, J = 6.7Hz), 2.71 (3H, d, J = 3.0Hz), 2.53∼2.66 (1.5H, m), 2.30 (3H, s) |

| F-10 |
|---|
| ¹H NMR (300 MHz, CDCl₃): δ = 9.48∼9.52 (1H, m), 7.89 (1H, d, J = 7.2Hz), 7.50∼7.56 (2H, m), 7.28∼7.35 (2.6H, m), 7.04 (1H, d, J = 7.5Hz), 6.68 (0.4H, t, J = 6.0Hz), 4.63-4.70 (2H, m), 3.75 (1.2H, s), 3.65 (1.8H, s), 2.76 (3H, d, J = 3.9Hz), 2.65∼2.74 (2H, m), 2.38 (3H, m) |

| F-11 |
|---|
| ¹H NMR (300 MHz, CDCl₃): δ = 9.53 (1H, dd, J = 6.9, 1.5Hz), 8.10∼8.16 (2H, m), 7.98∼7.99 (1H, m), 7.60 (1H, d, J = 9.0Hz), 7.47∼7.49 (2H, m), 7.36 (1H, t, J = 6.9Hz), 4.56 (2H, t, J = 7.8Hz), 3.83 (3H, s), 2.61∼2.66 (2H, m), 1.94 (3H, s) |

| F-12 |
|---|
| ¹H NMR (300 MHz, CDCl₃): δ = 9.52 (1H, d, J = 6.9Hz), 8.10∼8.16 (2H, m), 7.97∼7.98 (1H, m), 7.82 (1H, d, J = 8.7Hz), 7.47 (2H, s), 7.37 (1H, t, J = 7.0Hz), 4.47 (2H, t, J = 7.3Hz), 3.90 (3H, s), 2.73 (2H, t, J = 8.1Hz), 2.00 (3H, s) |

**[Table 24-22]**

| F-13 |
|---|
| ¹H NMR (300 MHz, DMSO d6): δ = 9.29∼9.31 (1H, m), 8.92 (1H, s), 8.29∼8.33 (1H, m), 8.15 (1H, s), 8.09 (1H, d, J = 7.5Hz), 7.92 (1H, d, J = 9.0Hz), 7.47∼7.57 (3H, m), 5.52 (2H, s), 4.46 (2H, t, J = 7.2Hz), 2.38 (2H, t, J = 7.3Hz) |

| F-14 |
|---|
| ¹H NMR (300 MHz, CDCl₃): δ = 9.51 (1H, dd, J = 6.9, 1.2Hz), 8.13∼8.19 (1H, m), 8.06 (1H, s), 7.94∼7.98 (1H, m), 7.89 (1H d, J = 9.0Hz), 7.47∼7.49 (2H, m), 7.38 (1H, dt, J = 6.9, 0.9Hz), 5.17 (2H, bs), 4.61 (2H, t, J = 7.3Hz), 2.78 (2H, t, J = 7.2Hz), 1.28 (9H, s) |

| F-15 |
|---|
| ¹H NMR (300 MHz, DMSO d6): δ = 10.9 (0.5H, s), 10.5 (0.5H, s), 9.30 (1H, d, J = 6.9Hz), 8.27∼8.35 (1H, m), 7.95 (1H, t, J = 9.9Hz), 7.63∼7.72 (2H, m), 7.50 (1H, t, J = 6.9Hz), 7.41 (0.5H, t, J = 5.7Hz), 7.30 (2H, t, J =7.8Hz), 7.15 (1H, tt, J =7.5, 1.2Hz), 6.86 (0.5H, t, J = 5.4Hz), 4.46 (2H, t, J = 7.2Hz), 2.47∼2.71 (2H, m) |

| F-16 |
|---|
| ¹H NMR (300 MHz, DMSO d6): δ = 10.9 (0.5H, s), 10.5 (0.5H, s), 9.28 (1H, d, J = 6.9Hz), 8.26∼8.35 (1H, m), 7.94 (1H, t, J = 9.9Hz), 7.37∼7.55 (3.5H, m), 7.18 (1H, t, J = 7.8Hz), 6.97 (1H, d. J = 7.5Hz), 6..86 (0.5H, t, J = 5.4Hz), 4.50 (2H, t, J = 6.9Hz), 2.46∼2.70 (2H, m), 2.30 (1H, s) |

| F-17 |
|---|
| ¹H NMR (300 MHz, CDCl₃): δ = 9.51 (1H, dd, J = 6.9, 1.5Hz), 8.08-8.14 (2H, m), 7.98∼8.01 (1H, m), 7.58 (1H, d, J = 9.0Hz), 7.45∼7.51 (2H, m), 7.35 (1H, t, J = 6.9Hz), 6.15 (1H, dt, J = 7.2, 1.7Hz), 5.96 (1H, dt, J = 7.2, 6.0Hz), 4.72 (2H, dd, J = 6.0, 1.5Hz), 4.56 (2H, t, J = 7.5Hz), 2.64 (2H, t, J = 7.7Hz), 1.95 (3H, s) |

| F-18 |
|---|
| ¹H NMR (300 MHz, CDCl₃): δ = 9.51 (1H, dd, J = 6.9, 1.5Hz), 8.10 (2H, dt, J = 8.0, 1.5Hz), 7.96∼7.99 (1H, m), 7.83 (1H, d, J = 8.7Hz), 7.46∼7.52 (2H, m), 7.36 (1H, t, J = 7.1Hz), 6.29 (1H, dt, J = 7.2, 1.2Hz), 6.09 (1 H, dt, J = 6.9, 6.6Hz), 4.79 (2H, dd, J = 6.6, 1.2Hz), 4.47 (2H, t, J = 8.0Hz), 2.74 (2H, t, J = 8.3Hz), 2.02 (3H. s) |

**[Table 24-23]**

| F-19 |
|---|
| ¹H NMR (300 MHz, CDCl₃): δ = 9.50 (1H, dd, J = 6.6, 1.2Hz), 8.11-8.16 (1H, m), 7.76 (2H, d, J = 1.8Hz), 7.58 (1H, d, J = 9.0Hz). 7.34∼7.39 (1H, m), 7.21 (1H, t, J = 1.8Hz), 6.16 (1H, dt, J = 7.2, 1.5Hz), 5.92∼5.99 (1H, m), 4.72 (2H, dd, J = 6.0, 1.8Hz), 4.53∼4.58 (2H, m), 2.61-2.66 (2H, m), 1.95 (3H, s) |

| F-20 |
|---|
| ¹H NMR (300 MHz, CDCl₃): δ = 9.48 (1H, d, J = 6.9Hz), 8.08∼8.14 (1H, m), 7.82 (1H, d, J = 9.0Hz), 7.75 (2H, d, J = 1.8Hz), 7.36 (1H, t, J = 7.1Hz), 7.21 (1H, t, J = 2.0Hz), 6.51 (1H, d, J = 9.3Hz), 6.08 (1H, dt, J = 7.2Hz), 4.79 (2H, dd, J = 6.3, 0.9Hz), 4.45 (2H, t, J = 6.9Hz), 2.73 (2H, t, J = 8.1Hz), 2.02 (3H, s) |

| F-21 |
|---|
| ¹H NMR (300 MHz, CDCl₃): δ = 9.48 (1H, d, J = 6.6Hz), 8.13 (1H, brs), 7.99∼8.01 (1H, m), 7.93 (1H, d, J = 6.6Hz), 7.46∼7.51 (1H, m), 7.30 (1H, t), 5.00 (0.5H, s), 4.82 (1.5H, s), 4.69 (2H, t, J = 6.9Hz), 3.37 (0.7H, s), 3.32 (2.3H, s), 2.71∼2.86 (2H, m), 2.80 (0.7H, s), 2.76 (2.3H, s) |

| F-22 |
|---|
| ¹H NMR (300 MHz, CDCl₃): δ = 9.50 (1H, d, J = 6.9Hz), 8.10∼8.16 (1H, m), 7.75 (2H, d, J = 1.8Hz), 7.64 (1H, d, J = 9.0Hz), 7.36 (1H, t, J = 7.2Hz), 7.21 (1H, t, J = 1.8Hz), 5.05 (2H, s), 4.57 (2H, t, J = 7.5Hz), 3.40 (3H, s), 2.68 (2H, t, J = 7.5Hz). 2.00 (1H, s) |

| F-23 |
|---|
| ¹H NMR (300 MHz, CDCl₃): δ = 9.50 (1H, d, J = 6.9Hz), 8.14 (1H, t, J = 8.0Hz), 7.85 (1H, d, J = 9.0Hz), 7.75 (2H, d, J = 1.8Hz), 7.38 (1H, t, J = 6.9Hz), 7.22 (1H, t, J = 1.8Hz), 5.14 (2H, s), 4.50 (2H, t, J = 7.8Hz), 3.47 (3H, s), 2.79 (2H, t, J = 8.1Hz), 2.04 (3H, s) |

| F-24 |
|---|
| ¹H NMR (300 MHz, CDCl₃): δ = 9.50 (1H, dd, J = 6.9, 1.5Hz), 8.10∼8.16 (1H, m), 7.76 (2H, d. J = 1.8Hz), 7.57 (1H, d, J = 9.0Hz), 7.37 (1H, t, J = 6.9Hz), 7.22 (1H, t, J = 2.0Hz), 5.34 (2H, d, J = 1.2Hz), 4.52∼4.57 (4H, m), 2.66 (2H, t, J = 7.5Hz), 2.00 (3H, s) |

**[Table 24-24]**

| F-25 |
|---|
| ¹H NMR (300 MHz, CDCl₃): δ = 9.49 (1H, dd, J = 6.9, 1.5Hz), 8.09∼8.16 (1H, m), 7.93 (1H, d, J =9.0Hz), 7.75 (2H, d, J = 1.8Hz), 7.37 (1H, t, J = 6.9Hz), 7.22 (1H, t, J = 2.0Hz), 5.54 (1H, s), 5.49 (1H, d, J = 1.5Hz), 4.70 (2H, s), 4.49 (2H, t, J = 8.1Hz), 2.76 (2H, t, J = 8.2Hz), 2.03 (3H, s) |

| F-26 |
|---|
| ¹H NMR (300 MHz, CDCl₃): δ = 9.54 (1H, dt, J = 6.9, 1.8Hz), 8.07∼8.13 (1H, m), 7.65 (0.5H, d, J = 9.0Hz), 7.47∼7.55 (3H, m), 7.28∼7.37 (2H, m), 7.06 (1H, d, J = 7.5Hz), 6.90 (0.5H, t. J = 5.9Hz), 4.47∼4.57 (2H, m), 3.93 (1.5H, s), 3.82 (1.5H, s), 2.73∼2.81 (1H, m), 2.64∼2.71 (1H, m), 2.38 (3H, m) |

| F-27 |
|---|
| ¹H NMR (300 MHz, DMSO d6): δ = 10.9 (0.5H, s), 10.5 (0.5H, s), 9.30 (1H, d, J = 6.9Hz). 8.30∼8.37 (1H, m), 7.97 (1H, t, J = 9.4Hz), 7.81 (1H, q, J = 2.1Hz), 7.72∼7.76 (1H, m), 7.52 (1H, t, J = 6.9Hz), 7.41 (0.5H, t. J = 5.7Hz), 7.33 (1H, t, J = 7.8Hz), 7.19∼7.23 (1H, m), 6.86 (0.5H, t, J = 5.4Hz), 4.46 (2H, t, J = 6.9Hz), 2.62∼2.69 (1H, m), 2.50∼2.55 (1H, m) |

| F-28 |
|---|
| ¹H NMR (300 MHz, DMSO d6): δ = 10.9 (0.5H, s), 10.5 (0.5H, s), 9.30 (1H, d, J = 6.9Hz), 8.32∼8.38 (1H, m), 7.97 (1H, t, J = 9.3Hz), 7.60∼7.68 (2H, m), 7.53 (1H, tt, J = 7.2, 1.2Hz), 7.42 (0.5H, t, J = 5.7Hz), 6.97 (1H, tt, J = 9.3, 2.4Hz), 6.87 (0.5H, t, J = 5.4Hz), 4.46 (2H, t, J = 7.5Hz), 2.62∼2.69 (1H, m), 2.50∼2.55 (1H, m) |

| F-29 |
|---|
| ¹H NMR (300 MHz, DMSO d6): δ = 10.9 (0.5H, s), 10.5 (0.5H, s), 9.30 (1H, d, J = 6.9Hz), 8.28∼8.35 (1H, m), 7.95 (1H, t, J = 9.6Hz), 7.50 (1H, t, J = 6.9Hz), 7.41 (0.5H, t, J = 5.7Hz), 7.32∼7.34 (0.5H, m), 7.26∼7.30 (1.5H, m), 7.18∼7.23 (1H, m), 6.86 (0.5H, t, J = 5.4Hz), 6.73∼6.77 (1H, m), 4.45 (2H, t, J = 6.3Hz), 3.74 (1H, s), 2.62∼2.69 (1H, m), 2.50∼2.55 (1H, m) |

**[Table 24-25]**

| G-1 |
|---|
| ¹H NMR (300 MHz, CDCl₃): δ = 9.52 (1H, dd, J = 6.9, 1.5Hz), 8.06∼8.20 (2H, m), 7.97∼8.06 (1H, m), 7.88 (0.6H, d, J = 6.0Hz), 7.41∼7.56 (3H, m), 7.37 (1H, t, J = 7.2Hz), 6.92 (0.4H, t, J = 6.0Hz), 4.76 (0.6H, ddd, J = 8.7, 5.7, 3.0Hz), 4.42∼4.67 (2.4H, m), 2.75 (1.2H, dd, J = 5.7, 1.5Hz), 2.69 (0.8H, dd, J = 5.7, 1.2Hz), 1.43∼1.95 (8H, m) |

| G-2 |
|---|
| ¹H NMR (300 MHz, CDCl₃): δ = 9.53 (1H, dd, J = 6.9, 0.9Hz), 8.06∼8.18 (2H, m), 7.92∼8.04 (1 H, m), 7.72 (0.3H, d, J = 8.7Hz), 7.61 (0.7H, d, J = 9.0Hz), 7.44∼7.56 (2.7H, m), 7.37 (1H, td, J = 7.2, 1.2Hz), 7.00 (0.3H, t, J = 5.7Hz), 5.79 (0.3H, dd, J = 5.7, 1.8Hz), 5.68 (0.7H, dd, J = 5.4, 1.8Hz), 4.46∼4.68 (2H, m), 3.86∼4.00 (2H, m), 2.73∼2.85 (0.6H, m), 2.72 (1.4H, ddd, J = 13.8, 7.2, 2.1Hz), 1.76∼2.24 (4H, m) |

| G-3 |
|---|
| ¹H NMR (300 MHz, CDCl₃): δ = 9.51 (1H, d, J = 6.9Hz), 8.06∼8.14 (2H, m), 7.98∼8.02 (1 H, m), 7.73 (0.4H, d, J = 9.0Hz), 7.63 (0.6H, t, J = 5.6Hz), 7.58 (0.6H, d, J = 8.7Hz), 7.46∼7.48 (2H, m), 7.35 (1H, dt, J = 6.9, 0.6Hz), 7.04 (0.4H, t, J = 6.1Hz), 5.16∼5.21 (1H, m), 4.52∼4.60 (2H, m), 3.81∼3.92 (1H, m), 3.52∼3.65 (1H, m), 2.82∼2.91 (0.8H, m), 2.74 (1.2H, dt, J = 7.2, 5.7, 1.2Hz), 1.52∼1.87 (6H, m) |

| G-4 |
|---|
| ¹H NMR (300 MHz, CDCl₃): δ = 9.54 (1H, d, J = 6.9Hz), 8.08 (1H, q, J = 6.3Hz), 7.75 (2H, d, J = 7.5Hz), 7.68 (0.4H, d, J = 9.0Hz), 7.57 (1H, d, J = 9.0Hz), 7.52 (0.6H, t, J = 5.4Hz), 7.30∼7.45 (1H, m), 7.22 (0.6H, m), 6.99 (0.4H, t, J = 5.7Hz), 5.79 (0.4H, dd, J = 5.4, 1.5Hz), 5.69 (0.6H, d, J = 5.1Hz), 4.45∼4.63 (2H, m), 3.85∼4.05 (2H, m), 2.80 (0.8H, dd, J = 5.4, 3.0Hz), 2.72 (1.2H, dd, J = 5.7, 2.4Hz), 1.80∼2.20 (4H, m) |

| G-5 |
|---|
| ¹H NMR (300 MHz, CDCl₃): δ = 9.53 (1H, dd, J = 6.9, 1.2Hz), 8.12 (1H, ddd, J = 6.9, 6.9, 1.5Hz), 7.67∼7.82 (2.4H, m), 7.59 (0.6H, d, J = 8.7Hz), 7.52 (0.6H, t, J = 5.4Hz), 7.37 (2H, dd, J = 6.3, 1.2Hz), 7.09 (0.6H, dd, 1.2, 0.9Hz), 7.07 (0.4H, d, J = 1.2Hz), 6.99 (0.4H, t, 6.0Hz), 5.79 (0.4H, dd, J = 5.1, 1.8Hz), 5.68 (0.6H, dd, J = 5.1, 1.5Hz), 4.54 (2H, ddd, J = 7.5, 7.5, 7.5Hz), 3.85∼4.00 (2H, m), 2.81 (0.8H, dd, J = 7.5, 2.1Hz), 2.72 (1.2H, dd, J = 7.2, 1.8Hz), 1.80∼2.20 (4H, m) |

**[Table 24-26]**

| G-6 |
|---|
| ¹H NMR (300 MHz, CDCl₃): δ = 9.53 (1H, dd, J = 6.9, 1.5Hz), 8.08 (1 H, ddd, J = 8.7, 6.9, 1.8Hz), 7.68 (0.4H, d, J = 8.7Hz), 7.46∼7.62 (3.2H, m), 7.27∼7.38 (2H, m), 7.06 (1H, d, J = 7.5Hz), 6.99 (0.4H, t, J = 6.0Hz), 5.79 (0.4H, dd, J = 5.1, 1.2Hz), 5.69 (0.6H, dd, J = 5.4, 1.8Hz), 4.45∼4.62 (2H, m), 3.86∼4.00 (2H, m), 2.74∼2.86 (0.8H, m), 2.71 (1.2H, dd, J - 6.9, 1.5Hz), 2.38 (1H, s), 1.80∼2.18 (4H, m) |

| G-7 |
|---|
| ¹H NMR (300 MHz, CDCl₃): δ = 9.54 (1H, d, J = 6.9Hz), 8.09 (1H, dd, J = 6.9, 1.8Hz), 7.69 (0.4H, d, J = 8.7Hz), 7.57 (0.6H, d, J = 8.7Hz), 7.52 (0.6H, t, J = 5.4Hz), 7.28∼7.40 (4H, m), 7.00 (0.4H, t, J = 5.7Hz), 6.76∼6.84 (1H, m), 5.80 (0.4H, dd, J = 5.4, 1.5Hz), 5.69 (0.6H, d, J = 5.1Hz), 4.45∼4.63 (2H, m), 3.86∼4.00 (2H, m), 3.83 (3H, s), 2.73∼2.86 (0.8H, m), 2.72 (1.2H, ddd, J = 8.1, 3.0Hz), 1.77∼2.20 (4H, m) |

| G-8 |
|---|
| ¹H NMR (300 MHz, CDCl₃): δ = 9.52 (1H, d, J =7.2Hz), 8.11 (1H, ddd, J = 8.7, 6.9, 1.8Hz), 7.77∼7.83 (1H, m), 7.65∼7.72 (1.4H, m), 7.58 (0.6H, d, J = 9.0Hz), 7.52 (0.6H, t, J = 5.4Hz), 7.26∼7.40 (2H, m), 7.20 (1H, dd, J = 9.0, 0.9Hz), 6.99 (0.4H, t, J = 6.0Hz), 5.79 (0.4H, dd, J = 5.4, 1.5Hz), 5.68 (0.6H, dd, J = 5.1, 1.5Hz), 4.46∼4.62 (2H, m), 3.85∼4.00 (2H, m), 2.79 (0.8H, dd, J = 7.2. 2.7Hz), 2.71 (1.2H, dd, J = 7.2, 1.8Hz), 1.80∼2.20 (4H, m) |

| G-9 |
|---|
| ¹H NMR (300 MHz, CDCl₃): δ = 9.50 (1H, d, J = 6.9Hz), 8.11 (1H, ddd, J = 7.2, 5.4, 1.8Hz), 7.71 (0.4H, d, J = 8.7Hz), 7.60 (0.6H, dd, J = 9.0, 2.7Hz), 7.51 (0.6H, t, J = 5.4Hz), 7.46 (2H, dd, J = 7.8, 2.6Hz), 7.20∼7.39 (3H, m). 6.98 (0.4H, t, J = 5.4Hz), 5.79 (0.4H, d, J = 5.4Hz), 5.69 (0.6H, dt, J = 5.1, 1.8Hz), 4.45∼4.67 (2H, m), 3.85∼4.01 (2H, m), 2.73∼2.87 (0.8H, m), 2.72 (1.2H, q, J = 7.2Hz), 1.83∼2.20 (4H, m) |

**[Table 24-27]**

| G-10 |
|---|
| ¹H NMR (300 MHz, CDCl₃): δ = 9.51 (1H, d, J = 6.9Hz), 8.10 (1H, td, J = 6.9, 1.8Hz), 7.74 (2H, dd. J = 8.7, 1.8Hz), 7.67 (0.4H, d, J = 9.0Hz), 7.57 (0.6H, d, J = 9.0Hz), 7.52 (0.6H, t, J = 5.7Hz), 7.30∼7.39 (3H, m), 6.99 (0.4H, t, J = 5.7Hz), 5.79 (0.4H, dd, J = 5.4, 1.5Hz), 5.68 (0.6H, dd, J = 5.1. 1.5Hz), 4.54 (1.2H, q, J = 7.2Hz), 4.52 (0.8H, q, J = 7.2Hz), 3.85∼4.00 (2H, m), 2.79 (0.8H, dd, J = 7.5, 2.1Hz), 2.71 (1.2H, dd, J = 7.2, 1.5Hz), 1.80∼2.20 (4H, m) |

| G-11 |
|---|
| ¹H NMR (300 MHz, CDCl₃): δ = 9.54 (1H, td, J = 6.9, 1.2Hz), 8.08∼8.20 (2H, m), 7.95∼8.04 (1H, m), 7.83 (0.7H, d, J = 9.0Hz), 7.35∼7.55 (4H, m), 6.80 (0.3H, t, J = 4.5Hz), 5.90 (0.3H, dd, J = 5.4, 2.1Hz), 5.76 (0.7H, d, J = 5.1Hz), 5.40 (0.3H. dd, J = 17.1, 4.2Hz), 5.14∼5.27 (1H, m), 5.06 (0.7H, dd, J = 15.6, 6.0Hz), 3.87∼4.10 (2H, m), 1.75∼2.30 (4H, m) |

| G-12 |
|---|
| ¹H NMR (300 MHz, CDCl₃): δ = 9.54 (0.1H, dd, J = 6.9, 1.5Hz), 9.49 (0.9H, d. J = 6.6Hz), 8.20 (1.9H, dd, J = 4.5, 1.2Hz), 8.14 (1H, bs), 8.10∼8.13 (0.1H, m), 7.96∼8.06 (1H, m), 7.54 (0.9H, t, J = 6.9Hz), 7.42∼7.51 (2H, m), 7.29∼7.41 (1H, m), 6.85 (0.1H, t, J = 5.7Hz), 5.79 (1H, dd, J = 5.1, 1.5Hz), 4.30∼4.60 (2H, m), 3.84∼4.05 (2H, m), 2.40∼2.60 (2H, m), 1.80∼2.20 (6H, m) |

| G-13 |
|---|
| ¹H NMR (300 MHz, CDCl₃): δ = 9.50 (1H, d, J = 6.9Hz), 8.14 (1H, dddd, J = 9.6, 9.0, 7.2, 1.5Hz), 7.77 (0.6H, d, J = 1.8Hz), 7.76 (1.4H, d, J = 1.8Hz), 7.71 (0.3H, d, J = 8.7Hz), 7.59 (0.7H, d, J = 9.0Hz), 7.52 (0.7H, t, J = 5.7Hz), 7.37 (1H, t, J = 6.9Hz), 7.21 (1H, dd, J = 2.1, 1.2Hz), 6.99 (0.3H, t, J =6.0Hz), 5.79 (0.3H, dd, J = 5.4, 1.5Hz), 5.66 (0.7H, dd, J = 5.4, 1.8Hz), 4.45∼4.66 (2H, m), 3.85∼4.00 (2H, m), 2.03∼2.85 (2H, m), 1.75∼2.20 (4H, m) |

**[Table 24-28]**

| G-14 |
|---|
| ¹H NMR (300 MHz, CDCl₃): δ = 9.48 (1H, d, 9.0Hz), 8.13 (1H, dd, J = 6.9, 1.5Hz), 7.72 (0.4H, d, J = 9.0Hz), 7.61 (0.6H, dd, J = 9.0, 3.3Hz), 7.46∼7.54 (1.6H, m), 7.32∼7.43 (2H, m), 7.24∼7.31 (1H, m), 6.99 (0.4H, t, J = 5.7Hz), 5.79 (0.4H, d, J = 5.4Hz), 5.68 (0.6H, dt, J = 5.1, 2.1Hz), 4.45∼4.67 (2H, m), 3.85∼4.00 (2H, m), 2.65∼2.85 (2H, m), 1.80∼2.20 (4H, m) |

| G-15 |
|---|
| ¹H NMR (300 MHz, CDCl₃): δ = 9.54 (1H, dt, J = 6.9, 1.5Hz), 8.03∼8.11 (1H, m), 7.71 (0.4H, d, J = 8.7Hz), 7.63 (0.6H, t, J = 5.7Hz), 7.50∼7.57 (3H, m), 7.28∼7.36 (1.5H, m), 7.02∼7.07 (1.5H, m), 5.17∼5.24 (1H, m), 4.55 (2H, q, J = 7.6Hz), 3.83-3.93 (1H, m), 3.55∼3.66 (1H, m), 2.83∼2.90 (0.8H, m), 2.70∼2.77 (1.2H, m), 2.38 (3H, s), 1.72∼1.88 (2H, m), 1.55∼1.57 (4H, m) |

| G-16 |
|---|
| ¹H NMR (300 MHz, CDCl₃): δ = 9.51 (1H, d, J = 6.9Hz), 8.04∼8.13 (1H, m), 7.69∼7.76 (2.5H, m), 7.63 (0.5H, t, J = 5.7Hz), 7.56 (0.5H, d, J = 9.0Hz), 7.31∼7.32 (3H, m), 7.04 (0.5H, t, J = 5.9Hz), 5.16∼5.22 (1H, m), 4.54 (2H, q, J = 7.5Hz), 3.82∼3.89 (1H, m), 3.52∼3.65 (1H, m), 2.82∼2.89 (0.8H, m), 2.69∼2.77 (1.2H, m), 1.75∼1.86 (2H, m), 1.50∼1.65 (4H, m) |

| G-17 |
|---|
| ¹H NMR (300 MHz, CDCl₃): δ = 9.51 (1H, dt, J = 6.9, 1.3Hz), 8.01∼8.09 (1H, m). 7.72∼7.76 (2H, m), 7.69 (0.5H, d, J = 9.3Hz), 7.62 (0.5H, t, J = 5.7Hz), 7.54 (0.5H, d, J = 9.0Hz), 7.19∼7.41 (4H, m), 7.04 (0.5H, t, J = 6.0Hz), 5.17∼5.23 (1H, m), 4.54 (2H, q, J = 7.2Hz), 3.82∼3.90 (1H, m), 3.52∼3.65 (1H, m), 2.82∼2.89 (0.8H, m), 2.69∼2.76 (1.2H, m), 1.73∼1.86 (2H, m), 1.52∼1.66 (4H, m) |

| H-1 |
|---|
| ¹H NMR (300 MHz, CDCl₃): δ = 9.51∼9.54 (1H, m), 8.09-8.15 (2H, m), 7.93∼8.00 (1H, m), 7.82 (0.3H, t, J = 4.6Hz), 7.57 (1H, d, J = 9.5Hz), 7.45∼7.50 (3H, m), 7.32∼7.38 (1H, m), 7.20 (0.7H, t, J = 4.9Hz), 4.63 (0.6H, t, J = 7.0Hz), 4.55 (1.4H, t, J = 7.5Hz), 2.78∼2.84 (0.6H, m), 2.62∼2.69 (1.4H, m) |

**[Table 24-29]**

| H-2 |
|---|
| ¹H NMR (300 MHz, CDCl₃): δ = 9.51 (1H, dd, J = 6.9, 1.3Hz), 8.10 (2H, dt, J = 9.0, 1.8Hz), 7.98∼8.00 (1H, m), 7.60 (0.5H, d, J = 9.0Hz), 7.45∼7.53 (3H, m), 7.34 (1H, dt, J = 6.6, 0.9Hz), 6.94 (0.5H, t, J = 4.9Hz), 4.55 (2H, t, J = 7.5Hz), 2.79 (3H, s), 2.61∼2.72 (2H, m) |

| H-3 |
|---|
| ¹H NMR (300 MHz, CDCl₃): δ = 9.52 (0.5H, d, J = 1.5Hz), 9.50 (0.5H, d, J = 1.5Hz), 8.07∼8.13 (2H, m), 7.99∼8.02 (1H, m), 7.62 (1H, d, 9.0Hz), 7.45∼7.52 (2H, m), 7.34 (1H, dt, J = 7.0, 1.0Hz), 6.67 (1H, s), 4.55 (2H, t, J = 7.5Hz), 2.74 (6H, s), 2.67∼2.72 (2H, m) |

| H-4 |
|---|
| ¹H NMR (300 MHz, DMSO d6): δ = 11.6 (1H, s), 9.32 (1H, dd, J = 6.9Hz, 1.5Hz), 8.36 (1H, t, J = 7.5Hz), 8.06 (1H, s), 7.95∼8.10 (2H, m), 7.70∼7.95 (3H, m), 7.38∼7.60 (6H, m), 4.57 (2H, t, J = 6.5Hz), 2.71 (2H, dd, J = 6.5, 4.5Hz) |

| H-5 |
|---|
| ¹H NMR (300 MHz, CDCl₃): δ = 9.54 (1H, dd, J = 7.2, 1.5Hz), 9.35 (1H, s), 8.16∼8.21 (1H, m), 8.05 (1H, s). 7.93∼7.96 (1H, m), 7.39∼7.55 (5H, m), 4.61 (2H, t, J = 7.2Hz), 3.62 (2H, s), 2.69∼2.79 (2H, m) |

| H-6 |
|---|
| ¹H NMR (300 MHz, CDCl₃): δ = 9.51 (1H, dd, J = 6.9, 1.2Hz), 8.47 (1H, bs), 8.10∼8.20 (2H, m), 8.04 (1H, s), 7.93 (1H, td, J = 4.5, 1.2Hz), 7.74 (1H, d, J = 9.0Hz), 7.56 (1H, dd, J = 7.8, 0.9Hz), 7.49∼7.54 (1H, m), 7.47 (2H, d, J = 5.4Hz), 7.37 (1H, td, J = 6.9, 0.9Hz), 6.73 (1H, dd, J = 7.8, 5.1Hz). 4.66 (2H, t, J = 6.9Hz), 2.95 (2H, dd, J = 12.4, 6.9Hz) |

| H-7 |
|---|
| ¹H NMR (300 MHz, DMSO d6): δ = 9.41 (1H, s), 9.32 (1H, dd, J = 6.9, 1.2Hz), 8.38 (1H, td, J = 6.9, 1.2Hz), 8.18 (1H, s), 8.11 (2H, t, J = 9.3Hz), 7.48∼7.65 (3H, m), 7.18 (2H, dt, J = 9.0, 2.1Hz), 7.03 (2H, dt, J = 9.0, 2.1Hz), 6.62 (1H, t, J = 5.1Hz), 4.55 (2H, t, J = 7.2Hz), 2.68 (2H, dd, J = 7.2, 5.4Hz) |

**[Table 24-30]**

| H-8 |
|---|
| ¹H NMR (300 MHz, DMSO d6): δ = 11.8 (1H, s), 9.33 (1H, dd, J = 6.6, 1.2Hz), 8.74 (2H, dd, J = 4.5, 3.0Hz), 8.35 (1H, td, J = 5.7, 1.2Hz), 8.11 (1H, s), 8.03 (2H, t, J = 6.9Hz), 7.86 (1H, t, J = 5.1Hz), 7.72 (2H, dd, J = 6.0, 1.5Hz), 7.40∼7.55 (3H, m),4.57 (2H, t, J = 6.9Hz), 2.71 (2H, dd, J = 6.3, 5.7Hz) |

| I-1 |
|---|
| ¹H NMR (300 MHz, CDCl₃): δ = 9.50∼9.56 (1H, m), 8.07∼8.17 (2H, m), 7.98∼8.02 (1H, m), 7.65 (0.5H, d, J = 8.7Hz), 7.55 (1H, d, J = 9.0Hz), 7.48∼7.50 (2H, m)7.35∼7.39 (1H, m), 6.81∼6.83 (0.5H, m), 5.34 (1H, d, J = 5.1Hz), 5.13 (1H, d, J = 5.4Hz), *OH 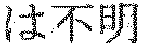 |

| 1-2 |
|---|
| ¹H NMR (300 MHz, CDCl₃): δ = 9.51∼9.55 (1H, m), 8.11∼8.17 (2H, m), 7.98∼8.02 (1H, m), 7.73 (0.6H, d, J = 9.3Hz), 7.38∼7.49 (4H, m), 6.70 (0.4H, t, J = 5.0Hz), 5.27 (0.8H, d, J = 4.8Hz), 5.11 (1.2H, d, J = 5.7Hz), 4.05 (1.3H, s), 3.86 (1.7H, s) |

| 1-3 |
|---|
| ¹H NMR (300 MHz, DMSO d6): δ = 11.6 (0.3H, s), 10.9 (0.7H, s), 9.23∼9.29 (1H, m), 8.32∼8.38 (1H, m), 7.84 (0.7H, d, J = 9.0Hz), 7.37∼7.58 (3H, m), 7.26∼7.34 (1H, m), 7.12∼7.18 (2H, m), 6.80 (0.3H, t, J = 4.0Hz), 5.16 (0.7H, d, J = 3.9Hz), 5.09 (1.3H, d, J = 3.3Hz) |

| I-4 |
|---|
| ¹H NMR (300 MHz, DMSO d6): δ = 11.3 (0.8H, s), 10.8 (0.2H, s), 9.28∼9.32 (1H, m), 8.21 (2H, d, J = 8.4Hz), 8.07∼8.16 (1.2H, m), 7.47∼7.57 (3H, m), 7.03 (0.8H, t, J = 3.5Hz), 5.13 (0.2H, d, J = 3.6Hz), 4.94 (1.8H, d, J = 3.6Hz), 2.70 (0.4H, s), 2.59 (2.6H, s) |

| I-5 |
|---|
| ¹H NMR (300 MHz, CDCl₃): δ = 9.48∼9.52 (1H, m), 8.14 (1H, s), 8.01∼8.03 (1H, m), 7.94∼7.96 (1H, m), 7.46∼7.58 (2.3H, m), 7.30∼7.35 (1H, m), 7.00 (0.7H, t, J = 3.7Hz), 5.18 (0.7H, d, J = 4.5Hz), 5.05 (1.3H, d. J = 3.6Hz), 3.95 (2H, s), 3.76 (1H, s), 2.80 (1H, s), 2.68 (2H, s) |

**[Table 24-31]**

| I-6 |
|---|
| ¹H NMR (300 MHz, CDCl₃): δ = 9.50 (0.5H, d, J = 1.2Hz), 9.47 (0.5H, d, J = 1.2Hz), 8.13 (1H, s), 8.05∼8.11 (1H, m), 8.00∼8.03 (1H, m), 7.95 (1H, d, J = 8.7Hz), 7.45∼7.49 (2H, m), 7.34 (1H, dt, J = 6.9, 1.2Hz), 6.51 (1H, t, J = 5.4Hz), 5.09 (2H, d, J = 5.7Hz), 2.82 (6H, s) |

| I-7 |
|---|
| ¹H NMR (300 MHz, DMSO d6): δ = 9.33 (1H, dd, J = 6.9, 1.2Hz), 8.31∼8.37 (1H, m), 8.05∼8.12 (3H, m), 7.89 (1H, d, J = 9.0Hz), 7.48∼7.58 (3H, m), 5.51 (2H, s), 0.96 (9H, s) |

| I-8 |
|---|
| ¹H NMR (300 MHz, DMSO d6): δ = 11.6 (0.8H, s), 11.0 (0.2H, s), 9.29∼9.35 (1H, m), 8.33∼8.39 (1H, m), 7.97∼8.03 (2H, m), 7.85 (0.2H, d, J = 8.7Hz), 7.65 (2H, d, J = 8.4Hz), 7.51∼7.60 (1.8H, m), 7.47 (0.2H, t, J = 3.6Hz), 6.81 (0.8H, t, J = 3.9Hz), 5.17 (1.6H, d, J = 3.9Hz), 5.11 (0.4H, d, J = 3.6Hz) |

| I-9 |
|---|
| ¹H NMR (300 MHz, DMSO d6): δ = 11.6 (0.5H, s), 11.0 (0.5H, s), 9.21∼9.27 (1H, m), 8.33∼8.41 (1H, m), 7.85 (0.5H, d, J = 8.7Hz), 7.74 (1H, J = 9.0Hz), 7.48∼7.69 (3.5H, m), 7.44 (0.5H, t, J = 3.6Hz), 7.34 (1H, t, J = 8.4Hz), 6.75 (0.5H, t, J = 3.9Hz), 5.20 (0.5H, dd, J = 20.1, 3.9Hz), 5.05∼5.15 (1.5H, m) |

| I-10 |
|---|
| ¹H NMR (300 MHz, CDCl₃): δ = 9.50∼9.54 (1H, m), 8.10∼8.16 (2H, m), 7.98∼8.03 (1H, m), 7.72 (0.7H, d, J = 8.7Hz), 7.44∼7.52 (3H, m), 7.36∼7.41 (1H, m), 6.77 (0.3H, t, J = 4.7Hz), 6.31 (0.4H, dt, J = 7.2, 1.4Hz), 6.19 (0.6H, dt, J = 7.5, 1.5Hz), 6.11 (0.4H, dt, J = 7.2, 6.3Hz), 5.93 (0.6H, dt, J = 7.5, 6.0Hz), 5.28 (0.7H, d, J = 5.1Hz), 5.11 (1.3H, d, J = 5.7Hz), 4.94 (0.7H, dd, J = 6.3, 1.5Hz), 4.76 (1.3H, dd, J = 6.3, 1.5Hz) |

**[Table 24-32]**

| I-11 |
|---|
| ¹H NMR (300 MHz. CDCl₃): δ = 9.54 (1H, dd, J = 6.9, 1.5Hz), 8.08-8.22 (2H, m), 7.94∼8.05 (1H, m), 7.70 (0.5H, d, J = 9.0Hz), 7.65 (0.5H, d, J = 8.7Hz), 7.54 (0.5H, t. J = 5.4Hz), 7.45∼7.52 (2H, m), 7.41 (1H, t, J = 6.3Hz), 6.81 (0.5H, t, J = 5.1Hz), 5.34 (1H, d, J = 5.4Hz), 5.14 (1H, d, J = 5.4Hz), 4.46∼4.54 (1H, m), 4.29 (1H, t, J = 5.7Hz), 3.81∼3.89 (1H, m), 3.66 (1H, t, J = 6.0Hz) |

| I-12 |
|---|
| ¹H NMR (300 MHz, CDCl₈): δ = 9.47∼9.59 (1H, m), 8.09∼8.20 (2H, m), 7.95∼8.05 (1H, m), 7.70 (0.8H, d, J = 9.0Hz), 7.35∼7.55 (4H, m), 6.75 (0.2H, t, J = 4.8Hz), 6.40 (0.2H, d, J = 13.5Hz), 6.24 (0.8H, d, J = 9.0Hz), 6.12∼6.24 (0.2H, m), 5.96∼6.08 (0.8H, m), 5.28 (0.4H, d. J = 5.1Hz), 5.13 (1.6H, d, J = 5.7Hz), 4.71 (0.4H, d, J = 6.9Hz), 4.52 (1.6H, dd, J = 6.6, 1.2Hz) |

| I-13 |
|---|
| ¹H NMR (300 MHz, CDCl₃): δ = 9.45∼9.55 (1H, m), 8.14 (1H, ddd, J = 9.0, 7.2, 2.4Hz), 7.76 (2.1H, d, J = 2.4Hz), 7.72 (0.6H, d, J = 9.0Hz), 7.34∼7.53 (2H, m), 7.21 (1H, t, J = 1.8Hz), 6.76 (0.3H, t, J = 5.1Hz), 6.31 (0.3H, dt, J = 5.7, 1.5Hz), 6.18 (0.7H, dt, J = 7.5, 1.5Hz), 6.12 (0.3H, td, J = 7.2, 6.9Hz), 5.93 (0.7H, td, J = 7.2, 6.9Hz), 5.26 (0.6H, d, J = 4.8Hz), 5.09 (1.4H, d, J = 5.7Hz), 4.94 (0.6H, dd, J = 6.0, 1.2Hz), 4.76 (1.4H, dd, J = 6.3, 1.5Hz) |

| I-14 |
|---|
| ¹H NMR (300 MHz, CDCl₃): δ = 9.49 (1H, dd, J = 6.9, 1.2Hz), 8.08∼8.17 (1H, m), 7.76 (2H, d, J = 2.1Hz), 7.69 (0.8H, d, J = 9.0Hz), 7.53∼7.60 (1H, m), 7.37∼7.43 (1H, m), 7.22 (1H, t, J = 2.1Hz), 6.79 (0.2H, t, J = 5.1Hz), 5.54 (0.2H, brs), 5.50 (0.2H, d, J = 1.5Hz), 5.36 (1.6H, s), 5.32 (0.4H, d, J = 5.1Hz), 5.12 (1.6H, d, J = 5.7Hz), 4.80 (0.4H, s), 4.60 (1.6H, s) |

| I-15 |
|---|
| ¹H NMR (300 MHz, CDCl₃): δ = 9.42∼9.52 (1H, m), 7.97 (0.7H, d. J = 7.2Hz), 7.93 (0.3H, d, J = 8.1Hz), 7.80 (1.4H, d, J = 1.8Hz), 7.78 (0.6H, d, J = 1.8Hz), 7.68 (0.3H, t, J = 4.2Hz), 7.29∼7.40 (1H, m), 7.18∼7.24 (1H, m), 7.10 (0.7H, t, J = 3.6Hz), 5.45 (1.4H, dd, J = 11.7, 1.5Hz), 5.30 (0.6H, s), 5.20 (0.6H. d, J = 4.2Hz), 5.08 (1.4H, d, J = 3.6Hz), 4.68 (1.4H, s). 4.48 (0.6H, s), 2.76 (0.9H, s), 2.70 (2.1H, s) |

**[Table 24-33]**

| I-16 |
|---|
| ¹H NMR (300 MHz, CDCl₃): δ = 9.47 (1H, t, J = 6.6Hz), 7.95 (1H, t, J = 6.6Hz), 7.80 (1.4H, d, J = 1.8Hz), 7.78 (0.6H, d, J = 1.8Hz), 7.59∼7.63 (0.3H, m), 7.30∼7.37 (1H, m), 7.20 (1H, t, J = 2.1Hz), 7.06 (0.7H, t, J = 3.6Hz), 6.23 (0.7H, dt, J = 7.2, 1.2Hz), 6.14 (0.3H, dt, J = 7.2, 1.5Hz), 5.98∼6.05 (0.7H, m), 5.83∼5.90 (0.3H, m), 5.18 (0.6H, d, J = 4.2Hz), 5.03 (1.4H, d, J = 3.6Hz), 4.84 (1.4H, dd, J = 6.0, 1.5Hz), 4.65 (0.6H, dd, J = 6.0, 1.5Hz), 2.79 (0.9H, s), 2.68 (2.1H, s) |

| I-17 |
|---|
| ¹H NMR (300 MHz, CDCl₃): δ = 9.49∼9.54 (1H, m), 8.12∼8.19 (1H, m), 7.76∼7.77 (2H, m), 7.69 (0.7H, d, J = 9.0Hz), 7.38∼7.50 (2H, m), 7.21∼7.23 (1H, m), 6.74 (0.3H, t, J = 5.1Hz), 6.40 (0.3H, dt, J = 13.5, 1.2Hz) 6.14∼6.26 (1H, m), 5.97∼6.06 (0.7H, m), 5.26 (0.6H, d, J = 5.1Hz), 5.10 (1.4H, d, J = 5.4Hz), 4.71 (0.6H, dd, J = 6.9, 1.2Hz), 4.52 (1.4H, dd, J = 6.6, 1.2Hz) |

| I-18 |
|---|
| ¹H NMR (300 MHz, CDCl₃): δ = 9.48 (1H, d, J = 6.9Hz), 7.96 (1H, t, J = 6.9Hz), 7.79 (2H, dd, J = 3.0, 1.8Hz), 7.74 (0.5H, t, J = 4.7Hz), 7.31∼7.38 (1H, m), 7.21 (1H, t, J = 2.0Hz), 7.10 (0.5H, t, J = 3.6Hz), 5.16 (1.5H, s), 5.15 (0.5H, s), 5.10 (1.2H, d, J = 3.9Hz), 4.99 (0.8H, s), 3.47 (1.8H, s), 3.35 (1.2H, s), 2.80 (1.2H, s), 2.70 (1.8H, s) |

| I-19 |
|---|
| ¹H NMR (300 MHz, CDCl₃): δ = 9.48 (1H, t, J = 6.5Hz), 7.95 (1H, t, J = 7.8Hz), 7.79 (2H, dd, J = 7.2, 1.8Hz), 7.63 (0.4H, t, J = 4.0Hz), 7.35 (1H, t, J = 6.6Hz), 7.21 (1H, t, J = 1.8Hz), 7.05 (0.6H, t, J = 3.8Hz), 5.32 (0.6H, q, J = 5.5Hz), 5.20∼5.21 (0.7H, m), 5.09 (0.4H, q, J = 5.4Hz), 5.05 (1.3H, d, J = 3.6Hz), 3.76∼3.86 (0.6H, m), 3.56∼3.65 (1H, m), 3.39∼3.48 (0.4H, m), 2.80 (1H, s), 2.70 (2H, s), 1.38 (2H, d, J = 5.4Hz), 1.32 (1H, d, J = 5.4Hz), 1.21 (2H, t, J = 7.2Hz), 1.13 (1H, t, J = 6.9Hz) |

| J-1 |
|---|
| ¹H NMR (300 MHz, CDCl₃): δ = 9.46 (1H, d, J = 6.6Hz), 8.83 (0.6H, d, J = 4.5Hz), 8.73 (0.4H, d, J = 4.8Hz), 8.41 (0.6H, t, J = 7.6Hz), 8.08∼8.31 (3H, m), 7.90∼8.02 (1H, m), 7.68∼7.86 (3H, m), 7.44∼7.50 (2H, m), 7.36∼7.41 (1H, m), 7.03 (0.4H, t, J = 5.2Hz), 5.52 (2H, d, J = 10.8Hz), 4.61 (2H, dt, J = 6.9, 6.6Hz), 3.00 (1H, dt, J = 7.8, 6.0Hz), 2.71 (1H, d, J = 5.7Hz) |

**[Table 24-34]**

| J-2 |
|---|
| ¹H NMR (300 MHz, CDCl₃): δ = 9.53 (0.6H, dd, J = 6.9, 1.2Hz), 9.47 (0.4H, dd, J = 7.2, 1.2Hz), 8.34 (1H, d, J = 15.0Hz), 8.11∼8.18 (2H, m), 7.98∼8.00 (1H, m), 7.88∼7.94 (0.5H, m), 7.28∼7.66 (6H, m), 6.98 (0.5H, t, J = 5.7Hz), 5.09 (1H, s), 4.99 (1H, s), 4.49∼4.56 (2H, m), 2.64∼2.84 (2H, m) |

| J-3 |
|---|
| ¹H NMR (300 MHz, CDCl₃): δ = 9.54 (0.5H, dd. J = 6.9, 1.2Hz), 9.48 (0.5H, dd, J = 6.9, 1.5Hz), 8.10∼8.16 (1.5H, m), 7.93∼8.02 (1.5H, m), 7.54 (0.5H, t, J = 5.2Hz), 7.44∼7.48 (4H, m), 7.30∼7.40 (1H, m), 7.01 (0.5H, t, J = 5.5Hz), 5.17 (1H, s), 5.08 (1H, s), 4.56 (1H, t, J = 7.5Hz), 4.49 (1H, J = 7.5Hz), 2.66∼2.83 (2H, m) |

| K-1 |
|---|
| ¹H NMR (300 MHz, DMSO d6): δ = 9.34 (1H, dd, J = 6.9, 1.2Hz), 8.44∼8.52 (1H, m), 8.37 (1H, ddd, J = 8.7, 6.9, 1.5Hz), 7.99 (1H, d, J = 8.7Hz), 7.56 (1H, td, J = 6.9, 0.9Hz), 7.16∼7.29 (3H, m), 6.77 (0.5H, dd, J = 6.0, 1.2Hz), 6.74 (0.5H, t, J = 2.1Hz), 4.57 (2H, t, J = 6.9Hz), 3.73 (3H, s), 3.27 (9H, s), 2.80 (2H, q, J = 5.7Hz) |

| K-2 |
|---|
| H NMR (300 MHz, CDCl₃): δ = 9.56 (0.6H, dd, J = 6.9, 1.2Hz), 9.52 (0.4H, dd, J = 6.9, 1.2Hz), 8.20∼8.23 (0.8H, m), 8.14∼8.19 (0.8H, m), 8.08∼8.10 (1.4H, m), 7.91∼7.99 (1.5H, m), 7.60 (0.5H, d, J = 9.3Hz), 7.44∼7.55 (3H, m). 7.33∼7.43 (2H, m), 7.21∼7.27 (1H, m), 4.62∼4.68 (2H, m), 3.00∼3.09 (0.7H, m), 2.83∼2.90 (1.3H, m) |

| K-3 |
|---|
| ¹H NMR (300 MHz, DMSO d6): δ = 9.32 (1H, dd, J = 6.8, 1.4Hz), 8.32∼8.38 (1H, m), 8.02∼8.13 (4H, m), 7.47∼7.56 (3H, m), 4.60∼4.65 (2H, t, J = 7.1Hz), 2.93∼2.96 (2H, m), 1.08 (9H, s) |

| K-4 |
|---|
| ¹H NMR (300 MHz, DMSO d6): δ = 10.9 (0.5H, s), 10.5 (0.5H, s), 9.31 (1H, d, J = 6.9Hz), 8.30∼8.35 (1H, m), 7.96 (1H, t, J = 9.5Hz), 7.89 (1H, s), 7.77 (1H, d, J = 6.6Hz), 7.49∼7.57 (3H, m), 7.39∼7.45 (2.5H, m), 7.33 (1H, d, J = 6.9Hz), 6.86 (0.5H, t, J = 5.4Hz), 4.46 (2H, t, J = 7.0Hz), 2.62∼2.69 (1H, m), 2.50∼2.55 (1H, m) |

**[Table 24-35]**

| K-5 |
|---|
| ¹H NMR (300 MHz, CDCl₃): δ = 9.55 (1H, dt, J = 6.9, 1.8Hz), 8.09∼8.15 (1H, m), 7.94 (1H, d, J = 1.5Hz), 7.80 (1H, d, J = 7.5Hz), 7.67 (0.5H, d, J = 9.0Hz), 7.23∼7.55 (5H, m), 7.13∼7.19 (2H, m), 6.90 (0.5H, t, J = 5.8Hz), 4.48∼4.58 (2H, m), 3.93 (1.5H, s), 3.81 (1.5H, s), 2.66∼2.81 (2H, m) |

| K-6 |
|---|
| ¹H NMR (300 MHz, DMSO d6): δ = 9.32 (1H, dt, J = 6.6, 1.5Hz), 8.48 (0.3H, d, J = 1.8Hz), 8.29∼8.36 (0.4H, m), 8.20∼8.26 (1H, m), 8.14 (1H. s), 8.06∼8.09 (1H, m), 7.92 (0.3H, dd, J = 6.0, 2.4Hz), 7.72 (0.7H, d, J = 8.7Hz), 7.48∼7.68 (5.3H, m), 7.38 (0.7H, dd, J = 8.1, 0.6Hz), 7.02 (0.3H, d, J = 3.9Hz), 5.22∼5.23 (1.4H, m), 5.13 (1.3H, d, J = 3.9Hz), 4.96 (1.3H, s) |

| K-7 |
|---|
| ¹H NMR (300 MHz, CDCl₃): δ = 9.45 (1H, td, J = 6.6, 1.5Hz), 8.00∼8.07 (0.5H, m), 7.89∼7.97 (0.5H, m), 7.74 (0.5H, d, J = 9.0Hz), 7.43∼7.52 (1H, m), 7.25∼7.34 (1H, m), 6.91 (0.5H, t, J = 6.0Hz), 5.30∼5.50 (2H, m), 4.64 (1H, d, J = 7.2Hz), 4.39∼4.55 (3H, m), 3.34 (2H, d, J = 7.2Hz), 2.58∼2.76 (2H, m), 1.67∼1.86 (12H. m) |

| K-8 |
|---|
| ¹H NMR (300 MHz, CDCl₃): δ = 9.46 (1H, dt, J = 6.9, 2.1Hz), 7.99∼8.09 (1H, m), 7.76 (0.5H, d, J = 8.7Hz), 7.54 (0.5H, t, J = 5.4Hz), 7.46 (0.5H, d, J = 9.0Hz), 7.30 (1H, td, J = 7.2, 0.6Hz), 6.93 (0.5H, t, J = 5.7Hz), 5.30∼5.42 (1.5H, m), 5.18 (0.5H, q, J = 5.4Hz), 4.43∼4.55 (2H, m), 3.69∼3.88 (1H, m), 3.48∼3.68 (1H, m), 3.34 (2H, d, J = 6.9Hz), 2.60∼2.80 (2H, m), 1.84 (3H, s), 1.69 (3H, s), 1.42 (1.5H, d, J = 5.4Hz), 1.37 (1.5H, d, J = 5.4Hz), 1.15∼1.24 (3H, m) |

| K-9 |
|---|
| ¹H NMR (300 MHz, CDCl₃): δ = 9.46 (1H, dt, J = 6.9, 2.1Hz), 8.03∼8.10 (1H, m), 7.78 (0.5H, J = 9.0Hz), 7.54 (0.5H, t, J = 5.4Hz), 7.48 (0.5H, d, J = 9.0Hz), 7.32 (1H, td, J = 6.9, 0.9Hz), 6.93 (0.5H, t, J = 6.0Hz), 5.92∼6.08 (1H, m), 5.34 (0.5H, q, J = 5.4Hz), 5.12∼5.25 (1.5H, m), 5.01 (1H, dd, J = 9,9, 1.5Hz). 4.43∼4.57 (2H, m), 3.68∼3.90 (1H, m), 3.47∼3.67 (1H, m), 3.39 (2H, dd, J = 6.3, 1.2Hz), 2.60∼2.81 (2H, m), 1.41 (1.5H, d, J = 5.4Hz), 1.36 (1.5H, d, J = 5.4Hz), 1.17∼1.25 (3H, m) |

**[Table 24-36]**

| K-10 |
|---|
| ¹H NMR (300 MHz, CDCl₃): δ = 9.51∼9.56 (1H, m), 8.07∼8.23 (1H, m), 8.01 (0.5H, d, *J*= 9.3Hz), 7.73∼7.78 (2H, m), 7.69 (0.5H, t, J= 5.7Hz), 7.54 (0.5H, d, *J*= 9.0Hz), 7.31∼7.42 (3H, m), 7.19∼7.26 (1H, m), 7.13 (0.5H, t, *J* = 6.0Hz), 5.35 (0.5H, t, *J* = 2.1Hz), 5.14 (0.5H, t, *J* = 3.0Hz), 4.47∼4.65 (2H, m), 3.99∼4.20 (1H, m), 3.53∼3.94 (5H, m), 2.71∼2.91 (2H, m) |

| K-11 |
|---|
| ¹H NMR (300 MHz, CDCl₃): δ = 9.51∼9.56 (1H, m), 8.07∼8.18 (1H, m), 7.73∼7.76 (2.5H, m), 7.45∼7.53 (1H, m), 7.31∼7.42 (3H, m), 7.20∼7.26 (1H, m), 6.98 (0.5H, t, J= 5.8Hz), 4.92∼4.96 (0.5H, m), 4.75∼4.80 (0.5H, m), 4.42∼4.61, (2H, m), 3.81∼4.01 (4H, m), 2.74∼2.81 (1H, m), 2.64∼2.71 (1H, m), 2.01∼2.25 (2H, m) |

### <Formulation Example>

Furthermore, formulation examples of the agricultural chemicals containing the compound (1) as active ingredient are described as follows.

### Formulation Example 1 [wettable powder]

Compound (1): 30 wt%
Clay: 30 wt%
Diatomaceous earth: 35 wt%
SAN X P252 (calcium lignosulfonate: manufactured by Nippon Paper Chemicals Co., Ltd.): 4 wt%
SORPOL 8070 (sodium laurylsulfate: manufactured by Toho Chemical Industry Co., Ltd.): 1 wt%

The components described above were uniformly mixed and pulverized to obtain a wettable powder.

### Formulation Example 2 [powder]

Compound (1): 2 wt%
Clay: 90 wt%
Talc: 7 wt%
Calcium stearate: 1 wt%

The components described above were uniformly mixed to obtain a powder

### Formulation Example 3 [emulsion 1]

Compound (1): 20 wt%
N,N-dimethyl formamide: 20 wt%
SOLVESSO 150 (aromatic solvent: manufactured by Exxon Mobil Corporation): 50 wt%
NEWKALGEN CL-H (POE alkyl phenyl ether: manufactured by Takemoto Oil and Fat Co., Ltd.): 10 wt%

The components described above were uniformly mixed and dissolved to obtain an emulsion.

### Formulation Example 4 [emulsion 2]

Compound (1): 5 wt%
Xylene: 42.5 wt%
DMSO: 42.5 wt%
NEWKALGEN 2003 (the mixture of POE allyl phenyl ether formaldehyde condensate and metal alkyl benzene sulfonate: manufactured by Takemoto Oil and Fat Co., Ltd.): 10 wt%

The components described above were uniformly mixed and dissolved to obtain an emulsion.

### Formulation Example 5 [Granules 1]

Compound (1): 5 wt%
Bentonite: 40 wt%
Talc: 10 wt%
Clay: 43 wt%
SANX P252 (calcium lignosulfonate: manufactured by Nippon Paper Chemicals Co., Ltd.): 2 wt%

The components described above were uniformly pulverized and mixed. Water was added to the mixture, which was then well kneaded, granulated and dried to obtain granules.

### Formulation Example 6 [Flowable formulation]

Compound (1): 25 wt%
SORPOL 7556 (POE styrylphenyl ether sulfate: manufactured by Toho Chemical Industry Co., Ltd.): 5 wt%
Propylene glycol: 6 wt%
Bentonite: 1 wt%
1% xanthan gum aqueous solution: 3 wt%
Water: 60 wt%

The whole amount of formulation other than 1% xanthan gum aqueous solution and a suitable amount of water was premixed and then pulverized by a wet pulverizer. Subsequently, the 1% xanthan gum aqueous solution and the remaining water were added to the pulverized product thus obtained, so that 100 wt% flowable formulation was produced.

### Formulation Example 7 [Particulates 2]

Compound (1): 5 wt%
Bentonite: 40 wt%
Talc: 10 wt%
Clay: 43 wt%
SAN X P252 (calcium lignosulfonate: manufactured by Nippon Paper Chemicals Co., Ltd.): 2 wt%

The components described above were uniformly pulverized and mixed. Water was added to the mixture, which was then well kneaded, granulated and dried, so that granules were produced.

### <Biological Test Examples>

The following tests demonstrate the control effect of the compounds of the present invention against a certain range of harmful organisms. "Control effect" represents the growth inhibition in harmful invertebrate organisms to significantly reduce eating including the mortality. The protection through control of harmful organisms achieved by the compounds, however, are not limited to these species. Compound numbers refer to the compounds in Table 11 to Table 21.

### Biological Examples of the present invention

### Biological Test Example 1: Test (foliar spraying) for controlling cotton aphid (Aphis gossypii)

A cucumber leaf cut to a diameter of 3.5 cm was placed on cotton wool moistened with water. Two adult cotton aphids were released there for offspring for 24 hours and the adult insects were then removed. The diluent of the test compounds diluted with water to 200 ppm in an amount of 2 mL was sprayed onto the cucumber leaves with a spray tower. After air-drying, the sample with cotton wool was placed in a plastic cup, which was covered with a lid and kept in a chamber at a constant temperature of 25°C for rearing. On 5 days after the treatment, the life or death was observed to calculate the mortality.

As a result, the compounds of A-1, A-4, A-5, A-6, A-8, A-9, A-10, A-11, A-15, A-16, A-18, A-20, A-27, A-29, A-30, B-1, B-2, B-3, B-4, B-5, C-1, C-2, C-3, C-4, C-5, C-6, C-7, C-8, C-9, C-10, C-11, C-12, C-13, D-1, D-3, D-4, D-5, D-6, D-7, E-1, E-2, E-3, E-4, E-5, E-7, E-8, E-9, E-11, E-14, E-15, E-16, E-17, E-18, E-21, E-22, E-27, E-28, F-1, F-2, F-6, F-10, F-13, F-14, F-15, F-16, F-17, F-18, F-27, F-28, F-29, G-2, G-3, G-5, G-6, G-12, H-3, 1-1, 1-2, 1-3, 1-7, I-14, 1-16, J-1, and K-2 exhibited a mortality of 80% or more.

### Biological Test Example 2: Test (foliage dipping) for controlling diamondback moth (Plutella xylostella)

A cabbage leaf cut to a diameter of 5.0 cm was dipped in the diluent of the test compounds diluted with water to 200 ppm in an amount of 20 mL and air-dried. After air-drying, the cabbage leaf was placed in a plastic cup, and 10 larvae of diamondback moths in the third instar larva were released there. The cup was covered with a lid and kept in a chamber at a constant temperature of 25°C for rearing. On 5 days after the treatment, the life or death of the larvae was observed to calculate the mortality.

As a result, the compounds of A-18, A-23, B-7, C-1, C-4, C-6, C-10, C-12, D-1, D-4, D-5, D-7, E-6, E-11, E-14, E-25, E-26, F-4, 1-18, and K-2 exhibited a mortality of 80% or more.

### Biological Test Example 3: Test (foliage dipping) for controlling brown planthopper (Nilaparvata lugens)

Ten sampled rice seedlings were dipped in the chemical solution of the test compounds diluted with water to 200 ppm in an amount of 20 mL and air-dried. After air-drying, the rice seedlings held with urethane in a glass cylinder having an inner diameter of 4.5 cm and a length of 14 cm were set up in a plastic cup containing 40 mL of water. Larvae of brown planthoppers in the third instar larva were released in the glass cylinder. The cylinder was covered with a lid of medicine paper and kept in a chamber at a constant temperature of 25°C for rearing. On 5 days after the treatment, the life or death of the larvae was observed to calculate the mortality.

As a result, the compounds of A-1, A-2, A-3, A-4, A-5, A-6, A-7, A-8, A-9, A-10, A-11, A-13, A-14, A-15, A-16, A-17, A-18, A-19, A-20, A-22, A-23, A-24, A-25, A-26, A-27, A-28, A-29, A-30, A-31, A-32, A-33, A-35, B-1, B-2, B-3, B-4, B-5, B-7, B-8, B-9, C-1, C-2, C-3, C-4, C-5, C-6, C-7, C-8, C-9, C-10, C-11, C-12, C-13, D-1, D-3, D-4, D-5, D-6, D-7, E-1, E-2, E-3, E-4, E-5, E-6, E-7, E-10, E-11, E-12, E-13, E-14, E-15, E-18, E-19, E-20, E-21, E-22, E-23, E-24, E-26, E-27, F-1, F-2, F-3, F-5, F-6, F-13, F-14, F-15, F-16, F-26, F-27, F-28, F-29, G-1, G-2, G-3, G-4, G-5, G-6, G-7, G-8, G-9, G-10, G-11, G-12, G-13, G-14, G-15, G-16, G-17, 1-2, I-5, I-8, I-9, J-1, J-2, J-3, K-1, K-3, K-7, K-8, K-9, and K-10 exhibited a mortality of 80% or more.

### Biological Test Example 4: Test (root dipping) for controlling brown planthopper (Nilaparvata lugens)

Ten sampled rice seedlings held with urethane in a glass cylinder having an inner diameter of 4.5 cm and a length of 14 cm were set up in a plastic cup containing 40 mL of chemical solution diluted with water to 0.8 ppm. After 2 days, 10 larvae of brown planthoppers in the third instar larva were released in the glass cylinder. The glass cylinder was covered with a lid of medicine paper and kept in a chamber at a constant temperature of 25°C for rearing. On 5 days after the treatment, the life or death of the larvae was observed to calculate the mortality.

As a result, the compounds of A-1, A-5, A-10, C-1, C-10, D-1, D-3, D-4, F-2, G-3, J-1, J-2 and K-2 exhibited a mortality of 80% or more.

### Biological Test Example 5: Test (foliar spraying) for controlling southern yellow thrips (Thrips palmi)

A cucumber leaf cut to a diameter of 1.5 cm was placed on cotton wool moistened with water. The diluent of the test compounds diluted with water to 200 ppm in an amount of 2 mL was sprayed onto the cucumber leaves with a spray tower. After air-drying, 5 larvae of southern yellow thrips in the first instar larva were released there and placed with cotton wool in a plastic cup, which was covered with a lid and kept in a chamber at a constant temperature of 25°C for rearing. On 3 days after the treatment, the life or death was observed to calculate the mortality.

As a result, the compounds of A-2, A-8, A-9, A-22, B-3, C-1, C-3, C-5, C-6, C-7, C-8, C-9, C-10, C-12, D-1, D-3, D-4, D-6, D-7, E-2, E-8, G-7, and K-2 exhibited a mortality of 80% or more.

## Claims

1. A compound represented by a formula (1) or a salt thereof: 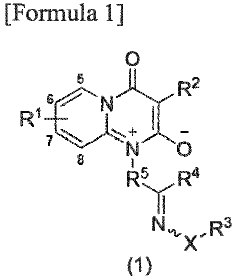 wherein
R¹ represents R^{a} or a C1 alkyl group, wherein the C1 alkyl group is substituted with one or more R^{a};
R² represents a phenyl group which is substituted with up to three R^{b};
R³ represents R^{a}; a C1-C6 alkyl group; wherein
the C1-C6 alkyl group is substituted with one or more R^{a}, and/or a C3-C6 cycloalkyl group which is substituted with one or more R^{a}, and/or a C1-C6 alkyloxy group which is substituted with one or more R^{a}, and/or a C3-C6 cycloalkyloxy group which is substituted with one or more R^{a}, and/or a C1-C6 alkylthio group which is substituted with one or more R^{a}, and/or a C1-C6 alkylsulfoxy group which is substituted with one or more R^{a}, and/or a C1-C6 alkylsulfonyl group which is substituted with one or more R^{a}, and/or an amino group, (wherein
the amino group has two substitutes independently selected from a hydrogen atom, a C1-C4 alkyl group and a C2 acyl group, wherein
the C1-C4 alkyl group is substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a}, and
the C2 acyl group is substituted with one or more R^{a}), and/or an ester group which is substituted with a C1-C4 alkyl group (-C(=O)O-C1-C4 alkyl), (wherein
the C1-C4 alkyl group is substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a}), and/or a phenyl group which is substituted with one or more R^{b}, and/or a heterocyclic group which is substituted with one or more R^{b}, wherein
the heterocyclic group has a ring composed of 2 to 6 carbon atoms and 1 to 2 heteroatoms, and the heteroatoms are independently selected from an oxygen atom, a nitrogen atom, and a sulfur atom;
a C2-C6 alkenyl group, wherein the C2-C6 alkenyl group is substituted with one or more R^{a};
a C2-C6 alkynyl group, wherein the C2-C6 alkynyl group is substituted with one or more R^{a};
a C3-C10 cycloalkyl group, wherein the C3-C10 cycloalkyl group is substituted with one or more R^{a}, and/or a C1-C6 alkyl group which is substituted with one or more R^{a} and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a};
a phenyl group which is substituted with one or more R^{b}; or
a heterocyclic group which is substituted with one or more R^{b}, wherein
the heterocyclic group has a ring composed of 2 to 6 carbon atoms and 1 to 2 heteroatoms, and the heteroatoms are independently selected from an oxygen atom, a nitrogen atom, and a sulfur atom;
R⁴ represents R^{a}; a C1-C6 alkyl group, wherein
the C1-C6 alkyl group is substituted with one or more R^{a}, and/or a C3-C6 cycloalkyl group which is substituted with one or more R^{a}, and/or a C1-C3 alkyloxy group which is substituted with one or more R^{a}, and/or a C3-C6 cycloalkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a};
a C2-C6 alkenyl group, wherein the C2-C6 alkenyl group is substituted with one or more R^{a};
a C2-C6 alkynyl group, wherein the C2-C6 alkynyl group is substituted with one or more R^{a};
a C1-C6 alkyloxy group, wherein the C1-C6 alkyloxy group is substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a};
an amino group, wherein the amino group has two substitutes independently selected from a hydrogen atom, a C1-C4 alkyl group and a C2-C6 acyl group, wherein
the C1-C4 alkyl group is substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a}, and
the C2-C6 acyl group is substituted with one or more R^{a};
a C1-C4 alkylthio group; a C1-C4 alkylsulfoxy group; or a C1-C4 alkylsulfonyl group, wherein
the C1-C4 alkylthio group, the C1-C4 alkylsulfoxy group, and the C1-C4 alkylsulfonyl group are substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a};
R⁵ represents a C1-C4 alkylene group which is substituted with one or more R^{a}, a C2-C4 alkenylene group which is substituted with one or more R^{a}, or a C2-C4 alkynylene group which is substituted with one or more R^{a};
X represents a direct bond, O, S(O)₀₋₂, NR^{c}, N(R^{c})-C(=O), N⁺(R^{c})₂, OC(=O), or OC(=O)O;
each R^{a} is independently selected from a hydrogen atom, a halogen atom, a cyano group, a nitro group, an SCN group and a hydroxy group;
each R^{b} is independently selected from R^{a}; a pentafluorosulfanyl group; a C1-C6 alkyl group, wherein
the C1-C6 alkyl group is substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a};
a C2-C3 alkenyl group, wherein the C2-C3 alkenyl group is substituted with one or more R^{a};
a C2-C3 alkynyl group, wherein the C2-C3 alkynyl group is substituted with one or more R^{a};
a C1-C6 alkyloxy group, wherein the C1-C6 alkyloxy group is substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a};
an amino group, wherein the amino group has two substitutes independently selected from a hydrogen atom, a C1-C4 alkyl group and a C2 acyl group, wherein
the C1-C4 alkyl group is substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a}, and the C2 acyl group is substituted with one or more R^{a};
a C1-C4 alkylthio group; a C1-C4 alkylsulfoxy group; and a C1-C4 alkylsulfonyl group, wherein
the C1-C4 alkylthio group, the C1-C4 alkylsulfoxy group, and the C1-C4 alkylsulfonyl group are substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a};
a phenyl group which is substituted with one or more R^{a}; and a heterocyclic group which is substituted with one or more R^{a}; and
each R^{c} is independently selected from R^{a}; a C1-C4 alkyl group; a C2-C3 alkenyl group; a C2-C3 alkynyl group; a C3-C6 cycloalkyl group; a C1-C6 alkyloxy group; a C1-C4 alkylthio group; a C1-C4 alkylsulfoxy group; a C1-C4 alkylsulfonyl group; a phenyl group; a heterocyclic group, wherein
the heterocyclic group has a ring composed of 2 to 6 carbon atoms and 1 to 2 heteroatoms, and the heteroatoms are independently selected from the group consisting of a nitrogen atom, an oxygen atom, and a sulfur atom, wherein
the C1-C4 alkyl group, the C2-C3 alkenyl group, the C2-C3 alkynyl group, the C3-C6 cycloalkyl group, the C1-C6 alkyloxy group, the C1-C4 alkylthio group, the C1-C4 alkylsulfoxy group, the C1-C4 alkylsulfonyl group, the phenyl group and the heterocyclic group are substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a};
a C2-C3 alkenyl group; a C2-C3 alkynyl group; and a C2 acyl group, wherein
the C2-C3 alkenyl group, the C2-C3 alkynyl group, and the C2 acyl group are substituted with one or more R^{a}.

2. The compound or the salt thereof according to claim 1, wherein
R¹ is a hydrogen atom or a methyl group, having a substitution position at the 8-position;
R² is a phenyl group which is substituted with up to three R^{b};
R³ is R^{a}; a C1-C6 alkyl group; wherein
the C1-C6 alkyl group is substituted with one or more R^{a}, and/or a C3-C6 cycloalkyl group which is substituted with one or more R^{a}, and/or a C1-C6 alkyloxy group which is substituted with one or more R^{a}, and/or a C3-C6 cycloalkyloxy group which is substituted with one or more R^{a}, and/or a C1-C6 alkylthio group which is substituted with one or more R^{a}, and/or a C1-C6 alkylsulfoxy group which is substituted with one or more R^{a}, and/or a C1-C6 alkylsulfonyl group which is substituted with one or more R^{a}, and/or a C1-C6 alkylamino group which is substituted with one or more R^{a}, (wherein the C1-C6 alkylamino group has two substitutes independently selected from a hydrogen atom, a C1-C4 alkyl group and a C2 acyl group), and/or a phenyl group which is substituted with one or more R^{b}, and/or a heterocyclic group which is substituted with one or more R^{b}, wherein the heterocyclic group has a ring composed of 2 to 6 carbon atoms and 1 to 2 heteroatoms, and the heteroatoms are independently selected from an oxygen atom, a nitrogen atom, and a sulfur atom;
a C2-C6 alkenyl group, wherein the C2-C6 alkenyl group is substituted with one or more R^{a};
a C2-C6 alkynyl group, wherein the C2-C6 alkenyl group is substituted with one or more R^{a};
a C3-C6 cycloalkyl group, wherein the C3-C6 cycloalkyl group is substituted with one or more R^{a}, and/or a C1-C6 alkyl group which is substituted with one or more R^{a} and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a};
a phenyl group which is substituted with one or more R^{b}; or
a heterocyclic group which is substituted with one or more R^{b}, wherein the heterocyclic group has a ring composed of 2 to 6 carbon atoms and 1 to 2 heteroatoms, and the heteroatoms are independently selected from an oxygen atom, a nitrogen atom, and a sulfur atom;
R⁴ is R^{a}; a C1-C6 alkyl group, wherein the C1-C6 alkyl group is substituted with one or more R^{a}, and/or a C1-C3 alkyloxy group which is substituted with one or more R^{a};
a C1-C6 alkyloxy group, wherein the C1-C6 alkyloxy group is substituted with one or more R^{a}, and/or a C1-C3 alkyloxy group which is substituted with one or more R^{a};
an amino group, wherein the amino group has two substitutes independently selected from a hydrogen atom, a C1-C4 alkyl group and a C2 acyl group, wherein
the C1-C4 alkyl group is substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a}, and the C2 acyl group is substituted with one or more R^{a};
R⁵ is a C1-C2 alkylene group, an alkenylene group or an alkynylene group;
X is O, S(O)₀₋₂, N(R^{c}), N(R^{c})-C(=O), N⁺R^{c}₂, OC(=O), or OC(=O)O;
each R^{a} is independently selected from a hydrogen atom, a halogen atom, a cyano group, a nitro group, and an SCN group;
each R^{b} is independently selected from Ra; a pentafluorosulfanyl group; a C1-C6 alkyl group, and a C1-C6 alkyloxy group, wherein
the C1-C6 alkyl group and the C1-C6 alkyloxy group are substituted with one or more R^{a}, and/or a C1-C3 alkyloxy group one or more R^{a}, a C1-C3 alkyloxy group which is substituted with one or more R^{a};
each R^{c} is independently selected from R^{a}, a C1-C4 alkyl group, a C3-C6 cycloalkyl group, and a C2 acyl group, wherein
the C1-C4 alkyl group and the C3-C6 cycloalkyl group are substituted with one or more R^{a}, and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a}, and the C2 acyl group is substituted with one or more R^{a}.

3. The compound or the salt thereof according to claim 1, wherein
R¹ is a hydrogen atom or a methyl group, having a substitution position at the 8-position;
R² represents a phenyl group which is substituted with up to three R^{b};
R³ represents R^{a}; a C1-C6 alkyl group, wherein
the C1-C6 alkyl group is substituted with one or more R^{a}, and/or a C3-C6 cycloalkyl group which is substituted with one or more R^{a}, and/or a C1-C4 alkyloxy group which is substituted with one or more R^{a}, and/or a C3-C6 cycloalkyloxy group which is substituted with one or more R^{a}, and/or a C1-C6 alkylthio group which is substituted with one or more R^{a}, and/or a C1-C6 alkylamino group which is substituted with one or more R^{a}, wherein the C1-C6 alkylamino group has two substitutes independently selected from a hydrogen atom, a C1-C4 alkyl group and a C2 acyl group, and/or a phenyl group which is substituted with one or more R^{a}, and/or a heterocyclic group which is substituted with one or more R^{a}, wherein the heterocyclic group has a ring composed of 2 to 6 carbon atoms and 1 to 2 heteroatoms, and the heteroatoms are independently selected from an oxygen atom, a nitrogen atom, and a sulfur atom;
a C2-C6 alkenyl group, wherein the C2-C6 alkenyl group is substituted with one or more R^{a};
a C3-C6 cycloalkyl group, wherein the C3-C6 cycloalkyl group is substituted with one or more R^{a}, and/or a C1-C3 alkyl group which is substituted with one or more R^{a} and/or a C1-C3 alkyloxy group which is substituted with one or more R^{a}, and/or a C1-C3 alkylthio group which is substituted with one or more R^{a}, and/or a C1-C3 alkylsulfoxy group which is substituted with one or more R^{a}, and/or a C1-C3 alkylsulfonyl group which is substituted with one or more R^{a};
a phenyl group which is substituted with one or more R^{b}; or
a heterocyclic group which is substituted with one or more R^{b}, wherein the heterocyclic group has a ring composed of 2 to 6 carbon atoms and 1 to 2 heteroatoms, and the heteroatoms are independently selected from an oxygen atom, a nitrogen atom, and a sulfur atom;
R⁴ is a hydrogen atom, a fluorine atom, a chlorine atom, a cyano group, a methyl group, an ethyl group, a methyloxy group, an ethyloxy group, an amino group, an N-methylamino group, an N,N-dimethylamino group, a trifluoromethyl group or a nitro group;
R⁵ is a C1-C2 alkylene group;
X is O, S(O)₀₋₂, NR^{c}, N(R^{c})-C(=O), N⁺R^{c}₂, OC(=O), or OC(=O)O;
each R^{a} is independently selected from a hydrogen atom, a halogen atom, a cyano group, a nitro group, an SCN group and a hydroxyl group;
each R^{b} is independently selected from a hydrogen atom, a fluorine atom, a chlorine atom, a bromine atom, an iodine atom, a pentafluorosulfanyl group, a methyl group, an ethyl group, an n-propyl group, an i-propyl group, a c-propyl group, a monofluoromethyl group, a difluroromethyl group, a trifluoromethyl group, a methyloxy group, an ethyloxy group, an n-propyloxy group, an i-propyloxy group, a trifluoromethyloxy group, a 2,2,2-trifluoroethyloxy group, a methylthio group, a methylsulfoxy group, and a methylsulfonyl group; and
R^{c} is independently selected from a hydrogen atom, a methyl group, an ethyl group, an n-propyl group, an i-propyl group, a c-propyl group, a monofluoromethyl group, a difluoromethyl group, a trifluoromethyl group, a methyloxy group, an ethyloxy group, an n-propyloxy group, an i-propyloxy group, a c-propyloxy group, an n-butyloxy group, an i-butyloxy group, an s-butyloxy group, a trifluoromethyloxy group, a 2,2,2-trifluoroethyloxy group, a methylthio group, a methylsulfoxy group, a methylsulfonyl group, a phenyl group which is substituted with one or more R^{a}, and a pyridyl group which is substituted with one or more R^{a}.

4. A compound selected from the group consisting of the following and a salt thereof:
1-(3-(((2-methoxy-2-propanyl)oxy)imino)propyl)-4-oxo-3-(3-(trifluoromethyl)phenyl)-4H-pyrido[1,2-a]pyrimidinium inner salt, i.e., a compound A-5 in Table 11;
1-(3-((1-ethoxyethoxy)imino)propyl)-4-oxo-3-(3-(trifluoromethyl)phenyl)-4H-pyrido[1,2-a]pyrimidinium inner salt, i.e., a compound A-7 in Table 11;
1-(3-((cyanomethoxy)imino)propyl)-4-oxo-3-(3-(trifluoromethyl)phenyl)-4H-pyrido[1,2-a]pyrimidinium inner salt, i.e., a compound A-8 in Table 11;
1-(3-(((methylthio)methoxy)imino)propyl)-4-oxo-3-(3-(trifluoromethyl)phenyl)-4H-pyrido[1,2-a]pyrimidinium inner salt, i.e., a compound A-9 in Table 11;
3-(3,5-dichlorophenyl)-1-(3-((1-ethoxyethoxy)imino)propyl)-4-oxo-4H-pyrido[1,2-a]pyrimidinium inner salt, i.e., a compound A-18 in Table 11;
4-oxo-1-(3-((1-phenylethoxy)imino)propyl)-3-(3-(trifluoromethyl)phenyl)-4H-pyrido[1,2-a]pyrimidinium inner salt, i.e., a compound B-7 in Table 12;
4-oxo-1-(3(phenoxyimino)propyl)-3-(3-(trifluoromethyl)phenyl)-4H-pyrido[1,2-a]pyrimidinium inner salt, i.e., a compound C-1 in Table 13;
1-(3-((2-fluorophenoxy)imino)propyl)-4-oxo-3-(3-(trifluoromethyl)phenyl)-4H-pyrido[1,2-a]pyrimidinium inner salt, i.e., a compound C-2 in Table 13;
4-oxo-1-(3-((2-methylphenoxy)imino)propyl)-3-(3-(trifluoromethyl)phenyl)-4H-pyrido[1,2-a]pyrimidinium inner salt, i.e., a compound C-4 in Table 13;
4-oxo-1-(3-((3-(trifluoromethyl)phenoxy)imino)propyl)-3-(3-(trifluoromethyl)phenyl)-4H-pyrido[1,2-a]pyrimidinium inner salt, i.e., a compound C-6 in Table 13;
1-(3-((3-chlorophenoxy)imino)propyl)-4-oxo-3-(3-(trifluoromethyl)phenyl)-4H-pyrido[1,2-a]pyrimidinium inner salt, i.e., a compound C-8 in Table 13;
1-(3-((4-chlorophenoxy)imino)propyl)-4-oxo-3-(3-(trifluoromethyl)phenyl)-4H-pyrido [1,2-a]pyrimidinium inner salt, i.e., a compound C-10 in Table 13;
4-oxo-1-(3-((pivaloyloxy)imino)propyl)-3-(3-(trifluoromethyl)phenyl)-4H-pyrido[1,2-a]pyrimidinium inner salt, i.e., a compound D-1 in Table 14;
1-(3-(((methoxycarbonyl)oxy)imino)propyl)-4-oxo-3-(3-(trifluoromethyl)phenyl)--4H-pyrido [1,2-a]pyrimidinium inner salt, i.e., a compound D-3 in Table 14;
1-(3-(((cyclopropanecarbonyl)oxy)imino)propyl)-4-oxo-3-(3-(trifluoromethyl)phenyl)-4H-pyrido[1,2-a]pyrimidinium inner salt, i.e., a compound D-4 in Table 14;
1-(3-(((3,5-dimethylbenzoyl)oxy)imino)propyl)-4-oxo-3-(3-(trifluoromethyl)phenyl)-4H-pyrido[1,2-a]pyrimidinium inner salt, i.e., a compound D-5 in Table 14;
1-(3-((((3R,5R,7R)-adamantane-1-carbonyl)oxy)imino)propyl)-4-oxo-3-(3-(trifluoromethyl)phenyl)-4H-pyrido[1,2-a]pyrimidinium inner salt, i.e., a compound D-7 in Table 14;
1-(3-((((Z)-3-chloroallyl)oxy)imino)propyl)-4-oxo-3-(3-(trifluoromethyl)phenyl)-4H-pyrido[1,2-a]pyrimidinium inner salt, i.e., a compound E-6 in Table 15;
preparation of 1-(3-(((3-methyl-2-butenyl)oxy)imino)propyl)-4-oxo-3-(3-(trifluoromethyl)phenyl)-4H-pyrido[1,2-a]pyrimidinium inner salt, i.e., a compound E-7 in Table 15;
3-(3,5-dichlorophenyl)-1-(3-(((3-methyl-2-butenyl)oxy)imino)propyl)-4-oxo-4H-pyrido[1,2-a]-pyrimidinium inner salt, i.e., a compound E-11 in Table 15;.
1-(3-((((Z)-3-chloroallyl)oxy)imino)propyl)-3-(3,5-dichlorophenyl)-4-oxo-4H-pyrido[1,2-a]-pyrimidinium inner salt, i.e., a compound E-14 in Table 15;
1-(3-((((Z)-3-chloroallyl)oxy)imino)propyl)-4-oxo-3-phenyl-4H-pyrido[1,2-a]pyrimidinium inner salt, i.e., a compound E-27 in Table 15;
1-(3-(hydroxyimino)propyl)-4-oxo-3-(3-(trifluoromethyl)phenyl)-4H-pyrido[1,2-a]pyrimidinium inner salt, i.e., a compound F-1 in Table 16;
3-(3,5-dichlorophenyl)-1-(3-(hydroxyimino)propyl)-4-oxo-4H-pyrido[1,2-a]pyrimidinium inner salt, i.e., a compound F-5 in Table 16;
1-(3-(hydroxyimino)propyl)-4-oxo-3-phenyl-4H-pyrido[1,2-a]pyrimidinium inner salt, i.e., a compound F-15 in Table 16;
1-(3-(hydroxyimino)propyl)-4-oxo-3-(3-methylphenyl)-4H-pyrido[1,2-a]pyrimidinium inner salt, i.e., a compound F-16 in Table 16;
4-oxo-1-(3-(((2-tetrahydrofuranyl)oxy)imino)propyl)-3-(3-(trifluoromethyl)phenyl)-4H-pyrido[1,2-a]pyrimidinium inner salt, i.e., a compound G-2 in Table 17;
4-oxo-1-(3-(((2-tetrahydrofranyl)oxy)imino)propyl)-3-(3-(trifluoromethyl)phenyl)-4H-pyrido[1,2-a]pyrimidinium inner salt, i.e., a compound G-3 in Table 17;
4-oxo-3-phenyl-1-(3-(((tetrahydrofuranyl)oxy)imino)propyl)-4H-pyrido[1,2-a]pyrimidinium inner salt, i.e., a compound G-4 in Table 17;
4-oxo-1-(3-(((tetrahydrofuranyl)oxy)imino)propyl)-3-(m-toluyl)-4H-pyrido[1,2-a]pyrimidinium inner salt, i.e., a compound G-6 in Table 17;
3-(3-methoxyphenyl)-4-oxo-1-(3-(((2-tetrahydrofuranyl)oxy)imino)propyl)-4H-pyrido[1,2-a]pyrimidinium inner salt, i.e., a compound G-7 in Table 17;
1-(3-(((6-chloro-3-pyridinyl)methoxy)imino)propyl)-4-oxo-3-(3-(trifluoromethyl)phenyl)-4H-pyrido[1,2-a]pyrimidinium inner salt, i.e., a compound J-2 in Table 20; and
1-(3-(((6-chloro-3-pyridinyl)oxy)imino)propyl)-4-oxo-3-(3-(trifluoromethyl)phenyl)-4H-pyrido[1,2-a]pyrimidinium inner salt, i.e., a compound K-2 in Table 21.

5. An insecticidal composition comprising the compound or the salt thereof according to any one of claims 1 to 4 and at least one component selected from the group consisting of a surfactant, a solid diluent and a liquid diluent.

6. The insecticidal composition according to any one of claims 1 to 4, further comprising at least one biologically active compound or chemical agent.

7. The composition according to claim 6, wherein the at least one biologically active compound or chemical agent is selected from the group consisting of:
alanycarb, aldicarb, bendiocarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylylcarb, metolcarb, fenothiocarb, fenoxycarb, acephate, azamethiphos, azinphos-ethyl, azinphos-methyl, ethylthiometon, chloroethoxyfos, cadusafos, chlorethoxyfos, chlorfenvinphos, chlormephos, chlorpyrifos, chlorpyrifos-methyl, coumaphos, cyanophos, demeton-S-methyl, diazinon, dichlorvos, dicrotophos, dimethoate, dimethylvinphos, EPN, ethion, ethoprophos, famphur, fenamiphos, fenitrothion, fenthion, fosthiazate, heptenophos, imicyafos, isofenphos, isopropyl O-(methoxyaminothiophosphoryl)salicylate, isoxathion, malathion, mecarbam, methamidophos, methidathion, mevinphos, monocrotophos, naled, omethoate, oxydemeton ethyl, parathion, parathion-methyl, PAP, phorate, phosalone, phosmet, phosphamidon, phoxim, pirimiphos-methyl, profenofos-propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, chlorpyrifos-ethyl, disulfoton, sulprofos, flupyrazofos, phenthoate, fonofos, tribufos, endosulfan, alpha-endosulfan, gamma-HCH, dicofol, chlordane, dieldrin, methoxychlor, acetoprole, fipronil, ethiprole, pyrafluprole, pyriprole, flufiprole, broflanilide, afoxolaner, fluralaner, sarolaner, fluxametamide, acrinathrin, allethrin, d-cis-trans allethrin, d-trans allethrin, bifenthrin, kappa-bifenthrin, bioallethrin S-cyclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, ethofenprox, fenpropathrin, fenvalerate, flucythrinate, flumethrin, tau-fluvalinate, halfenprox, imiprothrin, kadethrin, permethrin, phenothrin, prallethrin, pyrethrin, resmethrin, silafluofen, tefluthrin, kappa-tefluthrin, phthalthrin, tetramethrin, tralomethrin, transfluthrin, methoxadiazone, metofluthrin, profluthrin, pyrethrum, terallethrin, momfluorothrin, heptafluthrin, meperfluthrin, tetramethylfluthrin, dimefluthrin, chloroprallethrin, epsilon-methofluthrin, epsilon-momfluorothrin, protrifenbute, acetamiprid, clothianidin, dinotefuran, imidacloprid, nitenpyram, thiacloprid, thiamethoxam, sulfoxaflor, flupyradifurone, triflumezopyrim, dicloromezotiaz, flupyrimin, spinosad, spinetoram, abamectin, ivermectin, emamectin benzoate, milbemectin, lepimectin, hydroprene, kinoprene, diofenolan, methoprene, pyriproxyfen, pymetrozine, flonicamid, etoxazole, diafenthiuron, azocyclotin, cyhexatin, fenbutatin oxide, propargite, tetradifon, chlorfenapyr, tralopyril, DNOC, bensultap, cartap, thiocyclam, thiosultap, thiosultap-sodium, bistrifluron, chlorfluazuron, diflubenzuron, flucycloxuron, flufenoxuron, hexaflumuron, lufenuron, novaluron, noviflumuron, teflubenzuron, triflumuron, bistrifluron, buprofezin, cyromazine, chromafenozide, halofenozide, methoxyfenozide, tebufenozide, amitraz, hydramethylnon, acequinocyl, fluacrypyrim, pyriminostrobin, flufenoxystrobin, fenazaquin, fenpyroximate, pyrimidifen, pyridaben, tebufenpyrad, tolfenpyrad, pyflubumide, spirodiclofen, spirotetramat, spiromesifen, spirodione, cyflumetofen, cyenopyrafen, flubendiamide, chlorantraniliprole, cyantraniliprole, cyclaniliprole, tetraniliprole, cyhalodiamide, quinomethionate, hexythiazox, bifenazate, flufenerim, pyrifluquinazon, flometoquin, fluopyram, fluazaindolizine, amidoflumet, nicotine, chloropicrin, sulfuryl fluoride, chlorothie, clofentezine, diflovidazin, rotenone, indoxacarb, piperonyl butoxide, chlordimeform, pyridalyl, azadirachtin, benzoxymate, afidopiropen, fluhexafon, tioxazafen, fluensulfone, benclothiaz, carzole, an insecticidal soap, dimehypo, nithiazine, a borate, metaldehyde, ryanodine, sulfluramid, acynonapyr, benzpyrimoxan, tyclopyrazoflor, metalaxyl, metalaxyl M, oxadixyl, ofurase, benalaxyl, benalaxyl-M, kiralaxyl, ofurace, furalaxyl, cyprofuram, bupirimate, dimethirimol, ethirimol, hymexazol, hydroxyisoxazole, oxathiapiprolin, octhilinone, oxolinic acid, benomyl, thiophanate-methyl, carbendazim, fuberidazole, thiabendazole, debacarb, diethofencarb, zoxamide, ethaboxam, pencycuron, fluopicolide, diflumetorim, bupirimate, benodanil, flutolanil, mepronil, isofetamid, fenfuram, oxycarboxin, carboxin, thifluzamide, fluxapyroxad, furametpyr, penflufen, penthiopyrad, benzovindiflupyr, bixafen, isopyrazam, sedaxane, inpyrfluxam, fluindapyr, isoflucypram, boscalid, azoxystrobin, coumetoxystrobin, kresoxym-methyl, trifloxystrobin, picoxystrobin, pyraclostrobin, dimoxystrobin, metominostrobin, orysastrobin, fluoxastrobin, pyraoxystrobin, pyrametostrobin, flufenoxystrobin, fenaminstrobin, enoxastrobin, coumoxystrobin, mandestrobin, triclopyricarb, famoxadon, fenamidone, triclopyricarb, pyribencarb, cyazofamid, amisulbrom, binapacryl, meptyldinocarb, dinocap, fluazinam, ferimzone, fentin-acetate, fentin chloride, fentin hydroxide, triphenyltin hydroxide, triphenyltin acetate, oxine-copper, silthiofam, ametoctradin, mepanipyrim, nitrapyrin, pyrimethanil, cyprodinil, blasticidin S, kasugamycin, kasugamycin hydrochloride hydrate, streptomycin, oxytetracyclin, quinoxyfen, proquinazid, fludioxonil, fenpiclonil, fluoroimide, procymidone, iprodione, vinclozolin, edifenphos, iprobenfos, pyrazophos, isoprothiolane, propamocarb, propamocarb hydrochloride, tea tree oil extract, triforine, pyrifenox, pyrisoxazole, fenarimol, nuarimol, azaconazole, bromuconazole, diniconazole, diniconazole-M, epoxiconazole, fluquinconazole, oxpoconazole, pefurazoate, difenoconazole, fenbuconazole, imibenconazole, ipconazole, metconazole, tetraconazole, triadimefon, triadimenol, triticonazole, uniconazole, imazalil, bitertanol, triflumizole, etaconazole, propiconazole, penconazole, flusilazole, flutriafol, myclobutanil, paclobutrazol, prothioconazole, cyproconazole, tebuconazole, hexaconazole, prochloraz, simeconazole, aldimorph, dodemorph, dodemorph acetate, tridemorph, fenpropimorph, dimethomorph, flumorph, pyrimorph, piperalin, fenpropidin, spiroxamine, fenhexamid, fenpyrazamine, ferbam, metam, methasulfocarb, metiram, thiram, manzeb, maneb, zineb, ziram, polycarbamate, propineb, thiuram, pyributicarb, validamycin, mildiomycin, polyoxin, benthiavalicarb, benthiavalicarb-isopropyl, valifenalate, iprovalicarb, mandipropamid, fenpicoxamid, fthalide, pyroquilon, tricyclazole, carpropamid, diclocymet, fenoxanil, acibenzolar-S-methyl, probenazole, dichlobentiazox, tiadinil, isotianil, cymoxanil, fosetyl, tecloftalam, triazoxide, flusulfamide, diclomezine, cyflufenamid, metrafenone, pyriofenon, flutianil, tebufloquin, fosetyl aluminum, tolclofos-methyl, echlomezol, tolprocarb, ipfentrifluconazole, mefentrifluconazole, quinofumelin, pydiflumetofen, Bordeaux mixture, copper acetate, basic copper sulfate, copper oxychloride, cupric hydroxide, oxyquinoline copper, copper, sulfur, captan, captafol, folpet, anilazine, chlorothalonil, dichlorophene, pentachlorophenol and salts thereof, hexachlorobenzene, quintozene, iminoctadine acetate, iminoctadine albesilate, guanidine, dodine, dodine free base, guazatine, guazatine acetate, albesilate, dithianon, quinomethionate, fluoroimide, tolylfluanid, dichlofluanid, dinobuton, dazomet, dipymetitrone, pyraziflumid, picarbutrazox, tecnazene, nitrothal-isopropyl, dicyclomet, acibenzolar, prohexadione-calcium, bronopol, diphenylamine, flumetover, benthoxazin, biphenyl, chloroneb, CNA, iodocarb, prothiocarb, aminopyrifen, 3 -chloro-5-phenyl-6-methyl-4-(2,6-difluorophenyl)pyridazine, Bacillus species and insecticidal proteins and bactericidal proteins produced from Bacillus species, and insecticidal proteins, bactericidal proteins, entomopathogenic bacteria, entomopathogenic viruses and entomopathogenic fungi produced from Bt crops.

8. A composition for protecting animals or birds from harmful parasitic invertebrate organisms, comprising the compound or the salt thereof according to any one of claims 1 to 4 in an effective amount for controlling harmful parasitic invertebrate organisms parasitic on animals or birds and at least one carrier.

9. A method for controlling harmful invertebrate organisms, comprising bringing the harmful invertebrate organisms into contact with the compound or the salt thereof according to any one of claims 1 to 4 in a biologically effective amount.

10. A method for producing crops with harmful invertebrate organisms controlled, comprising performing the method according to claim 9.

11. A method for treating crops comprising bringing the seeds or placentas of crops into contact with the compound or the salt thereof according to any one of claims 1 to 4.

12. A method for producing treated crops comprising performing the method according to claim 11.

13. A treated seed comprising the compound or the salt thereof according to any one of claims 1 to 4 in an amount of about 0.0001 to 1 mass% relative to the seed.
